# EUROPEAN PATENT SPECIFICATION

(11) **EP 2 569 276 B1**
(45) Date of publication and mention of the grant of the patent: **24.02.2021**
(21) Application number: 11731455.9
(22) Date of filing: 12.05.2011
(51) Int. Cl.: C07C 217/08, A61K 48/00, C12N 15/11, C07C 217/46, C07C 229/12, C07C 229/30, C07C 237/06, C07C 271/20, C07C 327/06, C07D 233/60, C07D 249/04, A61K 49/04, C12N 15/88

(54) **NOVEL CATIONIC LIPIDS AND METHODS OF USE THEREOF**
NEUE KATIONISCHE LIPIDE UND VERFAHREN ZU IHRER VERWENDUNG
NOUVEAUX LIPIDES CATIONIQUES ET PROCÉDÉS D'UTILISATION DE CEUX-CI

(30) Priority: 17.09.2010 US 384050 P; 12.05.2010 US 334104 P
(43) Date of publication of application: 20.03.2013
(73) Proprietor: Arbutus Biopharma Corporation, Vancouver, BC V7Y 1B3 (CA)
(72) Inventor: HEYES, James, Vancouver, British Columbia V5Z 4C1 (CA); WOOD, Mark, Port Moody, British Columbia V3H 5K4 (CA); MARTIN, Alan, Vancouver, British Columbia V6B 0B9 (CA)
(74) Representative: J A Kemp LLP
(86) International application number: PCT/GB2011/000723
(87) International publication number: WO 2011/141705

(56) References cited:
- WO-A1-2009/132131
- WO-A1-2010/042877
- WO-A1-2010/054401
- WO-A1-2010/144740
- WO-A1-2011/017548
- WO-A1-2011/075656

## Description

### BACKGROUND OF THE INVENTION

Therapeutic nucleic acids include, *e.g*., small interfering RNA (siRNA), microRNA (miRNA), antisense oligonucleotides, ribozymes, plasmids, and immune-stimulating nucleic acids. These nucleic acids act via a variety of mechanisms. In the case of interfering RNA molecules such as siRNA and miRNA, these nucleic acids can down-regulate intracellular levels of specific proteins through a process termed RNA interference (RNAi). Following introduction of interfering RNA into the cell cytoplasm, these double-stranded RNA constructs can bind to a protein termed RISC. The sense strand of the interfering RNA is displaced from the RISC complex, providing a template within RISC that can recognize and bind mRNA with a complementary sequence to that of the bound interfering RNA. Having bound the complementary mRNA, the RISC complex cleaves the mRNA and releases the cleaved strands. RNAi can provide down-regulation of specific proteins by targeting specific destruction of the corresponding mRNA that encodes for protein synthesis.

The therapeutic applications of RNAi are extremely broad, since interfering RNA constructs can be synthesized with any nucleotide sequence directed against a target protein. To date, siRNA constructs have shown the ability to specifically down-regulate target proteins in both *in vitro* and *in vivo* models. In addition, siRNA constructs are currently being evaluated in clinical studies.

However, two problems currently faced by interfering RNA constructs are, first, their susceptibility to nuclease digestion in plasma and, second, their limited ability to gain access to the intracellular compartment where they can bind RISC when administered systemically as free interfering RNA molecules. These double-stranded constructs can be stabilized by the incorporation of chemically modified nucleotide linkers within the molecule, *e.g*., phosphothioate groups. However, such chemically modified linkers provide only limited protection from nuclease digestion and may decrease the activity of the construct. Intracellular delivery of interfering RNA can be facilitated by the use of carrier systems such as polymers, cationic liposomes, or by the covalent attachment of a cholesterol moiety to the molecule. However, improved delivery systems are required to increase the potency of interfering RNA molecules such as siRNA and miRNA and to reduce or eliminate the requirement for chemically modified nucleotide linkers.

In addition, problems remain with the limited ability of therapeutic nucleic acids such as interfering RNA to cross cellular membranes (*see,* Vlassov et al., Biochim. Biophys. Acta, 1197:95-1082 (1994)) and in the problems associated with systemic toxicity, such as complement-mediated anaphylaxis, altered coagulatory properties, and cytopenia (Galbraith et al., Antisense Nucl. Acid Drug Des., 4:201-206 (1994)).

To attempt to improve efficacy, investigators have also employed lipid-based carrier systems to deliver chemically modified or unmodified therapeutic nucleic acids. Zelphati et al. (J. Contr. Rel., 41:99-119 (1996)) describes the use of anionic (conventional) liposomes, pH sensitive liposomes, immunoliposomes, fusogenic liposomes, and cationic lipid/antisense aggregates. Similarly, siRNA has been administered systemically in cationic liposomes, and these nucleic acid-lipid particles have been reported to provide improved down-regulation of target proteins in mammals including non-human primates (Zimmermann et al., Nature, 441: 111-114 (2006)).

In spite of this progress, there remains a need in the art for improved lipid-therapeutic nucleic acid compositions that are suitable for general therapeutic use. Preferably, these compositions would encapsulate nucleic acids with high efficiency, have high drug:lipid ratios, protect the encapsulated nucleic acid from degradation and clearance in serum, be suitable for systemic delivery, and provide intracellular delivery of the encapsulated nucleic acid. In addition, these nucleic acid-lipid particles should be well-tolerated and provide an adequate therapeutic index, such that patient treatment at an effective dose of the nucleic acid is not associated with significant toxicity and/or risk to the patient. The present invention provides such compositions, methods of making the compositions, and methods of using the compositions to introduce nucleic acids into cells, including for the treatment of diseases.

WO 2010/042877 describes amino lipids and methods for the delivery of nucleic acids.

WO 2009/132131 describes an amino lipid based lipid formulation.

### BRIEF SUMMARY OF THE INVENTION

The present invention provides novel lipid particles comprising cationic (amino) lipids, which are advantageous for the *in vivo* delivery of active agents or therapeutic agents such as nucleic acids, as well as lipid particles such as nucleic acid-lipid particle compositions suitable for *in vivo* therapeutic use. The present invention also provides in vitro methods of introducing active agents or therapeutic agents such as nucleic acids into cells using these lipid compositions, and lipid particles comprising a therapeutic agent, for use in a method for the treatment of various disease conditions.

In one aspect, the present invention provides a lipid particle comprising a cationic lipid of Formula I having the following structure: 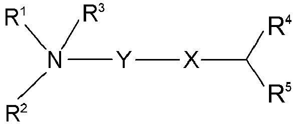 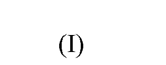 or salts thereof, wherein R¹ and R² are either the same or different and are independently hydrogen (H) or an optionally substituted C₁-C₆ alkyl, C₂-C₆ alkenyl, or C₂-C₆ alkynyl, or R¹ and R² may join to form an optionally substituted heterocyclic ring;
R³ is either absent or is hydrogen (H) or a C₁-C₆ alkyl to provide a quaternary amine;
R⁴ and R⁵ are independently selected from the group consisting of a dodecatrienyl moiety, a tetradectrienyl moiety, a hexadecatrienyl moiety, an octadecatrienyl moiety, and an icosatrienyl moiety;
X is O, S, N(R⁶), C(O), C(O)N(R⁶ ), N(R⁶)C(O), OC(O)N(R⁶), N(R⁶)C(O)O, C(O)S, C(S)O, S(O), S(O)(O), C(S), wherein R⁶ is hydrogen (H) or an optionally substituted C₁-C₁₀ alkyl, C₂-C₁₀ alkenyl, or C₂-C₁₀ alkynyl; and
Y is either absent or is an optionally substituted C₁-C₆ alkyl, C₂-C₆ alkenyl, or C₂-C₆ alkynyl.

In one embodiment, R¹ and R² are independently selected from the group consisting of a methyl group and an ethyl group, *i.e.,* R¹ and R² are both methyl groups, R¹ and R² are both ethyl groups, or R¹ and R² are a combination of one methyl group and one ethyl group. In another embodiment, R¹ and R² are joined to form an optionally substituted heterocyclic ring having from 2 to 5 carbon atoms (*e.g.,* 2, 3, 4, or 5 carbon atoms, or from 2-5, 2-4, 2-3, 3-5, 3-4, or 4-5 carbon atoms) and from 1 to 3 heteroatoms (*e.g.,* 1, 2, or 3 heteroatoms, or from 1-3, 1-2, or 2-3 heteroatoms) selected from the group consisting of nitrogen (N), oxygen (O), sulfur (S), and combinations thereof. In certain instances, R¹ and R² are joined to form an optionally substituted diazole (*e.g.,* an optionally substituted imidazole) or an optionally substituted triazole.

In yet another embodiment, X is O, C(O)N(R ⁶), N(R⁶)C(O)O, or C(O)S. In certain instances, R⁶ is hydrogen (H), an optionally substituted methyl group, an optionally substituted ethyl group, or an optionally substituted C₃-C₁₀ alkyl, alkenyl, or alkynyl group (*e.g.,* an optionally substituted C₃, C₄, C₅, C₆, C₇, C₈, C₉, or C₁₀ alkyl, alkenyl, or alkynyl group). In a further embodiment, X is an optionally substituted heterocyclic ring having from 2 to 5 carbon atoms (*e.g.,* 2, 3, 4, or 5 carbon atoms, or from 2-5, 2-4, 2-3, 3-5, 3-4, or 4-5 carbon atoms) and from 1 to 3 heteroatoms (*e.g*., 1, 2, or 3 heteroatoms, or from 1-3, 1-2, or 2-3 heteroatoms) selected from the group consisting of nitrogen (N), oxygen (O), sulfur (S), and combinations thereof. In certain instances, X is an optionally substituted diazole (*e.g.,* an optionally substituted imidazole) or an optionally substituted triazole. In other embodiments, Y is (CH₂)ₙ and n is 0, 1, 2, 3, 4, 5, or 6. In particular embodiments, n is 2, 3, or 4 (*e.g.,* n is 2-4, 2-3, or 3-4).

In one embodiment, at least one of R⁴ and R⁵ (*e*.*g*., both R⁴ and R⁵), which can be independently optionally substituted, comprises at least one site of unsaturation. R⁴ and R⁵ are independently selected from the group consisting of a dodecenyl moiety, a tetradecenyl (*e.g.,* myristoleyl) moiety, a hexadecenyl (*e.g.,* palmitoleyl) moiety, an octadecenyl (*e.g.,* oleyl) moiety, and an icosenyl moiety. In preferred embodiments, one of R⁴ or R⁵ is an oleyl moiety or R⁴ and R⁵ are both oleyl moieties. In some embodiments, one of R⁴ or R⁵ comprises one site of unsaturation and the other side-chain comprises at least two or three sites of unsaturation as described herein.

In another embodiment, at least one of R⁴ and R⁵ (*e.g.,* both R⁴ and R⁵), which can be independently optionally substituted, comprises at least two sites of unsaturation. R⁴ and R⁵ are independently selected from the group consisting of a dodecadienyl moiety, a tetradecadienyl moiety, a hexadecadienyl moiety, an octadecadienyl moiety, and an icosadienyl moiety. In certain instances, the octadecadienyl moiety is a linoleyl moiety. In preferred embodiments, one of R⁴ or R⁵ is a linoleyl moiety or R⁴ and R⁵ are both linoleyl moieties. In some embodiments, one of R⁴ or R⁵ comprises two sites of unsaturation and the other side-chain comprises at least three sites of unsaturation as described herein. In a different cationic lipid described herein, R¹ and R² are not both methyl groups, X is C(O)O, Y is (CH₂)₂ or (CH₂)₃, and R⁴ and R⁵ are both linoleyl moieties.

In yet another embodiment, at least one of R⁴ and R⁵ (*e*.*g*., both R⁴ and R⁵), which can be independently optionally substituted, comprises at least three sites of unsaturation. R⁴ and R⁵ are independently selected from the group consisting of a dodecatrienyl moiety, a tetradectrienyl moiety, a hexadecatrienyl moiety, an octadecatrienyl moiety, and an icosatrienyl moiety. In certain instances, the octadecatrienyl moiety is a linolenyl moiety or a γ-linolenyl moiety. In preferred embodiments, one of R⁴ or R⁵ is a linolenyl or γ-linolenyl moiety, or R⁴ and R⁵ are both linolenyl or γ-linolenyl moieties. In some embodiments, one of R⁴ or R⁵ comprises three sites of unsaturation and the other side-chain comprises at least four sites of unsaturation as described herein.

In other cationic lipids described herein, at least one of R⁴ and R⁵ (*e.g.,* both R⁴ and R⁵) comprises a substituted C₁₂-C₂₄ alkyl. In particular embodiments, the substituted C₁₂-C₂₄ alkyl comprises a C₁₂-C₂₄ alkyl having at least 1-6 C₁-C₆ alkyl substituents. In certain instances, one of R⁴ or R⁵ is a phytanyl moiety or R⁴ and R⁵ are both phytanyl moieties. In some cases, one of R⁴ or R⁵ comprises a substituted C₁₂-C₂₄ alkyl and the other side-chain comprises at least one, two, or three sites of unsaturation as described herein.

In still yet another case, one of R⁴ or R⁵ comprises at least one optionally substituted cyclic alkyl group. In particular cases, the optionally substituted cyclic alkyl group comprises an optionally substituted saturated cyclic alkyl group, an optionally substituted unsaturated cyclic alkyl group, or a combination thereof. In certain instances, the optionally substituted saturated cyclic alkyl group comprises an optionally substituted C₃₋₈ cycloalkyl group such as, for example, a cyclopropyl group. In certain other instances, the optionally substituted unsaturated cyclic alkyl group comprises an optionally substituted C₃₋₈ cycloalkenyl group. In some cases, one of R⁴ or R⁵ comprises at least one optionally substituted cyclic alkyl group and the other side-chain comprises any one of the optionally substituted C₁₀-C₂₄ alkyl, C₁₀-C₂₄ alkenyl, C₁₀-C₂₄ alkynyl, or C₁₀-C₂₄ acyl groups described herein (*e.g.,* a side-chain comprising at least one, two, or three sites of unsaturation).

In some embodiments, each of the at least one, two, or three sites of unsaturation present in one or both R⁴ and R⁵ correspond to a *cis* double bond, a *trans* double bond, or combinations thereof at specific positions in one or both R⁴ and R⁵. In certain instances, one or both R⁴ and R⁵ are C₁₈ alkyl groups containing any combination of double bonds in the *cis* and/or *trans* configuration at one or more positions in the side-chain (*e.g., cis* and/or *trans* double bonds at position 9, at positions 6 and 9, at positions 3, 6, and 9, at positions 6, 9, and 12, or at positions 7 and 9 of a C₁₈ alkyl group). One skilled in the art will understand that the at least one, two, or three sites of unsaturation present in one or both R⁴ and R⁵ can also be characterized by either the "E" chemical notation and/or the "Z" chemical notation.

In particular embodiments, the cationic lipid of Formula I is selected from the group consisting of Compounds 34 and 35, as described herein.

The present invention provides a lipid particle comprising one or more of the above cationic lipids of Formula I or salts thereof. In certain embodiments, the lipid particle further comprises one or more non-cationic lipids such as neutral lipids. In certain other embodiments, the lipid particle further comprises one or more conjugated lipids capable of reducing or inhibiting particle aggregation. In additional embodiments, the lipid particle further comprises one or more active agents or therapeutic agents.

In certain embodiments, the non-cationic lipid component of the lipid particle may comprise a phospholipid, cholesterol (or cholesterol derivative), or a mixture thereof. In one particular embodiment, the phospholipid comprises dipalmitoylphosphatidylcholine (DPPC), distearoylphosphatidylcholine (DSPC), or a mixture thereof. In some embodiments, the conjugated lipid component of the lipid particle comprises a polyethyleneglycol (PEG)-lipid conjugate. In certain instances, the PEG-lipid conjugate comprises a PEG-diacylglycerol (PEG-DAG) conjugate, a PEG-dialkyloxypropyl (PEG-DAA) conjugate, or a mixture thereof. In other embodiments, the lipid conjugate comprises a polyoxazoline (POZ)-lipid conjugate such as a POZ-DAA conjugate.

In some embodiments, the active agent or therapeutic agent comprises a nucleic acid. In certain instances, the nucleic acid comprises an interfering RNA molecule which includes any double-stranded RNA capable of mediating RNAi, such as, *e.g.,* an siRNA, Dicer-substrate dsRNA, shRNA, aiRNA, pre-miRNA, or mixtures thereof. In certain other instances, the nucleic acid comprises single-stranded or double-stranded DNA, RNA, or a DNA/RNA hybrid such as, *e.g.,* an antisense oligonucleotide, a ribozyme, a plasmid, an immunostimulatory oligonucleotide, or mixtures thereof.

In other embodiments, the active agent or therapeutic agent is fully encapsulated within the lipid portion of the lipid particle such that the active agent or therapeutic agent in the lipid particle is resistant in aqueous solution to enzymatic degradation, *e.g.,* by a nuclease or protease. In further embodiments, the lipid particle is substantially non-toxic to mammals such as humans.

In preferred embodiments, the present invention provides serum-stable nucleic acid-lipid particles (SNALP) comprising: (a) one or more nucleic acids such as interfering RNA molecules; (b) one or more cationic lipids of Formula I or salts thereof; (c) one or more non-cationic lipids; and (d) one or more conjugated lipids that inhibit aggregation of particles.

In some embodiments, the present invention provides nucleic acid-lipid particles (*e.g.,* SNALP) comprising: (a) one or more nucleic acids; (b) one or more cationic lipids of Formula I or salts thereof comprising from about 50 mol % to about 85 mol % of the total lipid present in the particle; (c) one or more non-cationic lipids comprising from about 13 mol % to about 49.5 mol % of the total lipid present in the particle; and (d) one or more conjugated lipids that inhibit aggregation of particles comprising from about 0.5 mol % to about 2 mol % of the total lipid present in the particle.

In one aspect of this embodiment, the nucleic acid-lipid particle comprises: (a) one or more nucleic acids; (b) one or more cationic lipids of Formula I or a salt thereof comprising from about 50 mol % to about 65 mol % of the total lipid present in the particle; (c) one or more non-cationic lipids comprising a mixture of one or more phospholipids and cholesterol or a derivative thereof, wherein the one or more phospholipids comprises from about 4 mol % to about 10 mol % of the total lipid present in the particle and the cholesterol or derivative thereof comprises from about 30 mol % to about 40 mol % of the total lipid present in the particle; and (d) one or more PEG-lipid conjugates comprising from about 0.5 mol % to about 2 mol % of the total lipid present in the particle. This embodiment of nucleic acid-lipid particle is generally referred to herein as the "1:57" formulation.

In certain instances, the 1:57 formulation comprises: (a) one or more nucleic acids; (b) one or more cationic lipids of Formula I or a salt thereof comprising from about 52 mol % to about 62 mol % of the total lipid present in the particle; (c) a mixture of one or more phospholipids and cholesterol or a derivative thereof comprising from about 36 mol % to about 47 mol % of the total lipid present in the particle; and (d) one or more PEG-lipid conjugates comprising from about 1 mol % to about 2 mol % of the total lipid present in the particle. In one particular embodiment, the 1:57 formulation is a four-component system comprising about 1.4 mol % PEG-lipid conjugate (*e.g.,* PEG2000-C-DMA), about 57.1 mol % cationic lipid of Formula I or a salt thereof, about 7.1 mol % DPPC (or DSPC), and about 34.3 mol % cholesterol (or derivative thereof).

In another aspect of this embodiment, the nucleic acid-lipid particle comprises: (a) one or more nucleic acids; (b) one or more cationic lipids of Formula I or a salt thereof comprising from about 56.5 mol % to about 66.5 mol % of the total lipid present in the particle; (c) cholesterol and/or one or more derivatives thereof comprising from about 31.5 mol % to about 42.5 mol % of the total lipid present in the particle; and (d) one or more PEG-lipid conjugates comprising from about 1 mol % to about 2 mol % of the total lipid present in the particle. This embodiment of nucleic acid-lipid particle is generally referred to herein as the "1:62" formulation. In one particular embodiment, the 1:62 formulation is a three-component system which is phospholipid-free and comprises about 1.5 mol % PEG-lipid conjugate (*e.g.,* PEG2000-C-DMA), about 61.5 mol % cationic lipid of Formula I or a salt thereof, and about 36.9 mol % cholesterol (or derivative thereof).

Additional embodiments related to the 1:57 and 1:62 formulations are described in PCT Publication No. WO 09/127060 and U.S. Publication No. 20110071208.

In other embodiments, the present invention provides nucleic acid-lipid particles *(e.g.,* SNALP) comprising: (a) one or more nucleic acids; (b) one or more cationic lipids of Formula I or salts thereof comprising from about 2 mol % to about 50 mol % of the total lipid present in the particle; (c) one or more non-cationic lipids comprising from about 5 mol % to about 90 mol % of the total lipid present in the particle; and (d) one or more conjugated lipids that inhibit aggregation of particles comprising from about 0.5 mol % to about 20 mol % of the total lipid present in the particle.

In one aspect of this embodiment, the nucleic acid-lipid particle comprises: (a) one or more nucleic acids; (b) one or more cationic lipids of Formula I or a salt thereof comprising from about 30 mol % to about 50 mol % of the total lipid present in the particle; (c) a mixture of one or more phospholipids and cholesterol or a derivative thereof comprising from about 47 mol % to about 69 mol % of the total lipid present in the particle; and (d) one or more PEG-lipid conjugates comprising from about 1 mol % to about 3 mol % of the total lipid present in the particle. This embodiment of nucleic acid-lipid particle is generally referred to herein as the "2:40" formulation. In one particular embodiment, the 2:40 formulation is a four-component system which comprises about 2 mol % PEG-lipid conjugate (e.g., PEG2000-C-DMA), about 40 mol % cationic lipid of Formula I or a salt thereof, about 10 mol % DPPC (or DSPC), and about 48 mol % cholesterol (or derivative thereof).

In further embodiments, the present invention provides nucleic acid-lipid particles (*e.g.,* SNALP) comprising: (a) one or more nucleic acids; (b) one or more cationic lipids of Formula I or salts thereof comprising from about 50 mol % to about 65 mol % of the total lipid present in the particle; (c) one or more non-cationic lipids comprising from about 25 mol % to about 45 mol % of the total lipid present in the particle; and (d) one or more conjugated lipids that inhibit aggregation of particles comprising from about 5 mol % to about 10 mol % of the total lipid present in the particle.

In one aspect of this embodiment, the nucleic acid-lipid particle comprises: (a) one or more nucleic acids; (b) one or more cationic lipids of Formula I or a salt thereof comprising from about 50 mol % to about 60 mol % of the total lipid present in the particle; (c) a mixture of one or more phospholipids and cholesterol or a derivative thereof comprising from about 35 mol % to about 45 mol % of the total lipid present in the particle; and (d) one or more PEG-lipid conjugates comprising from about 5 mol % to about 10 mol % of the total lipid present in the particle. This embodiment of nucleic acid-lipid particle is generally referred to herein as the "7:54" formulation. In certain instances, the non-cationic lipid mixture in the 7:54 formulation comprises: (i) a phospholipid of from about 5 mol % to about 10 mol % of the total lipid present in the particle; and (ii) cholesterol or a derivative thereof of from about 25 mol % to about 35 mol % of the total lipid present in the particle. In one particular embodiment, the 7:54 formulation is a four-component system which comprises about 7 mol % PEG-lipid conjugate (*e.g.,* PEG750-C-DMA), about 54 mol % cationic lipid of Formula I or a salt thereof, about 7 mol % DPPC (or DSPC), and about 32 mol % cholesterol (or derivative thereof).

In another aspect of this embodiment, the nucleic acid-lipid particle comprises: (a) one or more nucleic acids; (b) one or more cationic lipids of Formula I or a salt thereof comprising from about 55 mol % to about 65 mol % of the total lipid present in the particle; (c) cholesterol and/or one or more derivatives thereof comprising from about 30 mol % to about 40 mol % of the total lipid present in the particle; and (d) one or more PEG-lipid conjugates comprising from about 5 mol % to about 10 mol % of the total lipid present in the particle. This embodiment of nucleic acid-lipid particle is generally referred to herein as the "7:58" formulation. In one particular embodiment, the 7:58 formulation is a three-component system which is phospholipid-free and comprises about 7 mol % PEG-lipid conjugate (e.g., PEG750-C-DMA), about 58 mol % cationic lipid of Formula I or a salt thereof, and about 35 mol % cholesterol (or derivative thereof).

Additional embodiments related to the 7:54 and 7:58 formulations are described in U.S. Patent Publication No. 20110076335.

The present invention also provides pharmaceutical compositions comprising a lipid particle such as a nucleic acid-lipid particle (*e.g.,* SNALP) and a pharmaceutically acceptable carrier.

In another aspect, the present invention provides in vitro methods for introducing one or more therapeutic agents such as nucleic acids into a cell, the method comprising contacting the cell with a lipid particle described herein (*e.g.,* SNALP). In one embodiment, the cell is in a mammal and the mammal is a human.

In yet another aspect, the present invention provides lipid particles of the invention containing a therapeutic agent for use in methods for the *in vivo* delivery of one or more therapeutic agents such as nucleic acids, the method comprising administering to a mammal the lipid particle (*e.g.,* SNALP). In certain embodiments, the lipid particles (*e.g.,* SNALP) are administered by one of the following routes of administration: oral, intranasal, intravenous, intraperitoneal, intramuscular, intra-articular, intralesional, intratracheal, subcutaneous, and intradermal. In particular embodiments, the lipid particles (*e.g.,* SNALP) are administered systemically, *e.g.,* via enteral or parenteral routes of administration. In preferred embodiments, the mammal is a human.

In a further aspect, the present invention provides lipid particles of the invention containing a therapeutic agent for use in methods for treating a disease or disorder in a mammal in need thereof, the method comprising administering to the mammal a therapeutically effective amount of the lipid particle (*e.g.,* SNALP) comprising one or more therapeutic agents such as nucleic acids. Non-limiting examples of diseases or disorders include a viral infection, a liver disease or disorder, and cancer. Preferably, the mammal is a human.

In certain embodiments, the lipid particles are for use in methods for treating a liver disease or disorder by administering a nucleic acid such as an interfering RNA (*e.g.,* siRNA) in nucleic acid-lipid particles (*e.g.,* SNALP), alone or in combination with a lipid-lowering agent. Examples of lipid diseases and disorders include, but are not limited to, dyslipidemia (*e.g.,* hyperlipidemias such as elevated triglyceride levels (hypertriglyceridemia) and/or elevated cholesterol levels (hypercholesterolemia)), atherosclerosis, coronary heart disease, coronary artery disease, atherosclerotic cardiovascular disease (CVD), fatty liver disease (hepatic steatosis), abnormal lipid metabolism, abnormal cholesterol metabolism, diabetes (including Type 2 diabetes), obesity, cardiovascular disease, and other disorders relating to abnormal metabolism. Non-limiting examples of lipid-lowering agents include statins, fibrates, ezetimibe, thiazolidinediones, niacin, beta-blockers, nitroglycerin, calcium antagonists, and fish oil.

In one particular embodiment, the lipid particles are for use in a method for lowering or reducing cholesterol levels in a mammal (*e.g.,* human) in need thereof (*e.g.,* a mammal with elevated blood cholesterol levels), the method comprising administering to the mammal a therapeutically effective amount of a nucleic acid-lipid particle (*e.g.,* a SNALP formulation) described herein comprising one or more interfering RNAs (*e.g.,* siRNAs) that target one or more genes associated with metabolic diseases and disorders. In another particular embodiment, the lipid particles are for use in a method for lowering or reducing triglyceride levels in a mammal (*e.g.,* human) in need thereof (*e.g.,* a mammal with elevated blood triglyceride levels), the method comprising administering to the mammal a therapeutically effective amount of a nucleic acid-lipid particle (*e.g.,* a SNALP formulation) described herein comprising one or more interfering RNAs (*e.g.,* siRNAs) that target one or more genes associated with metabolic diseases and disorders. These methods can be carried out *in vitro* using standard tissue culture techniques or *in vivo* by administering the interfering RNA (*e.g.,* siRNA) using any means known in the art. In preferred embodiments, the interfering RNA (*e.g.,* siRNA) is delivered to a liver cell (*e.g.,* hepatocyte) in a mammal such as a human.

Additional embodiments related to treating a liver disease or disorder using a lipid particle are described in, *e.g.,* PCT Publication No. WO 2010/083615, and U.S. Patent Publication No. 20060134189.

In other embodiments, the lipid particles are for use in methods for treating a cell proliferative disorder such as cancer by administering a nucleic acid such as an interfering RNA (*e.g.,* siRNA) in nucleic acid-lipid particles (*e.g.,* SNALP), alone or in combination with a chemotherapy drug. The methods can be carried out *in vitro* using standard tissue culture techniques or *in vivo* by administering the interfering RNA (*e.g.,* siRNA) using any means known in the art. In preferred embodiments, the interfering RNA (*e.g.,* siRNA) is delivered to a cancer cell in a mammal such as a human, alone or in combination with a chemotherapy drug. The nucleic acid-lipid particles and/or chemotherapy drugs may also be co-administered with conventional hormonal, immunotherapeutic, and/or radiotherapeutic agents.

Additional embodiments related to treating a cell proliferative disorder using a lipid particle are described in, *e.g.,* PCT Publication No. WO 09/082817, U.S. Patent Publication No. 20090149403, PCT Publication No. WO 09/129319, and PCT Publication No. WO 2011/038160.

In further embodiments, the lipid particles are for use in methods for preventing or treating a viral infection such as an arenavirus (*e.g.,* Lassa virus) or filovirus (*e.g.,* Ebola virus, Marburg virus, *etc.*) infection which causes hemorrhagic fever or a hepatitis (*e.g.,* Hepatitis C virus) infection which causes acute or chronic hepatitis by administering a nucleic acid such as an interfering RNA (*e.g.,* siRNA) in nucleic acid-lipid particles (*e.g.,* SNALP), alone or in combination with the administration of conventional agents used to treat or ameliorate the viral condition or any of the symptoms associated therewith. The methods can be carried out *in vitro* using standard tissue culture techniques or *in vivo* by administering the interfering RNA using any means known in the art. In certain preferred embodiments, the interfering RNA (*e.g.,* siRNA) is delivered to cells, tissues, or organs of a mammal such as a human that are infected and/or susceptible of being infected with the hemorrhagic fever virus, such as, *e.g.,* cells of the reticuloendothelial system (*e.g.,* monocytes, macrophages, *etc.*)*,* endothelial cells, liver cells (*e.g.,* hepatocytes), fibroblast cells, and/or platelet cells. In certain other preferred embodiments, the interfering RNA (e.g., siRNA) is delivered to cells, tissues, or organs of a mammal such as a human that are infected and/or susceptible of being infected with the hepatitis virus, such as, *e.g.,* cells of the liver (*e.g.,* hepatocytes).

Additional embodiments related to preventing or treating a viral infection using a lipid particle are described in, *e.g.,* U.S. Patent Publication No. 20070218122, U.S. Patent Publication No. 20070135370, PCT Publication No. WO 2011/011447, PCT Publication No. WO 2010/105372, and U.S. Patent No. 8,455,455, filed March 31, 2011.

The lipid particles of the invention (*e.g.,* SNALP) comprising one or more cationic lipids of Formula I or salts thereof are particularly advantageous and suitable for use in the administration of nucleic acids such as interfering RNA to a subject (*e.g.,* a mammal such as a human) because they are stable in circulation, of a size required for pharmacodynamic behavior resulting in access to extravascular sites, and are capable of reaching target cell populations.

Other objects, features, and advantages of the present invention will be apparent to one of skill in the art from the following detailed description.

### BRIEF DESCRIPTION OF THE DRAWINGS

None

### DETAILED DESCRIPTION OF THE INVENTION

### I. Introduction

The present invention is based, in part, upon the discovery of novel cationic (amino) lipids that provide advantages when used in lipid particles for the *in vivo* delivery of an active or therapeutic agent such as a nucleic acid into a cell of a mammal. In particular, the present invention provides nucleic acid-lipid particle compositions comprising one or more of the novel cationic lipids described herein that provide increased activity of the nucleic acid (*e.g.,* interfering RNA) and improved tolerability of the compositions *in vivo,* resulting in a significant increase in the therapeutic index as compared to nucleic acid-lipid particle compositions previously described.

In particular embodiments, the present invention provides lipid particles comprising novel cationic lipids that enable the formulation of improved compositions for the *in vitro* and *in vivo* delivery of interfering RNA such as siRNA. It is shown herein that these improved lipid particle compositions are effective in down-regulating (*e.g.,* silencing) the protein levels and/or mRNA levels of target genes. Furthermore, it is shown herein that the activity of these improved lipid particle compositions is dependent on the presence of the novel cationic lipids.

The lipid particles and compositions of the present invention may be used for a variety of purposes, including the delivery of encapsulated or associated (*e.g.,* complexed) therapeutic agents such as nucleic acids to cells, both *in vitro* and *in vivo.* Accordingly, the present invention further provides lipid particles for use in methods of treating diseases or disorders in a subject in need thereof by contacting the subject with the lipid particle that encapsulates or is associated with a suitable therapeutic agent, wherein the lipid particle comprises one or more of the novel cationic lipids described herein.

As described herein, the lipid particles of the present invention are particularly useful for the delivery of nucleic acids, including, e.g., interfering RNA molecules such as siRNA. Therefore, the lipid particles and compositions of the present invention may be used to decrease the expression of target genes and proteins both *in vitro* and *in vivo* by contacting cells with a lipid particle comprising one or more novel cationic lipids described herein, wherein the lipid particle encapsulates or is associated with a nucleic acid that reduces target gene expression (*e.g.,* an siRNA). Alternatively, the lipid particles and compositions of the present invention may be used to increase the expression of a desired protein both *in vitro* and *in vivo* by contacting cells with a lipid particle comprising one or more novel cationic lipids described herein, wherein the lipid particle encapsulates or is associated with a nucleic acid that enhances expression of the desired protein *(e.g.,* a plasmid encoding the desired protein).

Various exemplary embodiments of the cationic lipids, lipid particles and compositions comprising the same, and their use to deliver active or therapeutic agents such as nucleic acids to modulate gene and protein expression, are described in further detail below.

### II. Definitions

As used herein, the following terms have the meanings ascribed to them unless specified otherwise.

The term "interfering RNA" or "RNAi" or "interfering RNA sequence" as used herein includes single-stranded RNA (*e.g.,* mature miRNA, ssRNAi oligonucleotides, ssDNAi oligonucleotides), double-stranded RNA (*i.e.,* duplex RNA such as siRNA, Dicer-substrate dsRNA, shRNA, aiRNA, or pre-miRNA), a DNA-RNA hybrid (*see, e.g.,* PCT Publication No. WO 2004/078941), or a DNA-DNA hybrid (*see, e.g.,* PCT Publication No. WO 2004/104199) that is capable of reducing or inhibiting the expression of a target gene or sequence (*e.g.,* by mediating the degradation or inhibiting the translation of mRNAs which are complementary to the interfering RNA sequence) when the interfering RNA is in the same cell as the target gene or sequence. Interfering RNA thus refers to the single-stranded RNA that is complementary to a target mRNA sequence or to the double-stranded RNA formed by two complementary strands or by a single, self-complementary strand. Interfering RNA may have substantial or complete identity to the target gene or sequence, or may comprise a region of mismatch (*i.e.,* a mismatch motif). The sequence of the interfering RNA can correspond to the full-length target gene, or a subsequence thereof. Preferably, the interfering RNA molecules are chemically synthesized.

Interfering RNA includes "small-interfering RNA" or "siRNA," *e.g.,* interfering RNA of about 15-60, 15-50, or 15-40 (duplex) nucleotides in length, more typically about 15-30, 15-25, or 19-25 (duplex) nucleotides in length, and is preferably about 20-24, 21-22, or 21-23 (duplex) nucleotides in length (*e.g.,* each complementary sequence of the double-stranded siRNA is 15-60, 15-50, 15-40, 15-30, 15-25, or 19-25 nucleotides in length, preferably about 20-24, 21-22, or 21-23 nucleotides in length, and the double-stranded siRNA is about 15-60, 15-50, 15-40, 15-30, 15-25, or 19-25 base pairs in length, preferably about 18-22, 19-20, or 19-21 base pairs in length). siRNA duplexes may comprise 3' overhangs of about 1 to about 4 nucleotides or about 2 to about 3 nucleotides and 5' phosphate termini. Examples of siRNA include, without limitation, a double-stranded polynucleotide molecule assembled from two separate stranded molecules, wherein one strand is the sense strand and the other is the complementary antisense strand; a double-stranded polynucleotide molecule assembled from a single stranded molecule, where the sense and antisense regions are linked by a nucleic acid-based or non-nucleic acid-based linker; a double-stranded polynucleotide molecule with a hairpin secondary structure having self-complementary sense and antisense regions; and a circular single-stranded polynucleotide molecule with two or more loop structures and a stem having self-complementary sense and antisense regions, where the circular polynucleotide can be processed *in vivo* or *in vitro* to generate an active double-stranded siRNA molecule. As used herein, the term "siRNA" includes RNA-RNA duplexes as well as DNA-RNA hybrids (*see, e.g.,* PCT Publication No. WO 2004/078941).

Preferably, siRNA are chemically synthesized. siRNA can also be generated by cleavage of longer dsRNA (e.g., dsRNA greater than about 25 nucleotides in length) with the *E. coli* RNase III or Dicer. These enzymes process the dsRNA into biologically active siRNA (*see*, *e*.*g*., Yang et al., Proc. Natl. Acad. Sci. USA, 99:9942-9947 (2002); Calegari et al., Proc. Natl. Acad. Sci. USA, 99:14236 (2002); Byrom et al., Ambion TechNotes, 10(1):4-6 (2003); Kawasaki et al., Nucleic Acids Res., 31:981-987 (2003); Knight et al., Science, 293:2269-2271 (2001); and Robertson et al., J. Biol. Chem., 243:82 (1968)). Preferably, dsRNA are at least 50 nucleotides to about 100, 200, 300, 400, or 500 nucleotides in length. A dsRNA may be as long as 1000, 1500, 2000, 5000 nucleotides in length, or longer. The dsRNA can encode for an entire gene transcript or a partial gene transcript. In certain instances, siRNA may be encoded by a plasmid (*e.g.,* transcribed as sequences that automatically fold into duplexes with hairpin loops).

As used herein, the term "mismatch motif' or "mismatch region" refers to a portion of an interfering RNA (*e.g.,* siRNA) sequence that does not have 100% complementarity to its target sequence. An interfering RNA may have at least one, two, three, four, five, six, or more mismatch regions. The mismatch regions may be contiguous or may be separated by 1, 2, 3, 4, 5, 6, 7, 8, 9, 10, 11, 12, or more nucleotides. The mismatch motifs or regions may comprise a single nucleotide or may comprise two, three, four, five, or more nucleotides.

The phrase "inhibiting expression of a target gene" refers to the ability of a nucleic acid such as an interfering RNA (*e.g.,* siRNA) to silence, reduce, or inhibit the expression of a target gene. To examine the extent of gene silencing, a test sample (*e.g.,* a sample of cells in culture expressing the target gene) or a test mammal (*e.g.,* a mammal such as a human or an animal model such as a rodent *(e.g.,* mouse) or a non-human primate (*e.g.,* monkey) model) is contacted with a nucleic acid such as an interfering RNA (*e.g.,* siRNA) that silences, reduces, or inhibits expression of the target gene. Expression of the target gene in the test sample or test animal is compared to expression of the target gene in a control sample (*e.g.,* a sample of cells in culture expressing the target gene) or a control mammal (*e.g.,* a mammal such as a human or an animal model such as a rodent (*e.g.,* mouse) or non-human primate (*e.g.,* monkey) model) that is not contacted with or administered the nucleic acid (*e.g.,* interfering RNA). The expression of the target gene in a control sample or a control mammal may be assigned a value of 100%. In particular embodiments, silencing, inhibition, or reduction of expression of a target gene is achieved when the level of target gene expression in the test sample or the test mammal relative to the level of target gene expression in the control sample or the control mammal is about 95%, 90%, 85%, 80%, 75%, 70%, 65%, 60%, 55%, 50%, 45%, 40%, 35%, 30%, 25%, 20%, 15%, 10%, 5%, or 0%. In other words, the nucleic acids (e.g., interfering RNAs) are capable of silencing, reducing, or inhibiting the expression of a target gene by at least about 5%, 10%, 15%, 20%, 25%, 30%, 35%, 40%, 45%, 50%, 55%, 60%, 65%, 70%, 75%, 80%, 85%, 90%, 95%, or 100% in a test sample or a test mammal relative to the level of target gene expression in a control sample or a control mammal not contacted with or administered the nucleic acid (*e.g.,* interfering RNA). Suitable assays for determining the level of target gene expression include, without limitation, examination of protein or mRNA levels using techniques known to those of skill in the art, such as, *e.g.,* dot blots, Northern blots, *in situ* hybridization, ELISA, immunoprecipitation, enzyme function, as well as phenotypic assays known to those of skill in the art.

An "effective amount" or "therapeutically effective amount" of an active agent or therapeutic agent such as a therapeutic nucleic acid (*e.g.,* interfering RNA such as an siRNA) is an amount sufficient to produce the desired effect, *e.g.,* an inhibition of expression of a target sequence in comparison to the normal expression level detected in the absence of the nucleic acid (*e.g.,* interfering RNA). Inhibition of expression of a target gene or target sequence is achieved when the value obtained with a nucleic acid such as an interfering RNA (*e.g.,* siRNA) relative to the control is about 95%, 90%, 85%, 80%, 75%, 70%, 65%, 60%, 55%, 50%, 45%, 40%, 35%, 30%, 25%, 20%, 15%, 10%, 5%, or 0%. Suitable assays for measuring expression of a target gene or target sequence include, *e.g.,* examination of protein or RNA levels using techniques known to those of skill in the art such as dot blots, northern blots, *in situ* hybridization, ELISA, immunoprecipitation, enzyme function, as well as phenotypic assays known to those of skill in the art.

By "decrease," "decreasing," "reduce," or "reducing" of an immune response by a nucleic acid such as an interfering RNA (*e.g.,* siRNA) is intended to mean a detectable decrease of an immune response to a given nucleic acid (*e.g.,* a modified interfering RNA). In some instances, the amount of decrease of an immune response by a nucleic acid such as a modified interfering RNA may be determined relative to the level of an immune response in the presence of an unmodified interfering RNA. As a non-limiting example, a detectable decrease can be about 5%, 10%, 15%, 20%, 25%, 30%, 35%, 40%, 45%, 50%, 55%, 60%, 65%, 70%, 75%, 80%, 85%, 90%, 95%, 100%, or more lower than the immune response detected in the presence of the unmodified interfering RNA. A decrease in the immune response to a nucleic acid (*e.g.,* interfering RNA) is typically measured by a decrease in cytokine production (*e.g.,* IFNy, IFNα, TNFα, IL-6, IL-8, or IL-12) by a responder cell *in vitro* or a decrease in cytokine production in the sera of a mammalian subject after administration of the nucleic acid (*e.g.,* interfering RNA).

As used herein, the term "responder cell" refers to a cell, preferably a mammalian cell, that produces a detectable immune response when contacted with an immunostimulatory nucleic acid such as an unmodified interfering RNA (*e.g.,* unmodified siRNA). Exemplary responder cells include, without limitation, dendritic cells, macrophages, peripheral blood mononuclear cells (PBMCs), splenocytes, and the like. Detectable immune responses include, *e.g.,* production of cytokines or growth factors such as TNF-α, IFN-α, IFN-β, IFN-γ, IL-1, IL-2, IL-3, IL-4, IL-5, IL-6, IL-8, IL-10, IL-12, IL-13, TGF, and combinations thereof. Detectable immune responses also include, *e.g.,* induction of interferon-induced protein with tetratricopeptide repeats 1 (*IFIT1*) mRNA.

The term "nucleic acid" as used herein refers to a polymer containing at least two deoxyribonucleotides or ribonucleotides in either single- or double-stranded form and includes DNA, RNA, and hybrids thereof. DNA may be in the form of, *e.g.,* antisense molecules, plasmid DNA, DNA-DNA duplexes, pre-condensed DNA, PCR products, vectors (P1, PAC, BAC, YAC, artificial chromosomes), expression cassettes, chimeric sequences, chromosomal DNA, or derivatives and combinations of these groups. RNA may be in the form of small interfering RNA (siRNA), Dicer-substrate dsRNA, small hairpin RNA (shRNA), asymmetrical interfering RNA (aiRNA), microRNA (miRNA), mRNA, tRNA, rRNA, tRNA, viral RNA (vRNA), and combinations thereof. Nucleic acids include nucleic acids containing known nucleotide analogs or modified backbone residues or linkages, which are synthetic, naturally occurring, and non-naturally occurring, and which have similar binding properties as the reference nucleic acid. Examples of such analogs include, without limitation, phosphorothioates, phosphoramidates, methyl phosphonates, chiral-methyl phosphonates, 2'-O-methyl ribonucleotides, and peptide-nucleic acids (PNAs). Unless specifically limited, the term encompasses nucleic acids containing known analogues of natural nucleotides that have similar binding properties as the reference nucleic acid. Unless otherwise indicated, a particular nucleic acid sequence also implicitly encompasses conservatively modified variants thereof (*e.g.,* degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res., 19:5081 (1991); Ohtsuka et al., J. Biol. Chem., 260:2605-2608 (1985); Rossolini et al., Mol. Cell. Probes, 8:91-98 (1994)). "Nucleotides" contain a sugar deoxyribose (DNA) or ribose (RNA), a base, and a phosphate group. Nucleotides are linked together through the phosphate groups. "Bases" include purines and pyrimidines, which further include natural compounds adenine, thymine, guanine, cytosine, uracil, inosine, and natural analogs, and synthetic derivatives of purines and pyrimidines, which include, but are not limited to, modifications which place new reactive groups such as, but not limited to, amines, alcohols, thiols, carboxylates, and alkylhalides.

The term "gene" refers to a nucleic acid (*e.g.,* DNA or RNA) sequence that comprises partial length or entire length coding sequences necessary for the production of a polypeptide or precursor polypeptide.

"Gene product," as used herein, refers to a product of a gene such as an RNA transcript or a polypeptide.

The term "lipid" refers to a group of organic compounds that include, but are not limited to, esters of fatty acids and are characterized by being insoluble in water, but soluble in many organic solvents. They are usually divided into at least three classes: (1) "simple lipids," which include fats and oils as well as waxes; (2) "compound lipids," which include phospholipids and glycolipids; and (3) "derived lipids" such as steroids.

The term "lipid particle" includes a lipid formulation that can be used to deliver an active agent or therapeutic agent, such as a nucleic acid (*e.g.,* interfering RNA) to a target site of interest (*e.g.,* cell, tissue, organ, tumor, and the like). In preferred embodiments, the lipid particle of the invention is a nucleic acid-lipid particle, which is typically formed from a cationic lipid, a non-cationic lipid, and optionally a conjugated lipid that prevents aggregation of the particle. In other preferred embodiments, the active agent or therapeutic agent, such as a nucleic acid (*e.g.,* interfering RNA), may be encapsulated in the lipid portion of the particle, thereby protecting it from enzymatic degradation.

As used herein, the term "SNALP" refers to a stable nucleic acid-lipid particle. A SNALP represents a particle made from lipids (*e.g.,* a cationic lipid, a non-cationic lipid, and optionally a conjugated lipid that prevents aggregation of the particle), wherein the nucleic acid (*e.g.,* an interfering RNA) is fully encapsulated within the lipid. In certain instances, SNALP are extremely useful for systemic applications, as they can exhibit extended circulation lifetimes following intravenous (i.v.) injection, they can accumulate at distal sites (*e.g.,* sites physically separated from the administration site), and they can mediate silencing of target gene expression at these distal sites. The nucleic acid may be complexed with a condensing agent and encapsulated within a SNALP as set forth in PCT Publication No. WO 00/03683.

The lipid particles of the invention (*e.g.,* SNALP) typically have a mean diameter of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm, and are substantially non-toxic. In addition, nucleic acids, when present in the lipid particles of the present invention, are resistant in aqueous solution to degradation with a nuclease. Nucleic acid-lipid particles and their method of preparation are disclosed in, *e.g.,* U.S. Patent Publication Nos. 20040142025 and 20070042031.

As used herein, "lipid encapsulated" can refer to a lipid particle that provides an active agent or therapeutic agent, such as a nucleic acid (*e.g.,* an interfering RNA such as an siRNA), with full encapsulation, partial encapsulation, or both. In a preferred embodiment, the nucleic acid (*e.g.,* interfering RNA) is fully encapsulated in the lipid particle (*e.g.,* to form a SNALP or other nucleic acid-lipid particle).

The term "lipid conjugate" refers to a conjugated lipid that inhibits aggregation of lipid particles. Such lipid conjugates include, but are not limited to, PEG-lipid conjugates such as, *e.g.,* PEG coupled to dialkyloxypropyls *(e.g.,* PEG-DAA conjugates), PEG coupled to diacylglycerols (*e.g.,* PEG-DAG conjugates), PEG coupled to cholesterol, PEG coupled to phosphatidylethanolamines, and PEG conjugated to ceramides (*see, e.g.,* U.S. Patent No. 5,885,613), cationic PEG lipids, polyoxazoline (POZ)-lipid conjugates (*e.g.,* POZ-DAA conjugates; *see, e.g.,* U.S. Publication No. 2011-0313017, filed January 13, 2011), polyamide oligomers (*e.g.,* ATTA-lipid conjugates), and mixtures thereof. Additional examples of POZ-lipid conjugates are described in PCT Publication No. WO 2010/006282. PEG or POZ can be conjugated directly to the lipid or may be linked to the lipid via a linker moiety. Any linker moiety suitable for coupling the PEG or the POZ to a lipid can be used including, *e.g.,* non-ester containing linker moieties and ester-containing linker moieties. In certain preferred embodiments, non-ester containing linker moieties, such as amides or carbamates, are used.

The term "amphipathic lipid" refers, in part, to any suitable material wherein the hydrophobic portion of the lipid material orients into a hydrophobic phase, while the hydrophilic portion orients toward the aqueous phase. Hydrophilic characteristics derive from the presence of polar or charged groups such as carbohydrates, phosphate, carboxylic, sulfato, amino, sulfhydryl, nitro, hydroxyl, and other like groups. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). Examples of amphipathic compounds include, but are not limited to, phospholipids, aminolipids, and sphingolipids.

Representative examples of phospholipids include, but are not limited to, phosphatidylcholine, phosphatidylethanolamine, phosphatidylserine, phosphatidylinositol, phosphatidic acid, palmitoyloleoyl phosphatidylcholine, lysophosphatidylcholine, lysophosphatidylethanolamine, dipalmitoylphosphatidylcholine, dioleoylphosphatidylcholine, distearoylphosphatidylcholine, and dilinoleoylphosphatidylcholine. Other compounds lacking in phosphorus, such as sphingolipid, glycosphingolipid families, diacylglycerols, and β-acyloxyacids, are also within the group designated as amphipathic lipids. Additionally, the amphipathic lipids described above can be mixed with other lipids including triglycerides and sterols.

The term "neutral lipid" refers to any of a number of lipid species that exist either in an uncharged or neutral zwitterionic form at a selected pH. At physiological pH, such lipids include, for example, diacylphosphatidylcholine, diacylphosphatidylethanolamine, ceramide, sphingomyelin, cephalin, cholesterol, cerebrosides, and diacylglycerols.

The term "non-cationic lipid" refers to any amphipathic lipid as well as any other neutral lipid or anionic lipid.

The term "anionic lipid" refers to any lipid that is negatively charged at physiological pH. These lipids include, but are not limited to, phosphatidylglycerols, cardiolipins, diacylphosphatidylserines, diacylphosphatidic acids, N-dodecanoyl phosphatidylethanolamines, N-succinyl phosphatidylethanolamines, N-glutarylphosphatidylethanolamines, lysylphosphatidylglycerols, palmitoyloleyolphosphatidylglycerol (POPG), and other anionic modifying groups joined to neutral lipids.

The term "hydrophobic lipid" refers to compounds having apolar groups that include, but are not limited to, long-chain saturated and unsaturated aliphatic hydrocarbon groups and such groups optionally substituted by one or more aromatic, cycloaliphatic, or heterocyclic group(s). Suitable examples include, but are not limited to, diacylglycerol, dialkylglycerol, N-N-dialkylamino, 1,2-diacyloxy-3-aminopropane, and 1,2-dialkyl-3-aminopropane.

The term "fusogenic" refers to the ability of a lipid particle, such as a SNALP, to fuse with the membranes of a cell. The membranes can be either the plasma membrane or membranes surrounding organelles, *e.g.,* endosome, nucleus, *etc.*

As used herein, the term "aqueous solution" refers to a composition comprising in whole, or in part, water.

As used herein, the term "organic lipid solution" refers to a composition comprising in whole, or in part, an organic solvent having a lipid.

"Distal site," as used herein, refers to a physically separated site, which is not limited to an adjacent capillary bed, but includes sites broadly distributed throughout an organism.

"Serum-stable" in relation to nucleic acid-lipid particles such as SNALP means that the particle is not significantly degraded after exposure to a serum or nuclease assay that would significantly degrade free DNA or RNA. Suitable assays include, for example, a standard serum assay, a DNAse assay, or an RNAse assay.

"Systemic delivery," as used herein, refers to delivery of lipid particles that leads to a broad biodistribution of an active agent such as an interfering RNA (*e.g.,* siRNA) within an organism. Some techniques of administration can lead to the systemic delivery of certain agents, but not others. Systemic delivery means that a useful, preferably therapeutic, amount of an agent is exposed to most parts of the body. To obtain broad biodistribution generally requires a blood lifetime such that the agent is not rapidly degraded or cleared (such as by first pass organs (liver, lung, *etc.*) or by rapid, nonspecific cell binding) before reaching a disease site distal to the site of administration. Systemic delivery of lipid particles can be by any means known in the art including, for example, intravenous, subcutaneous, and intraperitoneal. In a preferred embodiment, systemic delivery of lipid particles is by intravenous delivery.

"Local delivery," as used herein, refers to delivery of an active agent such as an interfering RNA (*e.g.,* siRNA) directly to a target site within an organism. For example, an agent can be locally delivered by direct injection into a disease site such as a tumor, other target site such as a site of inflammation, or a target organ such as the liver, heart, pancreas, kidney, and the like.

The term "mammal" refers to any mammalian species such as a human, mouse, rat, dog, cat, hamster, guinea pig, rabbit, livestock, and the like.

The term "cancer" refers to any member of a class of diseases characterized by the uncontrolled growth of aberrant cells. The term includes all known cancers and neoplastic conditions, whether characterized as malignant, benign, soft tissue, or solid, and cancers of all stages and grades including pre- and post-metastatic cancers. Examples of different types of cancer include, but are not limited to, liver cancer, lung cancer, colon cancer, rectal cancer, anal cancer, bile duct cancer, small intestine cancer, stomach (gastric) cancer, esophageal cancer; gallbladder cancer, pancreatic cancer, appendix cancer, breast cancer, ovarian cancer; cervical cancer, prostate cancer, renal cancer (*e.g.,* renal cell carcinoma), cancer of the central nervous system, glioblastoma, skin cancer, lymphomas, choriocarcinomas, head and neck cancers, osteogenic sarcomas, and blood cancers. Non-limiting examples of specific types of liver cancer include hepatocellular carcinoma (HCC), secondary liver cancer (*e.g.,* caused by metastasis of some other non-liver cancer cell type), and hepatoblastoma. As used herein, a "tumor" comprises one or more cancerous cells.

### III. Novel Cationic Lipids

The present invention provides, *inter alia,* lipid particles comprising novel cationic (amino) lipids that can advantageously be used in the lipid particles described herein for the *in vitro* and/or *in vivo* delivery of therapeutic agents such as nucleic acids to cells. The novel cationic lipids have the structure set forth in Formula I herein, and include the (R) and/or (S) enantiomers thereof.

In some embodiments, a lipid of the present invention comprises a racemic mixture. In other embodiments, a lipid of the present invention comprises a mixture of one or more diastereomers. In certain embodiments, a lipid of the present invention is enriched in one enantiomer, such that the lipid comprises at least about 55%, 60%, 65%, 70%, 75%, 80%, 85%, 90%, or 95% enantiomeric excess. In certain other embodiments, a lipid of the present invention is enriched in one diastereomer, such that the lipid comprises at least about 55%, 60%, 65%, 70%, 75%, 80%, 85%, 90%, or 95% diastereomeric excess. In certain additional embodiments, a lipid of the present invention is chirally pure (*e.g.,* comprises a single optical isomer). In further embodiments, a lipid of the present invention is enriched in one optical isomer (*e.g.,* an optically active isomer), such that the lipid comprises at least about 55%, 60%, 65%, 70%, 75%, 80%, 85%, 90%, or 95% isomeric excess. The present invention provides the synthesis of the cationic lipids of Formula I as a racemic mixture or in optically pure form.

The terms "cationic lipid" and "amino lipid" are used interchangeably herein to include those lipids and salts thereof having one, two, three, or more fatty acid or fatty alkyl chains and a pH-titratable amino head group (*e.g.,* an alkylamino or dialkylamino head group). The cationic lipid is typically protonated (*i.e.,* positively charged) at a pH below the pKₐ of the cationic lipid and is substantially neutral at a pH above the pKₐ. The cationic lipids of the invention may also be termed titratable cationic lipids.

The term "salts" includes any anionic and cationic complex, such as the complex formed between a cationic lipid disclosed herein and one or more anions. Non-limiting examples of anions include inorganic and organic anions, *e.g.,* hydride, fluoride, chloride, bromide, iodide, oxalate (*e.g.,* hemioxalate), phosphate, phosphonate, hydrogen phosphate, dihydrogen phosphate, oxide, carbonate, bicarbonate, nitrate, nitrite, nitride, bisulfite, sulfide, sulfite, bisulfate, sulfate, thiosulfate, hydrogen sulfate, borate, formate, acetate, benzoate, citrate, tartrate, lactate, acrylate, polyacrylate, fumarate, maleate, itaconate, glycolate, gluconate, malate, mandelate, tiglate, ascorbate, salicylate, polymethacrylate, perchlorate, chlorate, chlorite, hypochlorite, bromate, hypobromite, iodate, an alkylsulfonate, an arylsulfonate, arsenate, arsenite, chromate, dichromate, cyanide, cyanate, thiocyanate, hydroxide, peroxide, permanganate, and mixtures thereof. In particular embodiments, the salts of the cationic lipids disclosed herein are crystalline salts.

The term "alkyl" includes a straight chain or or branched saturated aliphatic hydrocarbon containing from 1 to 24 carbon atoms. Representative saturated straight chain alkyls include, but are not limited to, methyl, ethyl, *n*-propyl, *n*-butyl, *n*-pentyl, *n*-hexyl, and the like, while saturated branched alkyls include, without limitation, isopropyl, *sec*-butyl, isobutyl, *tert*-butyl, isopentyl, and the like.

The term "heteroalkyl," includes a straight chain or branched saturated aliphatic hydrocarbon as defined above having from about 1 to about 5 heteroatoms *(i.e.,* 1, 2, 3, 4, or 5 heteroatoms) such as, for example, O, N, Si, and/or S, wherein the nitrogen and sulfur atoms may optionally be oxidized and the nitrogen heteroatom may optionally be quaternized. The heteroalkyl group can be attached to the remainder of the molecule through a carbon atom or a heteroatom.

The term "cyclic alkyl" includes any of the substituted or unsubstituted cycloalkyl, heterocycloalkyl, cycloalkenyl, and heterocycloalkenyl groups described below.

The term "cycloalkyl" includes a substituted or unsubstituted cyclic alkyl group having from about 3 to about 8 carbon atoms (*i.e.,* 3, 4, 5, 6, 7, or 8 carbon atoms) as ring vertices. Preferred cycloalkyl groups include those having from about 3 to about 6 carbon atoms as ring vertices. Examples of C₃₋₈ cycloalkyl groups include, but are not limited to, cyclopropyl, methyl-cyclopropyl, dimethyl-cyclopropyl, cyclobutyl, methyl-cyclobutyl, cyclopentyl, methyl-cyclopentyl, cyclohexyl, methyl-cyclohexyl, dimethyl-cyclohexyl, cycloheptyl, and cyclooctyl, as well as other substituted C₃₋₈ cycloalkyl groups.

The term "heterocycloalkyl" includes a substituted or unsubstituted cyclic alkyl group as defined above having from about 1 to about 3 heteroatoms as ring members selected from the group consisting of O, N, Si and S, wherein the nitrogen and sulfur atoms may optionally be oxidized and the nitrogen heteroatom may optionally be quaternized. The heterocycloalkyl group can be attached to the remainder of the molecule through a carbon atom or a heteroatom.

The term "cycloalkenyl" includes a substituted or unsubstituted cyclic alkenyl group having from about 3 to about 8 carbon atoms (*i.e.,* 3, 4, 5, 6, 7, or 8 carbon atoms) as ring vertices. Preferred cycloalkenyl groups are those having from about 3 to about 6 carbon atoms as ring vertices. Examples of C₃₋₈ cycloalkenyl groups include, but are not limited to, cyclopropenyl, methyl-cyclopropenyl, dimethyl-cyclopropenyl, cyclobutenyl, cyclopentenyl, cyclohexenyl, cycloheptenyl, and cyclooctenyl, as well as other substituted C₃₋₈ cycloalkenyl groups.

The term "heterocycloalkenyl" includes a substituted or unsubstituted cyclic alkenyl group as defined above having from about 1 to about 3 heteroatoms as ring members selected from the group consisting of O, N, Si and S, wherein the nitrogen and sulfur atoms may optionally be oxidized and the nitrogen heteroatom may optionally be quaternized. The heterocycloalkenyl group can be attached to the remainder of the molecule through a carbon atom or a heteroatom.

The term "alkoxy" includes a group of the formula alkyl-O-, wherein "alkyl" has the previously given definition. Non-limiting examples of alkoxy groups include methoxy, ethoxy, *n*-propoxy, *iso*-propoxy, *n*-butoxy, *iso*-butoxy, sec-butoxy and *tert*-butoxy.

The term "alkenyl" includes an alkyl, as defined above, containing at least one double bond between adjacent carbon atoms. Alkenyls include both *cis* and *trans* isomers. Representative straight chain and branched alkenyls include, but are not limited to, ethylenyl, propylenyl, 1-butenyl, 2-butenyl, isobutylenyl, 1-pentenyl, 2-pentenyl, 3-methyl-1-butenyl, 2-methyl-2-butenyl, 2,3-dimethyl-2-butenyl, and the like. Representative cyclic alkenyls are described above.

The term "alkynyl" includes any alkyl or alkenyl, as defined above, which additionally contains at least one triple bond between adjacent carbons. Representative straight chain and branched alkynyls include, without limitation, acetylenyl, propynyl, 1-butynyl, 2-butynyl, 1-pentynyl, 2-pentynyl, 3-methyl-1 butynyl, and the like.

The term "aryl" includes a polyunsaturated, typically aromatic, hydrocarbon group which can be a single ring or multiple rings (up to three rings) which are fused together or linked covalently, and which optionally carries one or more substituents, such as, for example, halogen, trifluoromethyl, amino, alkyl, alkoxy, alkylcarbonyl, cyano, carbamoyl, alkoxycarbamoyl, methylendioxy, carboxy, alkoxycarbonyl, aminocarbonyl, alkylaminocarbonyl, dialkylaminocarbonyl, hydroxy, nitro, and the like. Non-limiting examples of unsubstituted aryl groups include phenyl, naphthyl, and biphenyl. Examples of substituted aryl groups include, but are not limited to, phenyl, chlorophenyl, trifluoromethylphenyl, chlorofluorophenyl, and aminophenyl.

The terms "alkylthio," "alkylsulfonyl," "alkylsulfinyl," and "arylsulfonyl" include groups having the formula -S-Rⁱ, -S(O)₂-Rⁱ, -S(O)-Rⁱ and -S(O)₂R^{j}, respectively, wherein Rⁱ is an alkyl group as previously defined and R^{J} is an aryl group as previously defined.

The terms "alkenyloxy" and "alkynyloxy" include groups having the formula -O-Rⁱ, wherein Rⁱ is an alkenyl or alkynyl group, respectively.

The terms "alkenylthio" and "alkynylthio" include groups having the formula -S-R^{k}, wherein R^{k} is an alkenyl or alkynyl group, respectively.

The term "alkoxycarbonyl" includes a group having the formula -C(O)O-Rⁱ, wherein Rⁱ is an alkyl group as defined above and wherein the total number of carbon atoms refers to the combined alkyl and carbonyl moieties.

The term "acyl" includes any alkyl, alkenyl, or alkynyl wherein the carbon at the point of attachment is substituted with an oxo group, as defined below. The following are non-limiting examples of acyl groups: -C(=O)alkyl, -C(=O)alkenyl, and -C(=O)alkynyl.

The term "heterocycle" includes a 5- to 7-membered monocyclic, or 7- to 10-membered bicyclic, heterocyclic ring which is either saturated, unsaturated, or aromatic, and which contains one, two, three, or more heteroatoms independently selected from nitrogen (N), oxygen (O), and sulfur (S), and wherein the nitrogen and sulfur heteroatoms may be optionally oxidized, and the nitrogen heteroatom may be optionally quaternized, including bicyclic rings in which any of the above heterocycles are fused to a benzene ring. The heterocycle may be attached via any heteroatom or carbon atom. Heterocycles include, but are not limited to, heteroaryls as defined below, as well as morpholinyl, pyrrolidinonyl, pyrrolidinyl, piperidinyl, piperizynyl, hydantoinyl, valerolactamyl, oxiranyl, oxetanyl, tetrahydrofuranyl, tetrahydropyranyl, tetrahydropyridinyl, tetrahydroprimidinyl, tetrahydrothiophenyl, tetrahydrothiopyranyl, tetrahydropyrimidinyl, tetrahydrothiophenyl, tetrahydrothiopyranyl, and the like.

The term "heteroaryl" includes an aromatic 5- to 10-membered heterocycle which contains one, two, three, or more heteroatoms selected from nitrogen (N), oxygen (O), and sulfur (S). The heteroaryl can be substituted on one or more carbon atoms with substituents such as, for example, halogen, alkyl, alkoxy, cyano, haloalkyl (*e.g.,* trifluoromethyl), heterocyclyl (*e.g.,* morpholinyl or pyrrolidinyl), and the like. Non-limiting examples of heteroaryls include pyridinyl and furanyl.

The term "halogen" includes fluoro, chloro, bromo, and iodo.

The terms "optionally substituted alkyl," "optionally substituted cyclic alkyl," "optionally substituted alkenyl," "optionally substituted alkynyl," "optionally substituted acyl," and "optionally substituted heterocycle" mean that, when substituted, at least one hydrogen atom is replaced with a substituent. In the case of an "oxo" substituent (=O), two hydrogen atoms are replaced. Non-limiting examples of substituents include oxo, halogen, heterocycle, -CN, -OR^{x}, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}, wherein n is 0, 1, or 2, R^{x} and R^{y} are the same or different and are independently hydrogen, alkyl, or heterocycle, and each of the alkyl and heterocycle substituents may be further substituted with one or more of oxo, halogen, -OH, -CN, alkyl, -OR^{x}, heterocycle, -NR^{x}R^{y}, -NR^{x}C(=O)R^{y}, -NR^{x}SO₂R^{y}, -C(=O)R^{x}, -C(=O)OR^{x}, -C(=O)NR^{x}R^{y}, -SOₙR^{x}, and -SOₙNR^{x}R^{y}. The term "optionally substituted," when used before a list of substituents, means that each of the substituents in the list may be optionally substituted as described herein.

Described herein is a cationic lipid of Formula I having the following structure: 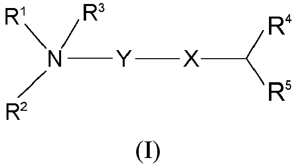 or salts thereof, wherein:
R¹ and R² are either the same or different and are independently hydrogen (H) or an optionally substituted C₁-C₆ alkyl, C₂-C₆ alkenyl, or C₂-C₆ alkynyl, or R¹ and R² may join to form an optionally substituted heterocyclic ring;
R³ is either absent or is hydrogen (H) or a C₁-C₆ alkyl to provide a quaternary amine;
R⁴ and R⁵ are either the same or different and are independently an optionally substituted C₁₀-C₂₄ alkyl, C₁₀-C₂₄ alkenyl, C₁₀-C₂₄ alkynyl, or C₁₀-C₂₄ acyl;
X is O, S, N(R⁶), C(O), C(O)O, OC(O), C(O)N(R ⁶), N(R⁶)C(O), OC(O)N(R⁶), N(R⁶)C(O)O, C(O)S, C(S)O, S(O), S(O)(O), C(S), or an optionally substituted heterocyclic ring, wherein R⁶ is hydrogen (H) or an optionally substituted C₁-C₁₀ alkyl, C₂-C₁₀ alkenyl, or C₂-C₁₀ alkynyl; and
Y is either absent or is an optionally substituted C₁-C₆ alkyl, C₂-C₆ alkenyl, or C₂-C₆ alkynyl.

In some embodiments, R¹ and R² are each independently hydrogen (H) or an optionally substituted C₁-C₂ alkyl, C₁-C₃ alkyl, C₁-C₄ alkyl, C₁-C₅ alkyl, C₂-C₃ alkyl, C₂-C₄ alkyl, C₂-C₅ alkyl, C₂-C₆ alkyl, C₃-C₄ alkyl, C₃-C₅ alkyl, C₃-C₆ alkyl, C₄-C₅ alkyl, C₄-C₆ alkyl, C₅-C₆ alkyl, C₂-C₃ alkenyl, C₂-C₄ alkenyl, C₂-C₅ alkenyl, C₂-C₆ alkenyl, C₃-C₄ alkenyl, C₃-C₅ alkenyl, C₃-C₆ alkenyl, C₄-C₅ alkenyl, C₄-C₆ alkenyl, C₅-C₆ alkenyl, C₂-C₃ alkynyl, C₂-C₄ alkynyl, C₂-C₅ alkynyl, C₂-C₆ alkynyl, C₃-C₄ alkynyl, C₃-C₅ alkynyl, C₃-C₆ alkynyl, C₄-C₅ alkynyl, C₄-C₆ alkynyl, or C₅-C₆ alkynyl. In particular embodiments, R¹ and R² are both methyl groups, both ethyl groups, or a combination of one methyl group and one ethyl group. In certain instances, R³ is absent when the pH is above the pKₐ of the cationic lipid and R³ is hydrogen (H) when the pH is below the pKₐ of the cationic lipid such that the amino head group is protonated. In certain other instances, R³ is an optionally substituted C₁-C₂ alkyl, C₁-C₃ alkyl, C₁-C₄ alkyl, C₁-C₅ alkyl, C₂-C₃ alkyl, C₂-C₄ alkyl, C₂-C₅ alkyl, C₂-C₆ alkyl, C₃-C₄ alkyl, C₃-C₅ alkyl, C₃-C₆ alkyl, C₄-C₅ alkyl, C₄-C₆ alkyl, or C₅-C₆ alkyl to provide a quaternary amine.

In certain embodiments, R¹ and R² are joined to form an optionally substituted heterocyclic ring comprising 1, 2, 3, 4, 5, 6, or more carbon atoms and 1, 2, 3, 4, or more heteroatoms such as nitrogen (N), oxygen (O), sulfur (S), and mixtures thereof. In some embodiments, the optionally substituted heterocyclic ring comprises from 2 to 5 carbon atoms and from 1 to 3 heteroatoms such as nitrogen (N), oxygen (O), and/or sulfur (S). In certain embodiments, the heterocyclic ring comprises an optionally substituted imidazole, triazole (*e.g.,* 1,2,3-triazole, 1,2,4-triazole), pyrazole, thiazole, pyrrole, furan, oxazole, isoxazole, oxazoline, oxazolidine, oxadiazole, tetrazole, and the like. In some instances, the optionally substituted heterocyclic ring comprises 5 carbon atoms and 1 nitrogen atom, wherein the heterocyclic ring can be substituted with a substituent such as a hydroxyl (-OH) group at the ortho, meta, and/or para positions. In certain instances, the heterocyclic ring comprises an optionally substituted imidazole group.

In other embodiments, R⁶ is hydrogen (H) or an optionally substituted C₁-C₂ alkyl, C₁-C₃ alkyl, C₁-C₄ alkyl, C₁-C₅ alkyl, C₁-C₆ alkyl, C₁-C₇ alkyl, C₁-C₈ alkyl, C₁-C₉ alkyl, C₂-C₃ alkyl, C₂-C₄ alkyl, C₂-C₅ alkyl, C₂-C₆ alkyl, C₂-C₇ alkyl, C₂-C₈ alkyl, C₂-C₉ alkyl, C₂-C₁₀ alkyl, C₃-C₄ alkyl, C₃-C₅ alkyl, C₃-C₆ alkyl, C₃-C₇ alkyl, C₃-C₈ alkyl, C₃-C₉ alkyl, C₃-C₁₀ alkyl, C₄-C₅ alkyl, C₄-C₆ alkyl, C₄-C₇ alkyl, C₄-C₈ alkyl, C₄-C₉ alkyl, C₄-C₁₀ alkyl, C₅-C₆ alkyl, C₅-C₇ alkyl, C₅-C₈ alkyl, C₅-C₉ alkyl, C₅-C₁₀ alkyl, C₆-C₇ alkyl, C₆-C₈ alkyl, C₆-C₉ alkyl, C₆-C₁₀ alkyl, C₇-C₈ alkyl, C₇-C₉ alkyl, C₇-C₁₀ alkyl, C₈-C₉ alkyl, C₈-C₁₀ alkyl, C₉-C₁₀ alkyl, C₂-C₃ alkenyl, C₂-C₄ alkenyl, C₂-C₅ alkenyl, C₂-C₆ alkenyl, C₂-C₇ alkenyl, C₂-C₈ alkenyl, C₂-C₉ alkenyl, C₃-C₄ alkenyl, C₃-C₅ alkenyl, C₃-C₆ alkenyl, C₃-C₇ alkenyl, C₃-C₈ alkenyl, C₃-C₉ alkenyl, C₃-C₁₀ alkenyl, C₄-C₅ alkenyl, C₄-C₆ alkenyl, C₄-C₇ alkenyl, C₄-C₈ alkenyl, C₄-C₉ alkenyl, C₄-C₁₀ alkenyl, C₅-C₆ alkenyl, C₅-C₇ alkenyl, C₅-C₈ alkenyl, C₅-C₉ alkenyl, C₅-C₁₀ alkenyl, C₆-C₇ alkenyl, C₆-C₈ alkenyl, C₆-C₉ alkenyl, C₆-C₁₀ alkenyl, C₇-C₈ alkenyl, C₇-C₉ alkenyl, C₇-C₁₀ alkenyl, C₈-C₉ alkenyl, C₈-C₁₀ alkenyl, C₉-C₁₀ alkenyl, C₂-C₃ alkynyl, C₂-C₄ alkynyl, C₂-C₅ alkynyl, C₂-C₆ alkynyl, C₂-C₇ alkynyl, C₂-C₈ alkynyl, C₂-C₉ alkynyl, C₃-C₄ alkynyl, C₃-C₅ alkynyl, C₃-C₆ alkynyl, C₃-C₇ alkynyl, C₃-C₈ alkynyl, C₃-C₉ alkynyl, C₃-C₁₀ alkynyl, C₄-C₅ alkynyl, C₄-C₆ alkynyl, C₄-C₇ alkynyl, C₄-C₈ alkynyl, C₄-C₉ alkynyl, C₄-C₁₀ alkynyl, C₅-C₆ alkynyl, C₅-C₇ alkynyl, C₅-C₈ alkynyl, C₅-C₉ alkynyl, C₅-C₁₀ alkynyl, C₆-C₇ alkynyl, C₆-C₈ alkynyl, C₆-C₉ alkynyl, C₆-C₁₀ alkynyl, C₇-C₈ alkynyl, C₇-C₉ alkynyl, C₇-C₁₀ alkynyl, C₈-C₉ alkynyl, C₈-C₁₀ alkynyl, or C₉-C₁₀ alkynyl. In one particular embodiment, R⁶ is selected from the group consisting of hydrogen (H), a C₁ alkyl (methyl) group, and a C₂, C₃, C₄, C₅, C₆, C₇, C₈, C₉, and C₁₀ alkyl, alkenyl, and alkynyl group.

In one particular case, X is C(O)O. In another particular case, X is O. In certain embodiments, X is C(O)N(R ⁶), N(R⁶)C(O)O, or C(O)S. In one particular embodiment, X is N(R⁶)C(O)O and R⁶ is hydrogen (H), a methyl group, or a C₂, C₃, C₄, C₅, C₆, C₇, C₈, C₉, or C₁₀ alkyl, alkenyl, or alkynyl group. In certain other embodiments, X is an optionally substituted heterocyclic ring. In particular embodiments, the heterocyclic ring comprises 1, 2, 3, 4, 5, 6, or more carbon atoms and 1, 2, 3, 4, or more heteroatoms such as nitrogen (N), oxygen (O), sulfur (S), and mixtures thereof. In some embodiments, the optionally substituted heterocyclic ring comprises from 2 to 5 carbon atoms and from 1 to 3 heteroatoms such as nitrogen (N), oxygen (O), and/or sulfur (S). In certain embodiments, the heterocyclic ring comprises an optionally substituted imidazole, triazole (*e.g.,* 1,2,3-triazole, 1,2,4-triazole), pyrazole, thiazole, pyrrole, furan, oxazole, isoxazole, oxazoline, oxazolidine, oxadiazole, tetrazole, and the like. In certain instances, R¹ and R² are not both methyl groups when X is C(O)O, Y is (CH₂)₂ or (CH₂)₃, and R⁴ and R⁵ are both linoleyl moieties.

In certain cases, at least one or both R⁴ and R⁵ independently comprises an optionally substituted C12-C24, C12-C22, C12-C20, C14-C24, C14-C22, C14-C20, C16-C24, C16-C22, or C₁₆-C₂₀ alkyl, alkenyl, alkynyl, or acyl group (*i.e.,* C₁₂, C₁₃, C₁₄, C₁₅, C₁₆, C₁₇, C₁₈, C₁₉, C₂₀, C₂₁, C₂₂, C₂₃, or C₂₄ alkyl, alkenyl, alkynyl, or acyl group). In other cases, at least one or both R⁴ and R⁵ independently comprises at least 1, 2, 3, 4, 5, or 6 sites of unsaturation (*e.g.,* 1-2, 1-3, 1-4, 1-5, 1-6, 2-3, 2-4, 2-5, or 2-6 sites of unsaturation) or a substituted alkyl or acyl group. In certain instances, the unsaturated side-chain comprises a dodecenyl moiety, a tetradecenyl (*e.g.,* myristoleyl) moiety, a hexadecenyl (*e.g.,* palmitoleyl) moiety, an octadecenyl (*e.g.,* oleyl) moiety, an icosenyl moiety, a dodecadienyl moiety, a tetradecadienyl moiety, a hexadecadienyl moiety, an octadecadienyl moiety, an icosadienyl moiety, a dodecatrienyl moiety, a tetradectrienyl moiety, a hexadecatrienyl moiety, an octadecatrienyl moiety, an icosatrienyl moiety, or an acyl derivative thereof (*e.g.,* oleoyl, linoleoyl, linolenoyl, γ-linolenoyl, *etc.*)*.* In some instances, the octadecadienyl moiety is a linoleyl moiety. In particular cases, R⁴ and R⁵ are both linoleyl moieties. In other instances, the octadecatrienyl moiety is a linolenyl moiety or a γ-linolenyl moiety. In particular cases, R⁴ and R⁵ are both linolenyl moieties or γ-linolenyl moieties. In cases where one or both R⁴ and R⁵ independently comprises a branched alkyl or acyl group (*e.g.,* a substituted alkyl or acyl group), the branched alkyl or acyl group may comprise a C₁₂-C₂₄ alkyl or acyl having at least 1-6 (*e.g.,* 1, 2, 3, 4, 5, 6, or more) C₁-C₆ alkyl substituents. In particular cases, the branched alkyl or acyl group comprises a C₁₂-C₂₀ or C₁₄-C₂₂ alkyl or acyl with 1-6 (*e.g.,* 1, 2, 3, 4, 5, 6) C₁-C₄ alkyl (*e.g.,* methyl, ethyl, propyl, or butyl) substituents. In some cases, the branched alkyl group comprises a phytanyl (3,7,11,15-tetramethyl-hexadecanyl) moiety and the branched acyl group comprises a phytanoyl (3,7,11,15-tetramethyl-hexadecanoyl) moiety. In particular cases, R⁴ and R⁵ are both phytanyl moieties. In further cases, at least one or both R⁴ and R⁵ are independently substituted with one, two, three, four, or more substituents such as oxo (=O) substituents, -OR^{x} substituents, and combinations thereof, wherein each R^{x} is independently hydrogen or an alkyl group. In certain instances, an oxo (=O) or -OR^{x} (*e.g.,* - OH) substituent is present in one or both R⁴ and R⁵ at the carbon which attaches R⁴ or R⁵ to the remainder of the compound.

In some cases, the 1, 2, 3, 4, 5, 6, or more sites of unsaturation present in one or both R⁴ and R⁵ correspond to, in each instance, *cis* double bonds, *trans* double bonds, or combinations thereof, at specific positions in one or both of the unsaturated side-chains. For those unsaturated side-chains where a double bond is located between hydrogen atoms and alkyl or alkylene chains, the chemical notation "E" refers to the *trans* double bond configuration and the chemical notation "Z" refers to the *cis* double bond configuration. As non-limiting examples, one or both R⁴ and R⁵ are C₁₈ alkyl groups containing any combination of double bonds in the *cis* and/or *trans* configuration at one or more positions in the side-chain (*e.g., cis* and/or *trans* double bonds at position 9, at positions 6 and 9, at positions 3, 6, and 9, at positions 6, 9, and 12, or at positions 7 and 9 of a C₁₈ alkyl group). Similarly, as non-limiting examples, one or both R⁴ and R⁵ are C₁₈ alkyl groups containing any combination of double bonds which can be characterized by either the "E" chemical notation and/or the "Z" chemical notation at one or more positions in the side-chain (*e.g.,* "Z" and/or "E" double bonds at position 9, at positions 6 and 9, at positions 3, 6, and 9, at positions 6, 9, and 12, or at positions 7 and 9 of a C₁₈ alkyl group).

In other cases, one of R⁴ or R⁵ independently comprises at least 1, 2, 3, 4, 5, 6, or more optionally substituted cyclic alkyl groups (*e.g.,* 1-2, 1-3, 1-4, 1-5, 1-6, 2-3, 2-4, 2-5, or 2-6 optionally substituted cyclic alkyl groups). In certain instances, one of R⁴ or R⁵ independently comprises an optionally substituted C₁₂-C₂₄, C₁₂-C₂₂, C₁₂-C₂₀, C₁₄-C₂₄, C₁₄-C₂₂, C₁₄-C₂₀, C₁₆-C₂₄, C₁₆-C₂₂, or C₁₆-C₂₀ alkyl, alkenyl, alkynyl, or acyl group (*i.e.,* C₁₂, C₁₃, C₁₄, C₁₅, C₁₆, C₁₇, C₁₈, C₁₉, C₂₀, C₂₁, C₂₂, C₂₃, or C₂₄ alkyl, alkenyl, alkynyl, or acyl group), and the other of R⁴ or R⁵ comprises at least 1, 2, 3, 4, 5, or 6 optionally substituted cyclic alkyl groups (*e.g.,* 1-2, 1-3, 1-4, 1-5, 1-6, 2-3, 2-4, 2-5, or 2-6 optionally substituted cyclic alkyl groups).

In particular cases, one or more of the optionally substituted cyclic alkyl groups present in one of R⁴ and R⁵ are independently selected from the group consisting of an optionally substituted saturated cyclic alkyl group, an optionally substituted unsaturated cyclic alkyl group, and combinations thereof. In certain instances, the optionally substituted saturated cyclic alkyl group comprises an optionally substituted C₃₋₈ cycloalkyl group (*e.g.,* cyclopropyl, cyclobutyl, cyclopentyl, cyclohexyl, cycloheptyl, cyclooctyl, *etc.*)*.* In preferred cases, the optionally substituted saturated cyclic alkyl group comprises a cyclopropyl group, optionally containing one or more substituents and/or heteroatoms. In other instances, the optionally substituted unsaturated cyclic alkyl group comprises an optionally substituted C₃₋₈ cycloalkenyl group (*e.g.,* cyclopropenyl, cyclobutenyl, cyclopentenyl, cyclohexenyl, cycloheptenyl, cyclooctenyl, *etc.*)*.*

In some cases, one of R⁴ or R⁵ comprises at least 1, 2, 3, 4, 5, or 6 sites of unsaturation (*e.g.,* 1-2, 1-3, 1-4, 1-5, 1-6, 2-3, 2-4, 2-5, or 2-6 sites of unsaturation) or a substituted alkyl or acyl group, and the other side-chain comprises at least 1, 2, 3, 4, 5, or 6 optionally substituted cyclic alkyl groups (*e.g.,* 1-2, 1-3, 1-4, 1-5, 1-6, 2-3, 2-4, 2-5, or 2-6 optionally substituted cyclic alkyl groups). In cases where one of R⁴ or R⁵ comprises at least 1, 2, 3, 4, 5, or 6 sites of unsaturation, the unsaturated side-chain may comprise a myristoleyl moiety, a palmitoleyl moiety, an oleyl moiety, a dodecadienyl moiety, a tetradecadienyl moiety, a hexadecadienyl moiety, an octadecadienyl moiety, an icosadienyl moiety, a dodecatrienyl moiety, a tetradectrienyl moiety, a hexadecatrienyl moiety, an octadecatrienyl moiety, an icosatrienyl moiety, or an acyl derivative thereof (*e.g.,* linoleoyl, linolenoyl, γ-linolenoyl, *etc.*)*.* In some instances, the octadecadienyl moiety is a linoleyl moiety. In other instances, the octadecatrienyl moiety is a linolenyl moiety or a γ-linolenyl moiety. In cases where one of R⁴ or R⁵ comprises a branched alkyl or acyl group (*e.g.,* a substituted alkyl or acyl group), the branched alkyl or acyl group may comprise a C₁₂-C₂₄ alkyl or acyl having at least 1-6 (*e.g.,* 1, 2, 3, 4, 5, 6, or more) C₁-C₆ alkyl substituents. In particular cases, the branched alkyl or acyl group comprises a C₁₂-C₂₀ or C₁₄-C₂₂ alkyl or acyl with 1-6 (*e.g.,* 1, 2, 3, 4, 5, 6) C₁-C₄ alkyl (*e.g.,* methyl, ethyl, propyl, or butyl) substituents. In some cases, the branched alkyl group comprises a phytanyl moiety and the branched acyl group comprises a phytanoyl moiety.

In preferred cases, the optionally substituted cyclic alkyl groups present in one of R⁴ or R⁵ are located at the site(s) of unsaturation in the corresponding unsaturated side-chain. As a non-limiting example, one of R⁴ of R⁵ is a C₁₈ alkyl group having 1, 2, or 3 optionally substituted cyclic alkyl groups, wherein the optionally substituted cyclic alkyl groups (*e.g.,* independently selected cyclopropyl groups) are located at one or more (*e.g.,* all) of the sites of unsaturation present in a corresponding linoleyl moiety, linolenyl moiety, or γ-linolenyl moiety.

In alternative cases to the cationic lipid of Formula I, R⁴ and R⁵ are different and are independently an optionally substituted C₁-C₂₄ alkyl, C₂-C₂₄ alkenyl, C₂-C₂₄ alkynyl, or C₁-C₂₄ acyl. In particular cases, one of R⁴ or R⁵ comprises at least 1, 2, 3, 4, 5, or 6 optionally substituted cyclic alkyl groups (*e.g.,* 1-2, 1-3, 1-4, 1-5, 1-6, 2-3, 2-4, 2-5, or 2-6 optionally substituted cyclic alkyl groups). In certain cases, R⁴ and R⁵ are different and are independently an optionally substituted C₄-C₂₀ alkyl, C₄-C₂₀ alkenyl, C₄-C₂₀ alkynyl, or C₄-C₂₀ acyl. In some instances, R⁴ is an optionally substituted C₁₂-C₂₄ alkyl, C₁₂-C₂₄ alkenyl, C₁₂-C₂₄ alkynyl, or C₁₂-C₂₄ acyl, and R⁵ is an optionally substituted C₄-C₁₀ alkyl, C₄-C₁₀ alkenyl, C₄-C₁₀ alkynyl, or C₄-C₁₀ acyl. In other instances, R⁴ is an optionally substituted C12-C20 or C14-C22 alkyl, C12-C20 or C14-C22 alkenyl, C12-C20 or C14-C22 alkynyl, or C12-C20 or C₁₄-C₂₂ acyl, and R⁵ is an optionally substituted C₄-C₈ or C₆ alkyl, C₄-C₈ or C₆ alkenyl, C₄-C₈ or C₆ alkynyl, or C₄-C₈ or C₆ acyl. In certain instances, R⁴ is an optionally substituted C₄-C₁₀ alkyl, C₄-C₁₀ alkenyl, C₄-C₁₀ alkynyl, or C₄-C₁₀ acyl, and R⁵ is an optionally substituted C₁₂-C₂₄ alkyl, C₁₂-C₂₄ alkenyl, C₁₂-C₂₄ alkynyl, or C₁₂-C₂₄ acyl. In certain other instances, R⁴ is an optionally substituted C₄-C₈ or C₆ alkyl, C₄-C₈ or C₆ alkenyl, C₄-C₈ or C₆ alkynyl, or C₄-C₈ or C₆ acyl, and R⁵ is an optionally substituted C₁₂-C₂₀ or C₁₄-C₂₂ alkyl, C₁₂-C₂₀ or C₁₄-C₂₂ alkenyl, C₁₂-C₂₀ or C₁₄-C₂₂ alkynyl, or C₁₂-C₂₀ or C₁₄-C₂₂ acyl. In particular cases, one or more of the optionally substituted cyclic alkyl groups, when present in one of R⁴ or R⁵, are as described above.

In some groups of cases to the cationic lipid of Formula I, R⁴ and R⁵ are either the same or different and are independently selected from the group consisting of:

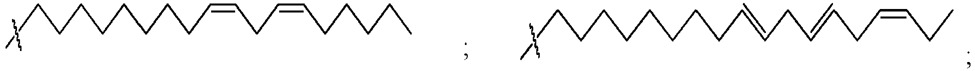

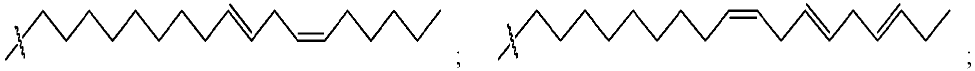

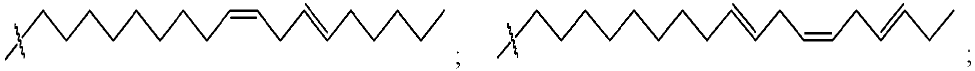

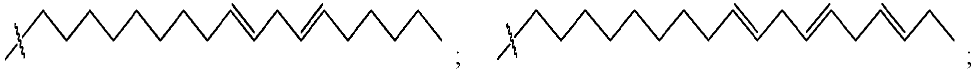

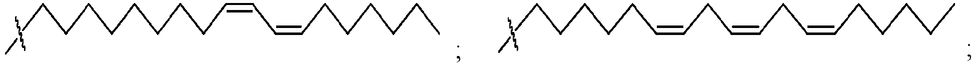

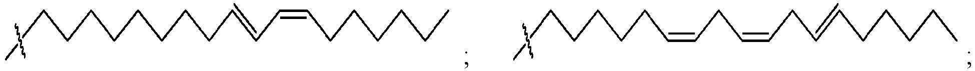

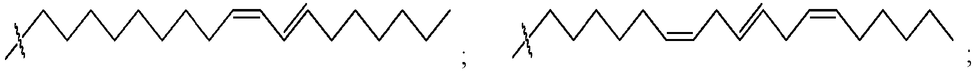

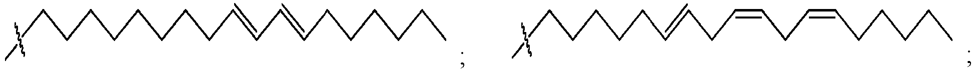

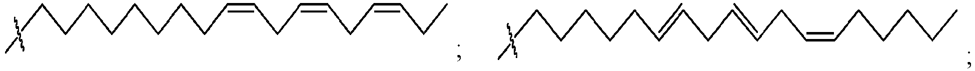

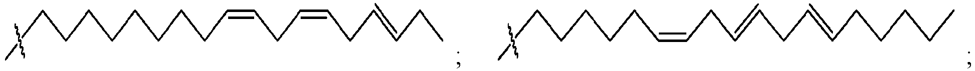

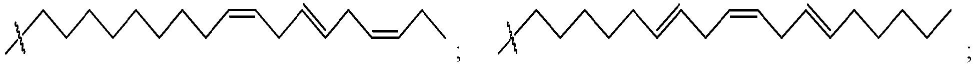

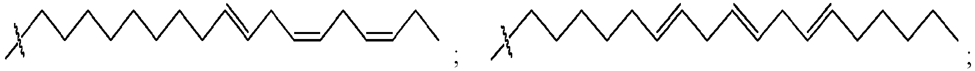

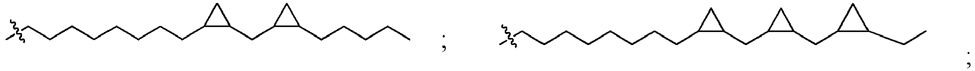

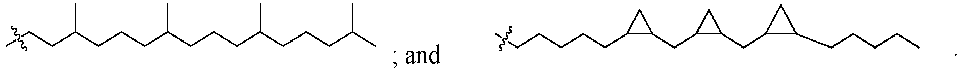

In certain embodiments, Y is an optionally substituted C₁-C₂ alkyl, C₁-C₃ alkyl, C₁-C₄ alkyl, C₁-C₅ alkyl, C₂-C₃ alkyl, C₂-C₄ alkyl, C₂-C₅ alkyl, C₂-C₆ alkyl, C₃-C₄ alkyl, C₃-C₅ alkyl, C₃-C₆ alkyl, C₄-C₅ alkyl, C₄-C₆ alkyl, C₅-C₆ alkyl, C₂-C₃ alkenyl, C₂-C₄ alkenyl, C₂-C₅ alkenyl, C₂-C₆ alkenyl, C₃-C₄ alkenyl, C₃-C₅ alkenyl, C₃-C₆ alkenyl, C₄-C₅ alkenyl, C₄-C₆ alkenyl, C₅-C₆ alkenyl, C₂-C₃ alkynyl, C₂-C₄ alkynyl, C₂-C₅ alkynyl, C₂-C₆ alkynyl, C₃-C₄ alkynyl, C₃-C₅ alkynyl, C₃-C₆ alkynyl, C₄-C₅ alkynyl, C₄-C₆ alkynyl, or C₅-C₆ alkynyl. In one particular embodiment, Y is (CH₂)ₙ and n is 0, 1, 2, 3, 4, 5, or 6 (*e.g.,* 1-2, 1-3, 1-4, 1-5, 1-6, 2-3, 2-4, 2-5, or 2-6). In a preferred embodiment, n is 2, 3, or 4.

In particular cases, the cationic lipid of Formula I has the following structure: 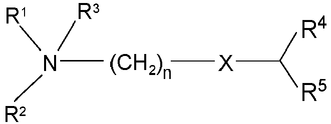 or salts thereof, wherein R¹, R², R³, R⁴, R⁵, X, and n are the same as described above.

In some embodiments, the cationic lipid of Formula I forms a salt (preferably a crystalline salt) with one or more anions. In one particular embodiment, the cationic lipid of Formula I is the oxalate (*e.g.,* hemioxalate) salt thereof, which is preferably a crystalline salt.

Described herein are particular cationic lipids of Formula I having one of the following structures: 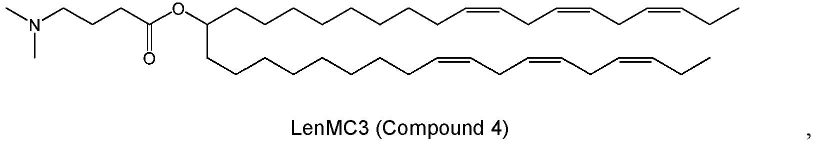 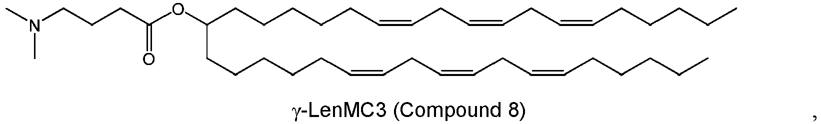 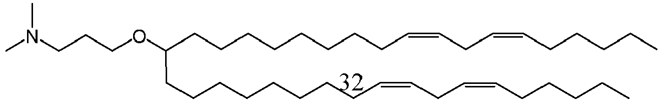 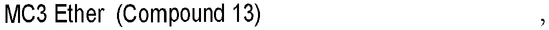 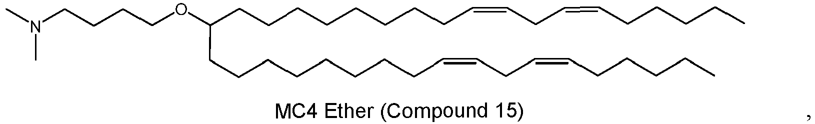 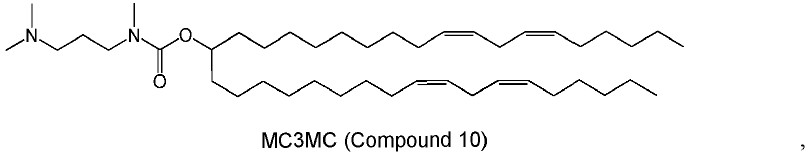 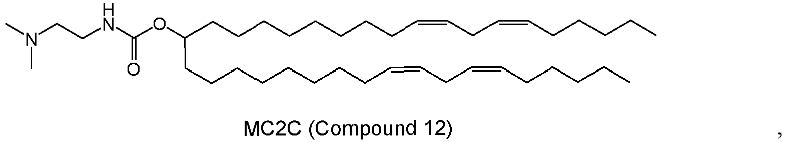 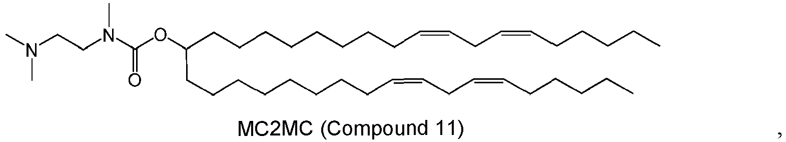 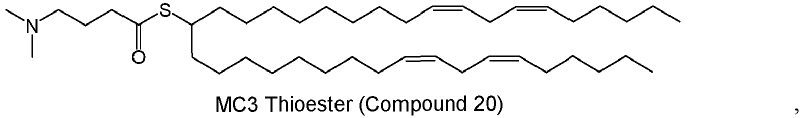 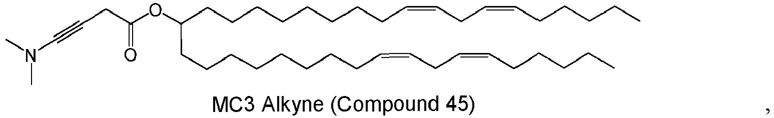 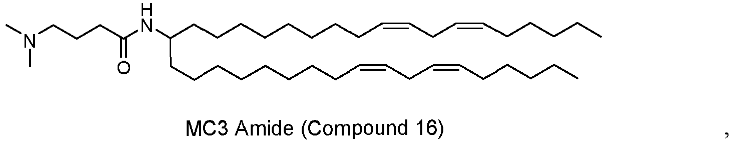 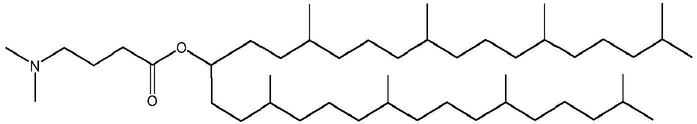 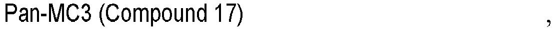 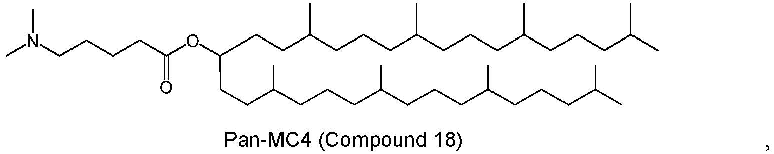 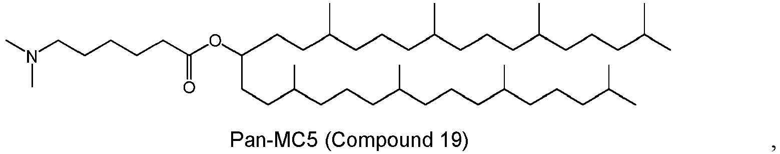 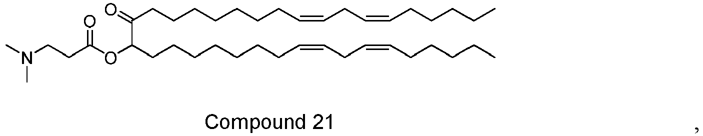 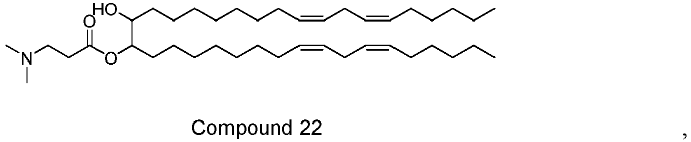 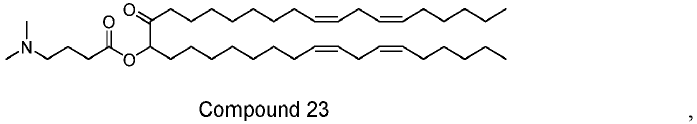 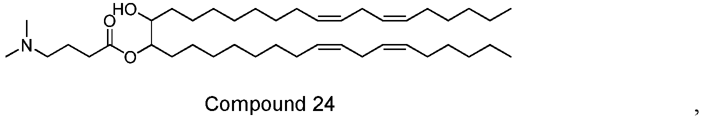 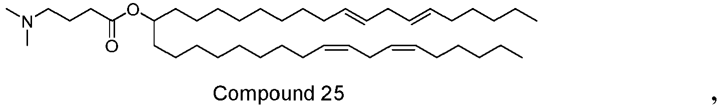 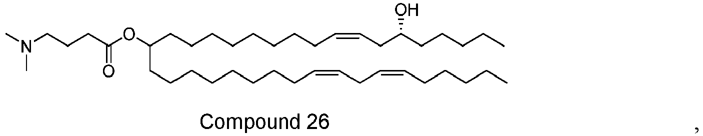 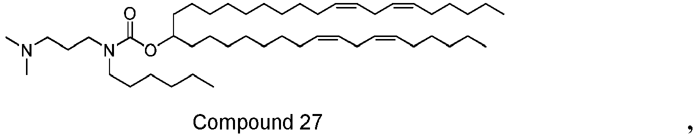 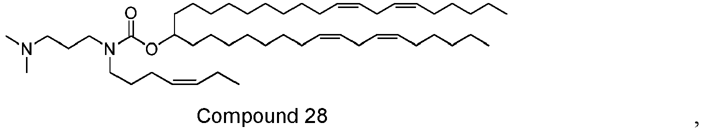 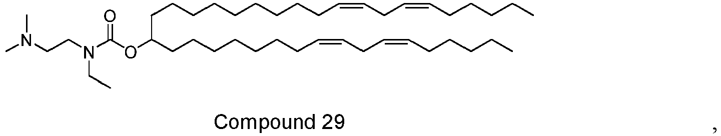 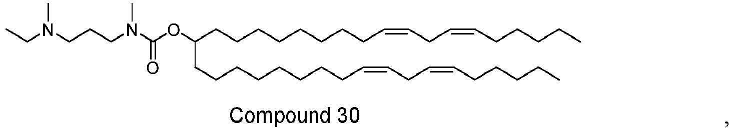 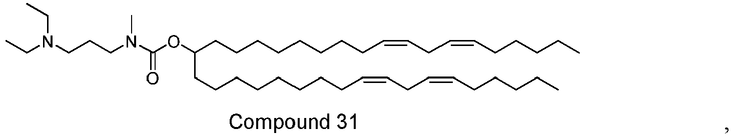 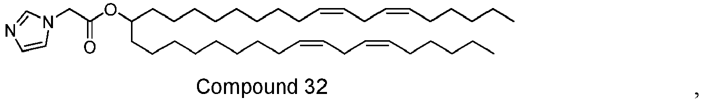 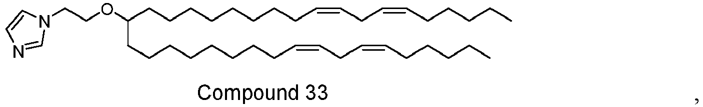 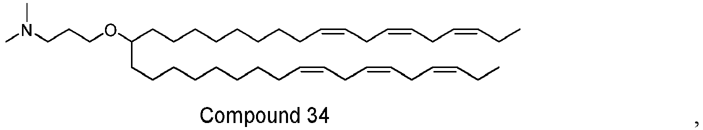 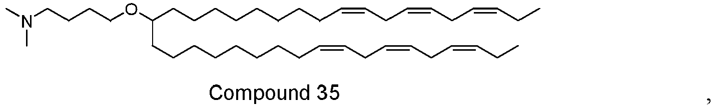 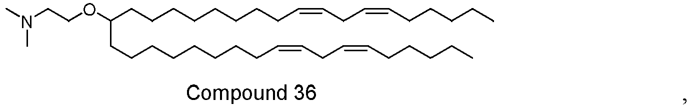 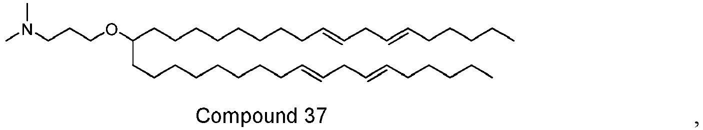 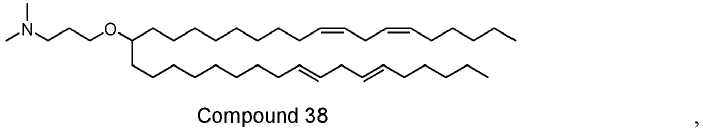 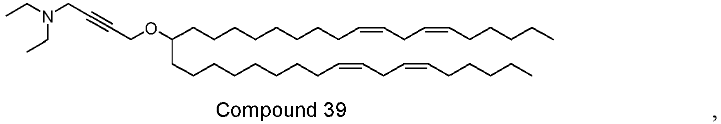 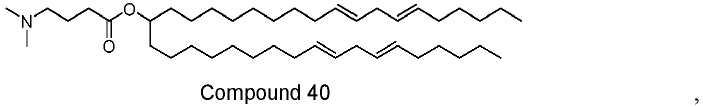 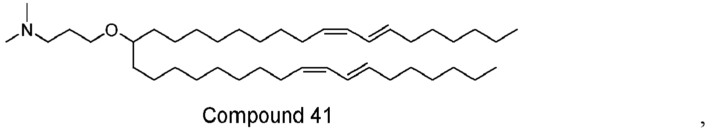 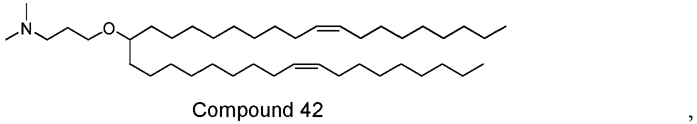 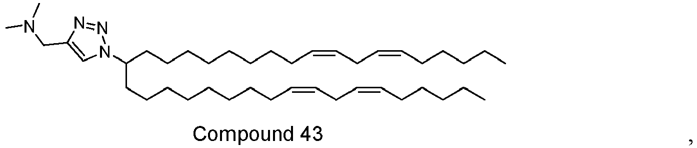 or

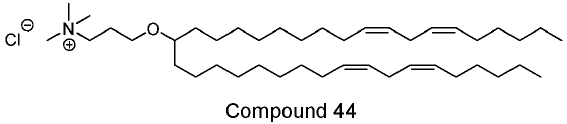

The compounds of the invention may be prepared by known organic synthesis techniques, including the methods described in the Examples. In some embodiments, the synthesis of the cationic lipids of the invention may require the use of protecting groups. Protecting group methodology is well known to those skilled in the art (*see, e.g.,* PROTECTIVE GROUPS IN ORGANIC SYNTHESIS, Green, T.W. et. al., Wiley-Interscience, New York City, 1999). Briefly, protecting groups within the context of this invention are any group that reduces or eliminates the unwanted reactivity of a functional group. A protecting group can be added to a functional group to mask its reactivity during certain reactions and then removed to reveal the original functional group. In certain instances, an "alcohol protecting group" is used. An "alcohol protecting group" is any group which decreases or eliminates the unwanted reactivity of an alcohol functional group. Protecting groups can be added and removed using techniques well known in the art.

In certain embodiments, the cationic lipids of the present invention have at least one protonatable or deprotonatable group, such that the lipid is positively charged at a pH at or below physiological pH (*e.g.,* pH 7.4), and neutral at a second pH, preferably at or above physiological pH. It will be understood by one of ordinary skill in the art that the addition or removal of protons as a function of pH is an equilibrium process, and that the reference to a charged or a neutral lipid refers to the nature of the predominant species and does not require that all of the lipid be present in the charged or neutral form. Lipids that have more than one protonatable or deprotonatable group, or which are zwiterrionic, are not excluded from use in the invention.

In certain other embodiments, protonatable lipids according to the invention have a pKₐ of the protonatable group in the range of about 4 to about 11. Most preferred is a pKₐ of about 4 to about 7, because these lipids will be cationic at a lower pH formulation stage, while particles will be largely (though not completely) surface neutralized at physiological pH of around pH 7.4. One of the benefits of this pKₐ is that at least some nucleic acid associated with the outside surface of the particle will lose its electrostatic interaction at physiological pH and be removed by simple dialysis, thus greatly reducing the particle's susceptibility to clearance.

### IV. Active Agents

Active agents (*e.g.,* therapeutic agents) include any molecule or compound capable of exerting a desired effect on a cell, tissue, tumor, organ, or subject. Such effects may be, *e.g.,* biological, physiological, and/or cosmetic. Active agents may be any type of molecule or compound including, but not limited to, nucleic acids, peptides, polypeptides, small molecules, and mixtures thereof. Non-limiting examples of nucleic acids include interfering RNA molecules (*e.g.,* dsRNA such as siRNA, Dicer-substrate dsRNA, shRNA, aiRNA, and/or miRNA), antisense oligonucleotides, plasmids, ribozymes, immunostimulatory oligonucleotides, and mixtures thereof. Examples of peptides or polypeptides include, without limitation, antibodies (*e.g.,* polyclonal antibodies, monoclonal antibodies, antibody fragments; humanized antibodies, recombinant antibodies, recombinant human antibodies, and/or Primatized™ antibodies), cytokines, growth factors, apoptotic factors, differentiation-inducing factors, cell-surface receptors and their ligands, hormones, and mixtures thereof. Examples of small molecules include, but are not limited to, small organic molecules or compounds such as any conventional agent or drug known to those of skill in the art.

In some embodiments, the active agent is a therapeutic agent, or a salt or derivative thereof. Therapeutic agent derivatives may be therapeutically active themselves or they may be prodrugs, which become active upon further modification. Thus, in one embodiment, a therapeutic agent derivative retains some or all of the therapeutic activity as compared to the unmodified agent, while in another embodiment, a therapeutic agent derivative is a prodrug that lacks therapeutic activity, but becomes active upon further modification.

In preferred embodiments, the lipid particles described herein are associated with a nucleic acid, resulting in a nucleic acid-lipid particle (*e.g.,* SNALP). Non-limiting exemplary embodiments related to selecting, synthesizing, and modifying nucleic acids such as siRNA, Dicer-substrate dsRNA, shRNA, aiRNA, miRNA, antisense oligonucleotides, ribozymes, and immunostimulatory oligonucleotides are described, for example, in U.S. Patent Publication No. 20070135372; in U.S. Patent Publication No. 20110076335; and in PCT Publication No. WO 2010/105372.

In certain embodiments, the nucleic acid (e.g., interfering RNA) component of the nucleic acid-lipid particle (*e.g.,* SNALP) comprises at least one modified nucleotide (*e.g.,* at least 1, 2, 3, 4, 5, 6, 7, 8, 9, 10, 11, 12, or more modified nucleotides). In certain instances, the nucleic acid (*e.g.,* interfering RNA such as an siRNA) comprises modified nucleotides including, but not limited to, 2'-O-methyl (2'OMe) nucleotides, 2'-deoxy-2'-fluoro (2'F) nucleotides, 2'-deoxy nucleotides, 2'-O-(2-methoxyethyl) (MOE) nucleotides, locked nucleic acid (LNA) nucleotides, 5-C-methyl nucleotides, 4'-thio nucleotides, 2'-amino nucleotides, 2'-C-allyl nucleotides, and mixtures thereof. In particular embodiments, the modified interfering RNA (*e.g.,* modified siRNA) is generally less immunostimulatory than a corresponding unmodified interfering RNA (*e.g.,* unmodified siRNA) sequence and retains RNAi activity against the target gene of interest. In some embodiments, the modified interfering RNA (*e.g.,* modified siRNA) contains at least one 2'OMe purine or pyrimidine nucleotide such as a 2'OMe-guanosine, 2'OMe-uridine, 2'OMe-adenosine, and/or 2'OMe-cytosine nucleotide. The modified nucleotides can be present in one strand (*i.e.,* sense or antisense) or both strands of the interfering RNA (*e.g.,* siRNA). In some preferred embodiments, one or more of the uridine and/or guanosine nucleotides are modified (*e.g.,* 2'OMe-modified) in one strand (*i.e.,* sense or antisense) or both strands of the interfering RNA (*e.g.,* siRNA). In these embodiments, the modified interfering RNA (*e.g.,* modified siRNA) can further comprise one or more modified (*e.g.,* 2'OMe-modified) adenosine and/or modified (*e.g.,* 2'OMe-modified) cytosine nucleotides. In other preferred embodiments, only uridine and/or guanosine nucleotides are modified (*e.g.,* 2'OMe-modified) in one strand (*i.e.,* sense or antisense) or both strands of the interfering RNA (*e.g.,* siRNA). The interfering RNA *(e.g.,* siRNA) sequences may have overhangs *(e.g.,* 3' or 5' overhangs as described in Elbashir et al., Genes Dev., 15:188 (2001) or Nykänen et al., Cell, 107:309 (2001)), or may lack overhangs *(i.e.,* have blunt ends). The interfering RNA *(e.g.,* siRNA) sequences may comprise one or more modified nucleotides in the double-stranded (duplex) region and/or in one or both of the overhangs (e.g., 3' overhangs) when present.

The nucleic acid (e.g., interfering RNA) component of the nucleic acid-lipid particle *(e.g.,* SNALP) can be used to downregulate or silence the translation *(i.e.,* expression) of a gene of interest. Non-limiting examples of genes of interest include genes associated with metabolic diseases and disorders (*e.g.,* liver diseases and disorders), genes associated with cell proliferation, tumorigenesis, and/or cell transformation (*e.g.,* a cell proliferative disorder such as cancer), angiogenic genes, receptor ligand genes, immunomodulator genes (*e.g.,* those associated with inflammatory and autoimmune responses), genes associated with viral infection and survival, and genes associated with neurodegenerative disorders. *See, e.g.,* U.S. Patent Publication No. 20110076335 for a description of exemplary target genes (including their Genbank Accession Nos.) which may be downregulated or silenced by the nucleic acid (*e.g.,* interfering RNA) of the nucleic acid-lipid particle (*e.g.,* SNALP).

Non-limiting examples of gene sequences associated with tumorigenesis or cell transformation include polo-like kinase 1 (PLK-1), cyclin-dependent kinase 4 (CDK4), COP1, ring-box 1 (RBX1), WEE1, Eg5 (KSP, KIF11), forkhead box M1 (FOXM1), RAM2 (R1, CDCA7L), XIAP, CSN5 (JAB1), and HDAC2. Non-limiting examples of gene sequences associated with metabolic diseases and disorders include apolipoprotein B (APOB), apolipoprotein CIII (APOC3), apolipoprotein E (APOE), proprotein convertase subtilisin/kexin type 9 (PCSK9), diacylglycerol O-acyltransferase type 1 (DGAT1), and diacylglyerol O-acyltransferase type 2 (DGAT2). Non-limiting examples of gene sequences associated with viral infection and survival include host factors such as tissue factor (TF) or nucleic acid sequences from Filoviruses such as Ebola virus and Marburg virus (*e.g.,* VP30, VP35, nucleoprotein (NP), polymerase protein (L-pol), VP40, glycoprotein (GP), and VP24); Arenaviruses such as Lassa virus (*e.g.,* NP, GP, L, and/or Z genes), Junin virus, Machupo virus, Guanarito virus, and Sabia virus; Hepatitis viruses such as Hepatitis A, B, C, D, and E viruses; Influenza viruses such as Influenza A, B, and C viruses; Human Immunodeficiency Virus (HIV); Herpes viruses; and Human Papilloma Viruses (HPV).

In other embodiments, the active agent associated with the lipid particles of the invention may comprise one or more therapeutic proteins, polypeptides, or small organic molecules or compounds. Non-limiting examples of such therapeutically effective agents or drugs include oncology drugs (*e.g.,* chemotherapy drugs, hormonal therapaeutic agents, immunotherapeutic agents, radiotherapeutic agents, *etc.*)*,* lipid-lowering agents, anti-viral drugs, anti-inflammatory compounds, antidepressants, stimulants, analgesics, antibiotics, birth control medication, antipyretics, vasodilators, anti-angiogenics, cytovascular agents, signal transduction inhibitors, cardiovascular drugs such as anti-arrhythmic agents, hormones, vasoconstrictors, and steroids. These active agents may be administered alone in the lipid particles of the invention, or in combination (*e.g.,* co-administered) with lipid particles of the invention comprising nucleic acid such as interfering RNA. Non-limiting examples of these types of active agents are described, *e.g.,* in U.S. Patent Publication No. 20110076335.

### V. Lipid Particles

In certain aspects, the present invention provides lipid particles comprising one or more of the cationic (amino) lipids or salts thereof described herein. In some embodiments, the lipid particles of the invention further comprise one or more non-cationic lipids. In other embodiments, the lipid particles further comprise one or more conjugated lipids capable of reducing or inhibiting particle aggregation. In additional embodiments, the lipid particles further comprise one or more active agents or therapeutic agents such as therapeutic nucleic acids (*e.g.,* interfering RNA such as siRNA).

Lipid particles include, but are not limited to, lipid vesicles such as liposomes. As used herein, a lipid vesicle includes a structure having lipid-containing membranes enclosing an aqueous interior. In particular embodiments, lipid vesicles comprising one or more of the cationic lipids described herein are used to encapsulate nucleic acids within the lipid vesicles. In other embodiments, lipid vesicles comprising one or more of the cationic lipids described herein are complexed with nucleic acids to form lipoplexes.

The lipid particles of the invention typically comprise an active agent or therapeutic agent, a cationic lipid, a non-cationic lipid, and a conjugated lipid that inhibits aggregation of particles. In some embodiments, the active agent or therapeutic agent is fully encapsulated within the lipid portion of the lipid particle such that the active agent or therapeutic agent in the lipid particle is resistant in aqueous solution to enzymatic degradation, *e.g.,* by a nuclease or protease. In other embodiments, the lipid particles described herein are substantially non-toxic to mammals such as humans. The lipid particles of the invention typically have a mean diameter of from about 30 nm to about 150 nm, from about 40 nm to about 150 nm, from about 50 nm to about 150 nm, from about 60 nm to about 130 nm, from about 70 nm to about 110 nm, or from about 70 to about 90 nm. The lipid particles of the invention also typically have a lipid:therapeutic agent (*e.g.,* lipid:nucleic acid) ratio (mass/mass ratio) of from about 1:1 to about 100:1, from about 1:1 to about 50:1, from about 2:1 to about 25:1, from about 3:1 to about 20:1, from about 5:1 to about 15:1, or from about 5:1 to about 10:1.

In preferred embodiments, the lipid particles of the invention are serum-stable nucleic acid-lipid particles (SNALP) which comprise an interfering RNA (*e.g.,* dsRNA such as siRNA, Dicer-substrate dsRNA, shRNA, aiRNA, and/or miRNA), a cationic lipid (*e.g.,* one or more cationic lipids of Formula I or salts thereof as set forth herein), a non-cationic lipid (*e.g.,* mixtures of one or more phospholipids and cholesterol), and a conjugated lipid that inhibits aggregation of the particles (*e.g.,* one or more PEG-lipid conjugates). The SNALP may comprise at least 1, 2, 3, 4, 5, 6, 7, 8, 9, 10, or more unmodified and/or modified interfering RNA molecules (*e.g.,* siRNA). Nucleic acid-lipid particles and their method of preparation are described in, *e.g.,* U.S. Patent Nos. 5,753,613; 5,785,992; 5,705,385; 5,976,567; 5,981,501; 6,110,745; and 6,320,017; and PCT Publication No. WO 96/40964.

In the nucleic acid-lipid particles of the invention, the nucleic acid may be fully encapsulated within the lipid portion of the particle, thereby protecting the nucleic acid from nuclease degradation. In preferred embodiments, a SNALP comprising a nucleic acid such as an interfering RNA is fully encapsulated within the lipid portion of the particle, thereby protecting the nucleic acid from nuclease degradation. In certain instances, the nucleic acid in the SNALP is not substantially degraded after exposure of the particle to a nuclease at 37°C for at least about 20, 30, 45, or 60 minutes. In certain other instances, the nucleic acid in the SNALP is not substantially degraded after incubation of the particle in serum at 37°C for at least about 30, 45, or 60 minutes or at least about 2, 3, 4, 5, 6, 7, 8, 9, 10, 12, 14, 16, 18, 20, 22, 24, 26, 28, 30, 32, 34, or 36 hours. In other embodiments, the nucleic acid is complexed with the lipid portion of the particle. One of the benefits of the formulations of the present invention is that the nucleic acid-lipid particle compositions are substantially non-toxic to mammals such as humans.

The term "fully encapsulated" indicates that the nucleic acid in the nucleic acid-lipid particle is not significantly degraded after exposure to serum or a nuclease assay that would significantly degrade free DNA or RNA. In a fully encapsulated system, preferably less than about 25% of the nucleic acid in the particle is degraded in a treatment that would normally degrade 100% of free nucleic acid, more preferably less than about 10%, and most preferably less than about 5% of the nucleic acid in the particle is degraded. "Fully encapsulated" also indicates that the nucleic acid-lipid particles are serum-stable, that is, that they do not rapidly decompose into their component parts upon *in vivo* administration.

In the context of nucleic acids, full encapsulation may be determined by performing a membrane-impermeable fluorescent dye exclusion assay, which uses a dye that has enhanced fluorescence when associated with nucleic acid. Specific dyes such as OliGreen® and RiboGreen® (Invitrogen Corp.; Carlsbad, CA) are available for the quantitative determination of plasmid DNA, single-stranded deoxyribonucleotides, and/or single- or double-stranded ribonucleotides. Encapsulation is determined by adding the dye to a liposomal formulation, measuring the resulting fluorescence, and comparing it to the fluorescence observed upon addition of a small amount of nonionic detergent. Detergent-mediated disruption of the liposomal bilayer releases the encapsulated nucleic acid, allowing it to interact with the membrane-impermeable dye. Nucleic acid encapsulation may be calculated as *E* = *(Iₒ* - *I)*/*Iₒ,* where *I* and *Iₒ* refer to the fluorescence intensities before and after the addition of detergent (*see,* Wheeler et al., Gene Ther., 6:271-281 (1999)).

In other embodiments, the present invention provides a nucleic acid-lipid particle (*e.g.,* SNALP) composition comprising a plurality of nucleic acid-lipid particles.

In some instances, the SNALP composition comprises nucleic acid that is fully encapsulated within the lipid portion of the particles, such that from about 30% to about 100%, from about 40% to about 100%, from about 50% to about 100%, from about 60% to about 100%, from about 70% to about 100%, from about 80% to about 100%, from about 90% to about 100%, from about 30% to about 95%, from about 40% to about 95%, from about 50% to about 95%, from about 60% to about 95%, from about 70% to about 95%, from about 80% to about 95%, from about 85% to about 95%, from about 90% to about 95%, from about 30% to about 90%, from about 40% to about 90%, from about 50% to about 90%, from about 60% to about 90%, from about 70% to about 90%, from about 80% to about 90%, or at least about 30%, 35%, 40%, 45%, 50%, 55%, 60%, 65%, 70%, 75%, 80%, 85%, 90%, 91%, 92%, 93%, 94%, 95%, 96%, 97%, 98%, or 99% (or any fraction thereof or range therein) of the particles have the nucleic acid encapsulated therein.

In other instances, the SNALP composition comprises nucleic acid that is fully encapsulated within the lipid portion of the particles, such that from about 30% to about 100%, from about 40% to about 100%, from about 50% to about 100%, from about 60% to about 100%, from about 70% to about 100%, from about 80% to about 100%, from about 90% to about 100%, from about 30% to about 95%, from about 40% to about 95%, from about 50% to about 95%, from about 60% to about 95%, from about 70% to about 95%, from about 80% to about 95%, from about 85% to about 95%, from about 90% to about 95%, from about 30% to about 90%, from about 40% to about 90%, from about 50% to about 90%, from about 60% to about 90%, from about 70% to about 90%, from about 80% to about 90%, or at least about 30%, 35%, 40%, 45%, 50%, 55%, 60%, 65%, 70%, 75%, 80%, 85%, 90%, 91%, 92%, 93%, 94%, 95%, 96%, 97%, 98%, or 99% (or any fraction thereof or range therein) of the input nucleic acid is encapsulated in the particles.

Depending on the intended use of the lipid particles of the invention, the proportions of the components can be varied and the delivery efficiency of a particular formulation can be measured using, *e.g.,* an endosomal release parameter (ERP) assay.

In particular embodiments, the present invention provides a lipid particle (*e.g.,* SNALP) composition comprising a plurality of lipid particles described herein and an antioxidant. In certain instances, the antioxidant in the lipid particle composition reduces, prevents, and/or inhibits the degradation of a cationic lipid (*e.g.,* a polyunsaturated cationic lipid) present in the lipid particle. In instances wherein the active agent is a therapeutic nucleic acid such as an interfering RNA (*e.g.,* siRNA), the antioxidant in the lipid particle composition reduces, prevents, and/or inhibits the degradation of the nucleic acid payload, *e.g.,* by reducing, preventing, and/or inhibiting the oxidation of the cationic lipid, by reducing, preventing, and/or inhibiting the degradation of the nucleic acid payload, by reducing, preventing, and/or inhibiting the desulfurization of a phophorothioate (PS)-modified nucleic acid payload, and/or by stabilizing both the lipid and nucleic acid components.

Examples of antioxidants include, but are not limited to, metal chelators (*e.g.,* ethylenediaminetetraacetic acid (EDTA), citrate, and the like), primary antioxidants (*e.g.,* vitamin E isomers such as α-tocopherol or a salt thereof, butylated hydroxyanisole (BHA), butylhydroxytoluene (BHT), *tert*-butylhydroquinone (TBHQ), and the like), secondary antioxidants (*e.g.,* ascorbic acid, ascorbyl palmitate, cysteine, glutathione, α-lipoic acid, and the like), salts thereof, and mixtures thereof. If needed, the antioxidant is typically present in an amount sufficient to prevent, inhibit, and/or reduce the degradation of the cationic lipid and/or active agent present in the lipid particle. In particular embodiments, the antioxidant comprises EDTA or a salt thereof (*e.g.,* from about 20 mM to about 100 mM), alone or in combination with a primary antioxidant such as α-tocopherol or a salt thereof (*e.g.,* from about 0.02 mol % to about 0.5 mol %) and/or secondary antioxidant such as ascorbyl palmitate or a salt thereof (*e.g.,* from about 0.02 mol % to about 5.0 mol %). An antioxidant such as EDTA may be included at any step or at multiple steps in the lipid particle formation process described in Section VI (*e.g.,* prior to, during, and/or after lipid particle formation).

Additional embodiments related to methods of preventing the degradation of cationic lipids and/or active agents (*e.g.,* therapeutic nucleic acids) present in lipid particles, compositions comprising lipid particles stabilized by these methods, methods of making these lipid particles, and methods of delivering and/or administering these lipid particles are described in PCT Publication No. WO/2011/066651, filed December 1, 2010.

In one aspect, the lipid particles of the invention may include a targeting lipid. In some embodiments, the targeting lipid comprises a GalNAc moiety (*i.e.,* an N-galactosamine moiety). As a non-limiting example, a targeting lipid comprising a GalNAc moiety can include those described in US Patent Publication No. 20090247608, filed December 4, 2008. A targeting lipid can also include any other lipid (*e.g.,* targeting lipid) known in the art, for example, as described in US Patent Publication No. 20090247608 or PCT Publication No. WO 2008/042973. In some embodiments, the targeting lipid includes a plurality of GalNAc moieties, *e.g.,* two or three GalNAc moieties. In some embodiments, the targeting lipid contains a plurality, *e.g.,* two or three N-acetylgalactosamine (GalNAc) moieties. In some embodiments, the lipid in the targeting lipid is 1,2-Di-*O*-hexadecyl-*sn*-glyceride (*i.e.,* DSG). In some embodiments, the targeting lipid includes a PEG moiety (*e.g.,* a PEG moiety having a molecular weight of at least about 500 Da, such as about 1000 Da, 1500 Da, 2000 Da or greater), for example, the targeting moiety is connected to the lipid via a PEG moiety. Examples of GalNAc targeting lipids include, but are not limited to, (GalNAc)₃-PEG-DSG, (GalNAc)₃-PEG-LCO, and mixtures thereof.

In some embodiments, the targeting lipid includes a folate moiety. For example, a targeting lipid comprising a folate moiety can include those described in US Patent Publication No. 20090247608. Examples of folate targeting lipids include, but are not limited to, 1,2-distearoyl-sn-glycero-3-phosphoethanolamine-N-[folate(polyethylene glycol)-2000] (ammonium salt) (Folate-PEG-DSPE), Folate-PEG2000-DSG, Folate-PEG3400-DSG, and mixtures thereof.

In another aspect, the lipid particles of the invention may further comprise one or more apolipoproteins. As used herein, the term "apolipoprotein" or "lipoprotein" refers to apolipoproteins known to those of skill in the art and variants and fragments thereof and to apolipoprotein agonists, analogues, or fragments thereof described in, *e.g.,* PCT Publication No. WO 2010/0088537. Suitable apolipoproteins include, but are not limited to, ApoA-I, ApoA-II, ApoA-IV, ApoA-V, and ApoE (*e.g.,* ApoE2, ApoE3, *etc.*)*,* and active polymorphic forms, isoforms, variants, and mutants as well as fragments or truncated forms thereof. Isolated ApoE and/or active fragments and polypeptide analogues thereof, including recombinantly produced forms thereof, are described in US Patent Nos. 5,672,685; 5,525,472; 5,473,039; 5,182,364; 5,177,189; 5,168,045; and 5,116,739.

### A. Cationic Lipids

The novel cationic lipids of Formula I or salts thereof as set forth herein may be used in the lipid particles of the present invention (*e.g.,* SNALP), either alone or in combination with one or more other cationic lipid species or non-cationic lipid species.

Other cationic lipids or salts thereof which may also be included in the lipid particles of the present invention include, but are not limited to, one or more of the cationic lipids of Formulas I-XXII or salts thereof as described in U.S. Patent No. 8,455,455, filed March 31, 2011, one or more of the cationic lipids of Formulas I-XIX or salts thereof as described in PCT Publication No.WO/2011/066651, filed December 1, 2010, and/or one or more of the cationic lipids of Formulas I-III or salts thereof as described in PCT Publication No. WO 2011/141704, filed May 12, 2011. Non-limiting examples of additional suitable cationic lipids include 1,2-dilinoleyloxy-N,N-dimethylaminopropane (DLinDMA), 1,2-dilinolenyloxy-N,N-dimethylaminopropane (DLenDMA), 2,2-dilinoleyl-4-(2-dimethylaminoethyl)-[1,3]-dioxolane (DLin-K-C2-DMA; "XTC2" or "C2K"), 2,2-dilinoleyl-4-dimethylaminomethyl-[1,3]-dioxolane (DLin-K-DMA), 2,2-dilinoleyl-4-(3-dimethylaminopropyl)-[1,3]-dioxolane (DLin-K-C3-DMA; "C3K"), 2,2-dilinoleyl-4-(4-dimethylaminobutyl)-[1,3]-dioxolane (DLin-K-C4-DMA; "C4K"), 2,2-dilinoleyl-5-dimethylaminomethyl-[1,3]-dioxane (DLin-K6-DMA), 2,2-dilinoleyl-4-N-methylpepiazino-[1,3]-dioxolane (DLin-K-MPZ), 2,2-dioleoyl-4-dimethylaminomethyl-[1,3]-dioxolane (DO-K-DMA), 2,2-distearoyl-4-dimethylaminomethyl-[1,3]-dioxolane (DS-K-DMA), 2,2-dilinoleyl-4-N-morpholino-[1,3]-dioxolane (DLin-K-MA), 2,2-Dilinoleyl-4-trimethylamino-[1,3]-dioxolane chloride (DLin-K-TMA.Cl), 2,2-dilinoleyl-4,5-bis(dimethylaminomethyl)-[1,3]-dioxolane (DLin-K²-DMA), 2,2-dilinoleyl-4-methylpiperzine-[1,3]-dioxolane (D-Lin-K-N-methylpiperzine), DLen-C2K-DMA, γ-DLen-C2K-DMA, DPan-C2K-DMA, DPan-C3K-DMA, DLen-C2K-DMA, γ-DLen-C2K-DMA, DPan-C2K-DMA, TLinDMA, C2-TLinDMA, C3-TLinDMA, 1,2-di-γ-linolenyloxy-*N*,*N*-dimethylaminopropane (γ-DLenDMA), 1,2-dilinoleyloxy-(N,N-dimethyl)-butyl-4-amine (C2-DLinDMA), 1,2-dilinoleoyloxy-(N,N-dimethyl)-butyl-4-amine (C2-DLinDAP), dilinoleylmethyl-3-dimethylaminopropionate (DLin-M-C2-DMA), (6Z,9Z,28Z,31Z)-heptatriaconta-6,9,28,31-tetraen-19-yl 4-(dimethylamino) butanoate (DLin-M-C3-DMA or "MC3"; also called dilinoleylmethyl 4-(dimethylamino)butanoate), CP-LenMC3, CP-γ-LenMC3, CP-MC3, CP-DLen-C2K-DMA, CP-γDLen-C2K-DMA, CP-C2K-DMA, CP-DODMA, CP-DPetroDMA, CP-DLinDMA, CP-DLenDMA, CP-yDLenDMA, analogs thereof, salts thereof, and mixtures thereof.

Examples of yet additional cationic lipids include, but are not limited to, 1,2-dioeylcarbamoyloxy-3-dimethylaminopropane (DO-C-DAP), 1,2-dimyristoleoyl-3-dimethylaminopropane (DMDAP), 1,2-dioleoyl-3-trimethylaminopropane chloride (DOTAP.C1), 1,2-dilinoleylcarbamoyloxy-3-dimethylaminopropane (DLin-C-DAP), 1,2-dilinoleyoxy-3-(dimethylamino)acetoxypropane (DLin-DAC), 1,2-dilinoleyoxy-3-morpholinopropane (DLin-MA), 1,2-dilinoleoyl-3-dimethylaminopropane (DLinDAP), 1,2-dilinoleylthio-3 -dimethylaminopropane (DLin-S-DMA), 1-linoleoyl-2-linoleyloxy-3 - dimethylaminopropane (DLin-2-DMAP), 1,2-dilinoleyloxy-3-trimethylaminopropane chloride salt (DLin-TMA.Cl), 1,2-dilinoleoyl-3-trimethylaminopropane chloride salt (DLin-TAP.C1), 1,2-dilinoleyloxy-3-(N-methylpiperazino)propane (DLin-MPZ), 3-(N,N-dilinoleylamino)-1,2-propanediol (DLinAP), 3-(N,N-dioleylamino)-1,2-propanedio (DOAP), 1,2-dilinoleyloxo-3-(2-N,N-dimethylamino)ethoxypropane (DLin-EG-DMA), 3-dimethylamino-2-(cholest-5-en-3-beta-oxybutan-4-oxy)-1-(cis,cis-9,12-octadecadienoxy)propane (CLinDMA), 2-[5'-(cholest-5-en-3-beta-oxy)-3'-oxapentoxy)-3-dimethy-1-(cis,cis-9',1-2'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 1,2-N,N'-dilinoleylcarbamyl-3-dimethylaminopropane (DLincarbDAP), N,N-dioleyl-N,N-dimethylammonium chloride (DODAC), 1,2-dioleyloxy-N,N-dimethylaminopropane (DODMA), 1,2-distearyloxy-N,N-dimethylaminopropane (DSDMA), N-(1-(2,3-dioleyloxy)propyl)-N,N,N-trimethylammonium chloride (DOTMA), N,N-distearyl-N,N-dimethylammonium bromide (DDAB), N-(1-(2,3-dioleoyloxy)propyl)-N,N,N-trimethylammonium chloride (DOTAP), 3 -(N-(N',N'-dimethylaminoethane)-carbamoyl)cholesterol (DC-Chol), N-(1,2-dimyristyloxyprop-3-yl)-N,N-dimethyl-N-hydroxyethyl ammonium bromide (DMRIE), 2,3-dioleyloxy-N-[2(spermine-carboxamido)ethyl]-N,N-dimethyl-1-propanaminiumtrifluoroacetate (DOSPA), dioctadecylamidoglycyl spermine (DOGS), analogs thereof, salts thereof, and mixtures thereof.

In some embodiments, the additional cationic lipid forms a salt (preferably a crystalline salt) with one or more anions. In one particular embodiment, the additional cationic lipid is the oxalate (*e.g.,* hemioxalate) salt thereof, which is preferably a crystalline salt.

The synthesis of cationic lipids such as DLinDMA and DLenDMA, as well as additional cationic lipids, is described in U.S. Patent Publication No. 20060083780.

The synthesis of cationic lipids such as DLin-K-C2-DMA, DLin-K-C3-DMA, DLin-K-C4-DMA, DLin-K6-DMA, DLin-K-MPZ, DO-K-DMA, DS-K-DMA, DLin-K-MA, DLin-K-TMA.Cl, DLin-K²-DMA, D-Lin-K-N-methylpiperzine, DLin-M-C2-DMA, DO-C-DAP, DMDAP, and DOTAP.Cl, as well as additional cationic lipids, is described in PCT Publication No. WO 2010/042877.

The synthesis of cationic lipids such as DLin-K-DMA, DLin-C-DAP, DLinDAC, DLinMA, DLinDAP, DLin-S-DMA, DLin-2-DMAP, DLinTMA.Cl, DLinTAP.Cl, DLinMPZ, DLinAP, DOAP, and DLin-EG-DMA, as well as additional cationic lipids, is described in PCT Publication No. WO 09/086558.

The synthesis of cationic lipids such as γ-DLenDMA, DLen-C2K-DMA, γ-DLen-C2K-DMA, DPan-C2K-DMA, DPan-C3K-DMA, DLen-C2K-DMA, γ-DLen-C2K-DMA, DPan-C2K-DMA, TLinDMA, C2-TLinDMA, C3-TLinDMA, C2-DLinDMA, and C2-DLinDAP, as well as additional cationic lipids, is described in PCT Publication No. WO 2011/000106.

The synthesis of cationic lipids such as CP-LenMC3, CP-γ-LenMC3, CP-MC3, CP-DLen-C2K-DMA, CP-γDLen-C2K-DMA, CP-C2K-DMA, CP-DODMA, CP-DPetroDMA, CP-DLinDMA, CP-DLenDMA, and CP-yDLenDMA is described in PCT Publication No. WO 2011/141704.

The synthesis of cationic lipids such as CLinDMA, as well as additional cationic lipids, is described in U.S. Patent Publication No. 20060240554.

The synthesis of a number of other cationic lipids and related analogs has been described in U.S. Patent Nos. 5,208,036; 5,264,618; 5,279,833; 5,283,185; 5,753,613; and 5,785,992; and PCT Publication No. WO 96/10390. Additionally, a number of commercial preparations of cationic lipids can be used, such as, *e.g.,* LIPOFECTIN® (including DOTMA and DOPE, available from GIBCO/BRL); LIPOFECTAMINE® (including DOSPA and DOPE, available from GIBCO/BRL); and TRANSFECTAM® (including DOGS, available from Promega Corp.).

The synthesis of additional cationic lipids suitable for use in the lipid particles of the present invention is described in PCT Publication Nos. WO 2010/054401, WO 2010/054405, WO 2010/054406, WO 2010/054384, and WO 2010/144740; U.S. Patent Publication No. 20090023673; and U.S. Provisional Application No. 61/287,995, entitled "Methods and Compositions for Delivery of Nucleic Acids," filed December 18, 2009.

In some embodiments, the cationic lipid comprises from about 45 mol % to about 90 mol %, from about 45 mol % to about 85 mol %, from about 45 mol % to about 80 mol %, from about 45 mol % to about 75 mol %, from about 45 mol % to about 70 mol %, from about 45 mol % to about 65 mol %, from about 45 mol % to about 60 mol %, from about 45 mol % to about 55 mol %, from about 50 mol % to about 90 mol %, from about 50 mol % to about 85 mol %, from about 50 mol % to about 80 mol %, from about 50 mol % to about 75 mol %, from about 50 mol % to about 70 mol %, from about 50 mol % to about 65 mol %, from about 50 mol % to about 60 mol %, from about 55 mol % to about 65 mol % or from about 55 mol % to about 70 mol % (or any fraction thereof or range therein) of the total lipid present in the particle.

In certain preferred embodiments, the cationic lipid comprises from about 50 mol % to about 58 mol %, from about 51 mol % to about 59 mol %, from about 51 mol % to about 58 mol %, from about 51 mol % to about 57 mol %, from about 52 mol % to about 58 mol %, from about 52 mol % to about 57 mol %, from about 52 mol % to about 56 mol %, or from about 53 mol % to about 55 mol % (or any fraction thereof or range therein) of the total lipid present in the particle. In particular embodiments, the cationic lipid comprises about 50 mol %, 51 mol %, 52 mol %, 53 mol %, 54 mol %, 55 mol %, 56 mol %, 57 mol %, 58 mol %, 59 mol %, 60 mol %, 61 mol %, 62 mol %, 63 mol %, 64 mol %, or 65 mol % (or any fraction thereof or range therein) of the total lipid present in the particle. In other embodiments, the cationic lipid comprises (at least) about 66, 67, 68, 69, 70, 71, 72, 73, 74, 75, 76, 77, 78, 79, 80, 81, 82, 83, 84, 85, 86, 87, 88, 89, or 90 mol % (or any fraction thereof or range therein) of the total lipid present in the particle.

In additional embodiments, the cationic lipid comprises from about 2 mol % to about 60 mol %, from about 5 mol % to about 50 mol %, from about 10 mol % to about 50 mol %, from about 20 mol % to about 50 mol %, from about 20 mol % to about 40 mol %, from about 30 mol % to about 40 mol %, or about 40 mol % (or any fraction thereof or range therein) of the total lipid present in the particle.

Additional percentages and ranges of cationic lipids suitable for use in the lipid particles of the present invention are described in PCT Publication No. WO 09/127060, U.S. Publication No. 20110071208, and U.S. Publication No. 20110076335.

It should be understood that the percentage of cationic lipid present in the lipid particles of the invention is a target amount, and that the actual amount of cationic lipid present in the formulation may vary, for example, by ± 5 mol %. For example, in the 1:57 lipid particle (*e.g.,* SNALP) formulation, the target amount of cationic lipid is 57.1 mol %, but the actual amount of cationic lipid may be ± 5 mol %, ± 4 mol %, ± 3 mol %, ± 2 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle). Similarly, in the 7:54 lipid particle (*e.g.,* SNALP) formulation, the target amount of cationic lipid is 54.06 mol %, but the actual amount of cationic lipid may be ± 5 mol %, ± 4 mol %, ± 3 mol %, ± 2 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle).

### B. Non-Cationic Lipids

The non-cationic lipids used in the lipid particles of the invention (*e.g.,* SNALP) can be any of a variety of neutral uncharged, zwitterionic, or anionic lipids capable of producing a stable complex.

Non-limiting examples of non-cationic lipids include phospholipids such as lecithin, phosphatidylethanolamine, lysolecithin, lysophosphatidylethanolamine, phosphatidylserine, phosphatidylinositol, sphingomyelin, egg sphingomyelin (ESM), cephalin, cardiolipin, phosphatidic acid, cerebrosides, dicetylphosphate, distearoylphosphatidylcholine (DSPC), dioleoylphosphatidylcholine (DOPC), dipalmitoylphosphatidylcholine (DPPC), dioleoylphosphatidylglycerol (DOPG), dipalmitoylphosphatidylglycerol (DPPG), dioleoylphosphatidylethanolamine (DOPE), palmitoyloleoyl-phosphatidylcholine (POPC), palmitoyloleoyl-phosphatidylethanolamine (POPE), palmitoyloleyol-phosphatidylglycerol (POPG), dioleoylphosphatidylethanolamine 4-(N-maleimidomethyl)-cyclohexane-1-carboxylate (DOPE-mal), dipalmitoyl-phosphatidylethanolamine (DPPE), dimyristoyl-phosphatidylethanolamine (DMPE), distearoyl-phosphatidylethanolamine (DSPE), monomethyl-phosphatidylethanolamine, dimethyl-phosphatidylethanolamine, dielaidoyl-phosphatidylethanolamine (DEPE), stearoyloleoyl-phosphatidylethanolamine (SOPE), lysophosphatidylcholine, dilinoleoylphosphatidylcholine, and mixtures thereof. Other diacylphosphatidylcholine and diacylphosphatidylethanolamine phospholipids can also be used. The acyl groups in these lipids are preferably acyl groups derived from fatty acids having C₁₀-C₂₄ carbon chains, *e.g.,* lauroyl, myristoyl, palmitoyl, stearoyl, or oleoyl.

Additional examples of non-cationic lipids include sterols such as cholesterol and derivatives thereof. Non-limiting examples of cholesterol derivatives include polar analogues such as 5α-cholestanol, 5β-coprostanol, cholesteryl-(2'-hydroxy)-ethyl ether, cholesteryl-(4'-hydroxy)-butyl ether, and 6-ketocholestanol; non-polar analogues such as 5α-cholestane, cholestenone, 5α-cholestanone, 5β-cholestanone, and cholesteryl decanoate; and mixtures thereof. In preferred embodiments, the cholesterol derivative is a polar analogue such as cholesteryl-(4'-hydroxy)-butyl ether. The synthesis of cholesteryl-(2'-hydroxy)-ethyl ether is described in PCT Publication No. WO 09/127060.

In some embodiments, the non-cationic lipid present in the lipid particles (*e.g.,* SNALP) comprises or consists of a mixture of one or more phospholipids and cholesterol or a derivative thereof. In other embodiments, the non-cationic lipid present in the lipid particles (*e.g.,* SNALP) comprises or consists of one or more phospholipids, *e.g.,* a cholesterol-free lipid particle formulation. In yet other embodiments, the non-cationic lipid present in the lipid particles (*e.g.,* SNALP) comprises or consists of cholesterol or a derivative thereof, *e.g.,* a phospholipid-free lipid particle formulation.

Other examples of non-cationic lipids suitable for use in the present invention include nonphosphorous containing lipids such as, *e.g.,* stearylamine, dodecylamine, hexadecylamine, acetyl palmitate, glycerolricinoleate, hexadecyl stereate, isopropyl myristate, amphoteric acrylic polymers, triethanolamine-lauryl sulfate, alkyl-aryl sulfate polyethyloxylated fatty acid amides, dioctadecyldimethyl ammonium bromide, ceramide, sphingomyelin, and the like.

In some embodiments, the non-cationic lipid comprises from about 10 mol % to about 60 mol %, from about 20 mol % to about 55 mol %, from about 20 mol % to about 45 mol %, from about 20 mol % to about 40 mol %, from about 25 mol % to about 50 mol %, from about 25 mol % to about 45 mol %, from about 30 mol % to about 50 mol %, from about 30 mol % to about 45 mol %, from about 30 mol % to about 40 mol %, from about 35 mol % to about 45 mol %, from about 37 mol % to about 42 mol %, or about 35 mol %, 36 mol %, 37 mol %, 38 mol %, 39 mol %, 40 mol %, 41 mol %, 42 mol %, 43 mol %, 44 mol %, or 45 mol % (or any fraction thereof or range therein) of the total lipid present in the particle.

In embodiments where the lipid particles contain a mixture of phospholipid and cholesterol or a cholesterol derivative, the mixture may comprise up to about 40 mol %, 45 mol %, 50 mol %, 55 mol %, or 60 mol % of the total lipid present in the particle.

In some embodiments, the phospholipid component in the mixture may comprise from about 2 mol % to about 20 mol %, from about 2 mol % to about 15 mol %, from about 2 mol % to about 12 mol %, from about 4 mol % to about 15 mol %, or from about 4 mol % to about 10 mol % (or any fraction thereof or range therein) of the total lipid present in the particle. In certain preferred embodiments, the phospholipid component in the mixture comprises from about 5 mol % to about 10 mol %, from about 5 mol % to about 9 mol %, from about 5 mol % to about 8 mol %, from about 6 mol % to about 9 mol %, from about 6 mol % to about 8 mol %, or about 5 mol %, 6 mol %, 7 mol %, 8 mol %, 9 mol %, or 10 mol % (or any fraction thereof or range therein) of the total lipid present in the particle. As a non-limiting example, a 1:57 lipid particle formulation comprising a mixture of phospholipid and cholesterol may comprise a phospholipid such as DPPC or DSPC at about 7 mol % (or any fraction thereof), *e.g.,* in a mixture with cholesterol or a cholesterol derivative at about 34 mol % (or any fraction thereof) of the total lipid present in the particle. As another non-limiting example, a 7:54 lipid particle formulation comprising a mixture of phospholipid and cholesterol may comprise a phospholipid such as DPPC or DSPC at about 7 mol % (or any fraction thereof), *e.g.,* in a mixture with cholesterol or a cholesterol derivative at about 32 mol % (or any fraction thereof) of the total lipid present in the particle.

In other embodiments, the cholesterol component in the mixture may comprise from about 25 mol % to about 45 mol %, from about 25 mol % to about 40 mol %, from about 30 mol % to about 45 mol %, from about 30 mol % to about 40 mol %, from about 27 mol % to about 37 mol %, from about 25 mol % to about 30 mol %, or from about 35 mol % to about 40 mol % (or any fraction thereof or range therein) of the total lipid present in the particle. In certain preferred embodiments, the cholesterol component in the mixture comprises from about 25 mol % to about 35 mol %, from about 27 mol % to about 35 mol %, from about 29 mol % to about 35 mol %, from about 30 mol % to about 35 mol %, from about 30 mol % to about 34 mol %, from about 31 mol % to about 33 mol %, or about 30 mol %, 31 mol %, 32 mol %, 33 mol %, 34 mol %, or 35 mol % (or any fraction thereof or range therein) of the total lipid present in the particle. In other embodiments, the cholesterol component in the mixture comprises about 36, 37, 38, 39, 40, 41, 42, 43, 44, or 45 mol % (or any fraction thereof or range therein) of the total lipid present in the particle. Typically, a 1:57 lipid particle formulation comprising a mixture of phospholipid and cholesterol may comprise cholesterol or a cholesterol derivative at about 34 mol % (or any fraction thereof), *e.g.,* in a mixture with a phospholipid such as DPPC or DSPC at about 7 mol % (or any fraction thereof) of the total lipid present in the particle. Typically, a 7:54 lipid particle formulation comprising a mixture of phospholipid and cholesterol may comprise cholesterol or a cholesterol derivative at about 32 mol % (or any fraction thereof), *e.g.,* in a mixture with a phospholipid such as DPPC or DSPC at about 7 mol % (or any fraction thereof) of the total lipid present in the particle.

In embodiments where the lipid particles are phospholipid-free, the cholesterol or derivative thereof may comprise up to about 25 mol %, 30 mol %, 35 mol %, 40 mol %, 45 mol %, 50 mol %, 55 mol %, or 60 mol % of the total lipid present in the particle.

In some embodiments, the cholesterol or derivative thereof in the phospholipid-free lipid particle formulation may comprise from about 25 mol % to about 45 mol %, from about 25 mol % to about 40 mol %, from about 30 mol % to about 45 mol %, from about 30 mol % to about 40 mol %, from about 31 mol % to about 39 mol %, from about 32 mol % to about 38 mol %, from about 33 mol % to about 37 mol %, from about 35 mol % to about 45 mol %, from about 30 mol % to about 35 mol %, from about 35 mol % to about 40 mol %, or about 30 mol %, 31 mol %, 32 mol %, 33 mol %, 34 mol %, 35 mol %, 36 mol %, 37 mol %, 38 mol %, 39 mol %, 40 mol %, 41 mol %, 42 mol %, 43 mol %, 44 mol %, or 45 mol % (or any fraction thereof or range therein) of the total lipid present in the particle. As a non-limiting example, a 1:62 lipid particle formulation may comprise cholesterol at about 37 mol % (or any fraction thereof) of the total lipid present in the particle. As another non-limiting example, a 7:58 lipid particle formulation may comprise cholesterol at about 35 mol % (or any fraction thereof) of the total lipid present in the particle.

In other embodiments, the non-cationic lipid comprises from about 5 mol % to about 90 mol %, from about 10 mol % to about 85 mol %, from about 20 mol % to about 80 mol %, about 10 mol % (*e.g.,* phospholipid only), or about 60 mol % (*e.g.,* phospholipid and cholesterol or derivative thereof) (or any fraction thereof or range therein) of the total lipid present in the particle.

Additional percentages and ranges of non-cationic lipids suitable for use in the lipid particles of the present invention are described in PCT Publication No. WO 09/127060, U.S. Publication No. 20110071208, and U.S. Publication No. 20110076335.

It should be understood that the percentage of non-cationic lipid present in the lipid particles of the invention is a target amount, and that the actual amount of non-cationic lipid present in the formulation may vary, for example, by ± 5 mol %. For example, in the 1:57 lipid particle (*e.g.,* SNALP) formulation, the target amount of phospholipid is 7.1 mol % and the target amount of cholesterol is 34.3 mol %, but the actual amount of phospholipid may be ± 2 mol %, ± 1.5 mol %, ± 1 mol %, ± 0.75 mol %, ± 0.5 mol %, ± 0.25 mol %, or ± 0.1 mol % of that target amount, and the actual amount of cholesterol may be ± 3 mol %, ± 2 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle). Similarly, in the 7:54 lipid particle (*e.g.,* SNALP) formulation, the target amount of phospholipid is 6.75 mol % and the target amount of cholesterol is 32.43 mol %, but the actual amount of phospholipid may be ± 2 mol %, ± 1.5 mol %, ± 1 mol %, ± 0.75 mol %, ± 0.5 mol %, ± 0.25 mol %, or ± 0.1 mol % of that target amount, and the actual amount of cholesterol may be ± 3 mol %, ± 2 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle).

### C. Lipid Conjugates

In addition to cationic and non-cationic lipids, the lipid particles of the invention (*e.g.,* SNALP) may further comprise a lipid conjugate. The conjugated lipid is useful in that it prevents the aggregation of particles. Suitable conjugated lipids include, but are not limited to, PEG-lipid conjugates, POZ-lipid conjugates, ATTA-lipid conjugates, cationic-polymer-lipid conjugates (CPLs), and mixtures thereof. In certain embodiments, the lipid particles comprise either a PEG-lipid conjugate or an ATTA-lipid conjugate together with a CPL. The term "ATTA" or "polyamide" includes, without limitation, compounds described in U.S. Patent Nos. 6,320,017 and 6,586,559.

In a preferred embodiment, the lipid conjugate is a PEG-lipid. Examples of PEG-lipids include, but are not limited to, PEG coupled to dialkyloxypropyls (PEG-DAA) as described in, *e.g.,* PCT Publication No. WO 05/026372, PEG coupled to diacylglycerol (PEG-DAG) as described in, *e.g.,* U.S. Patent Publication Nos. 20030077829 and 2005008689, PEG coupled to phospholipids such as phosphatidylethanolamine (PEG-PE), PEG conjugated to ceramides as described in, *e.g.,* U.S. Patent No. 5,885,613, PEG conjugated to cholesterol or a derivative thereof, and mixtures thereof.

Additional PEG-lipids suitable for use in the invention include, without limitation, mPEG2000-1,2-di-O-alkyl-*sn*3-carbomoylglyceride (PEG-C-DOMG). The synthesis of PEG-C-DOMG is described in PCT Publication No. WO 09/086558. Yet additional suitable PEG-lipid conjugates include, without limitation, 1-[8'-(1,2-dimyristoyl-3-propanoxy)-carboxamido-3',6'-dioxaoctanyl]carbamoyl-ω-methyl-poly(ethylene glycol) (2KPEG-DMG). The synthesis of 2KPEG-DMG is described in U.S. Patent No. 7,404,969.

PEG is a linear, water-soluble polymer of ethylene PEG repeating units with two terminal hydroxyl groups. PEGs are classified by their molecular weights; for example, PEG 2000 has an average molecular weight of about 2,000 daltons, and PEG 5000 has an average molecular weight of about 5,000 daltons. PEGs are commercially available from Sigma Chemical Co. and other companies and include, but are not limited to, the following: monomethoxypolyethylene glycol (MePEG-OH), monomethoxypolyethylene glycol-succinate (MePEG-S), monomethoxypolyethylene glycol-succinimidyl succinate (MePEG-S-NHS), monomethoxypolyethylene glycol-amine (MePEG-NH₂), monomethoxypolyethylene glycol-tresylate (MePEG-TRES), monomethoxypolyethylene glycol-imidazolyl-carbonyl (MePEG-IM), as well as such compounds containing a terminal hydroxyl group instead of a terminal methoxy group (*e*.*g*., HO-PEG-S, HO-PEG-S-NHS, HO-PEG-NH₂, *etc*.). Other PEGs such as those described in U.S. Patent Nos. 6,774,180 and 7,053,150 (*e.g.,* mPEG (20 KDa) amine) are also useful for preparing the PEG-lipid conjugates of the present invention. In addition, monomethoxypolyethyleneglycol-acetic acid (MePEG-CH2COOH) is particularly useful for preparing PEG-lipid conjugates including, *e.g.,* PEG-DAA conjugates.

The PEG moiety of the PEG-lipid conjugates described herein may comprise an average molecular weight ranging from about 550 daltons to about 10,000 daltons. In certain instances, the PEG moiety has an average molecular weight of from about 750 daltons to about 5,000 daltons (*e.g.,* from about 1,000 daltons to about 5,000 daltons, from about 1,500 daltons to about 3,000 daltons, from about 750 daltons to about 3,000 daltons, from about 750 daltons to about 2,000 daltons, *etc.*)*.* In other instances, the PEG moiety has an average molecular weight of from about 550 daltons to about 1000 daltons, from about 250 daltons to about 1000 daltons, from about 400 daltons to about 1000 daltons, from about 600 daltons to about 900 daltons, from about 700 daltons to about 800 daltons, or about 200, 250, 300, 350, 400, 450, 500, 550, 600, 650, 700, 750, 800, 850, 900, 950, or 1000 daltons. In preferred embodiments, the PEG moiety has an average molecular weight of about 2,000 daltons or about 750 daltons.

In certain instances, the PEG can be optionally substituted by an alkyl, alkoxy, acyl, or aryl group. The PEG can be conjugated directly to the lipid or may be linked to the lipid via a linker moiety. Any linker moiety suitable for coupling the PEG to a lipid can be used including, *e.g.,* non-ester containing linker moieties and ester-containing linker moieties. In a preferred embodiment, the linker moiety is a non-ester containing linker moiety. As used herein, the term "non-ester containing linker moiety" refers to a linker moiety that does not contain a carboxylic ester bond (-OC(O)-). Suitable non-ester containing linker moieties include, but are not limited to, amido (-C(O)NH-), amino (-NR-), carbonyl (-C(O)-), carbamate (-NHC(O)O-), urea (-NHC(O)NH-), disulphide (-S-S-), ether (-O-), succinyl (-(O)CCH₂CH₂C(O)-), succinamidyl (-NHC(O)CH₂CH₂C(O)NH-), ether, disulphide, as well as combinations thereof (such as a linker containing both a carbamate linker moiety and an amido linker moiety). In a preferred embodiment, a carbamate linker is used to couple the PEG to the lipid.

In other embodiments, an ester containing linker moiety is used to couple the PEG to the lipid. Suitable ester containing linker moieties include, *e.g.,* carbonate (-OC(O)O-), succinoyl, phosphate esters (-O-(O)POH-O-), sulfonate esters, and combinations thereof.

Phosphatidylethanolamines having a variety of acyl chain groups of varying chain lengths and degrees of saturation can be conjugated to PEG to form the lipid conjugate. Such phosphatidylethanolamines are commercially available, or can be isolated or synthesized using conventional techniques known to those of skilled in the art. Phosphatidylethanolamines containing saturated or unsaturated fatty acids with carbon chain lengths in the range of Cio to C₂₀ are preferred. Phosphatidylethanolamines with mono- or diunsaturated fatty acids and mixtures of saturated and unsaturated fatty acids can also be used. Suitable phosphatidylethanolamines include, but are not limited to, dimyristoyl-phosphatidylethanolamine (DMPE), dipalmitoyl-phosphatidylethanolamine (DPPE), dioleoylphosphatidylethanolamine (DOPE), and distearoyl-phosphatidylethanolamine (DSPE).

The term "diacylglycerol" or "DAG" includes a compound having 2 fatty acyl chains, R¹ and R², both of which have independently between 2 and 30 carbons bonded to the 1- and 2-position of glycerol by ester linkages. The acyl groups can be saturated or have varying degrees of unsaturation. Suitable acyl groups include, but are not limited to, lauroyl (C12), myristoyl (C14), palmitoyl (C₁₆), stearoyl (C₁₈), and icosoyl (C20). In preferred embodiments, R¹ and R² are the same, *i*.*e*., R¹ and R² are both myristoyl (*i.e.,* dimyristoyl), R¹ and R² are both stearoyl (*i*.*e*., distearoyl), *etc.* Diacylglycerols have the following general formula:

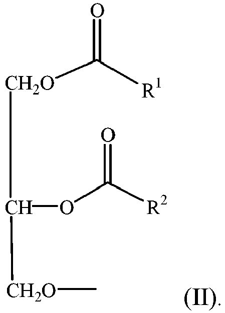

The term "dialkyloxypropyl" or "DAA" includes a compound having 2 alkyl chains, R¹ and R², both of which have independently between 2 and 30 carbons. The alkyl groups can be saturated or have varying degrees of unsaturation. Dialkyloxypropyls have the following general formula:

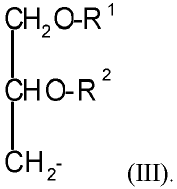

In a preferred embodiment, the PEG-lipid is a PEG-DAA conjugate having the following formula: 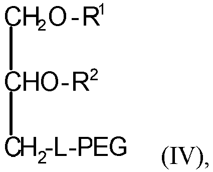 wherein R¹ and R² are independently selected and are long-chain alkyl groups having from about 10 to about 22 carbon atoms; PEG is a polyethyleneglycol; and L is a non-ester containing linker moiety or an ester containing linker moiety as described above. The long-chain alkyl groups can be saturated or unsaturated. Suitable alkyl groups include, but are not limited to, decyl (C₁₀), lauryl (C₁₂), myristyl (C₁₄), palmityl (C₁₆), stearyl (C₁₈), and icosyl (C₂₀). In preferred embodiments, R¹ and R² are the same, *i.e.,* R¹ and R² are both myristyl (*i.e.,* dimyristyl), R¹ and R² are both stearyl (*i.e.,* distearyl), *etc.*

In Formula IV above, the PEG has an average molecular weight ranging from about 550 daltons to about 10,000 daltons. In certain instances, the PEG has an average molecular weight of from about 750 daltons to about 5,000 daltons (*e.g.,* from about 1,000 daltons to about 5,000 daltons, from about 1,500 daltons to about 3,000 daltons, from about 750 daltons to about 3,000 daltons, from about 750 daltons to about 2,000 daltons, *etc.*). In other instances, the PEG moiety has an average molecular weight of from about 550 daltons to about 1000 daltons, from about 250 daltons to about 1000 daltons, from about 400 daltons to about 1000 daltons, from about 600 daltons to about 900 daltons, from about 700 daltons to about 800 daltons, or about 200, 250, 300, 350, 400, 450, 500, 550, 600, 650, 700, 750, 800, 850, 900, 950, or 1000 daltons. In preferred embodiments, the PEG has an average molecular weight of about 2,000 daltons or about 750 daltons. The PEG can be optionally substituted with alkyl, alkoxy, acyl, or aryl groups. In certain embodiments, the terminal hydroxyl group is substituted with a methoxy or methyl group.

In a preferred embodiment, "L" is a non-ester containing linker moiety. Suitable non-ester containing linkers include, but are not limited to, an amido linker moiety, an amino linker moiety, a carbonyl linker moiety, a carbamate linker moiety, a urea linker moiety, an ether linker moiety, a disulphide linker moiety, a succinamidyl linker moiety, and combinations thereof. In a preferred embodiment, the non-ester containing linker moiety is a carbamate linker moiety (*i.e.,* a PEG-C-DAA conjugate). In another preferred embodiment, the non-ester containing linker moiety is an amido linker moiety (*i.e.,* a PEG-*A*-DAA conjugate). In yet another preferred embodiment, the non-ester containing linker moiety is a succinamidyl linker moiety (*i.e.,* a PEG-*S*-DAA conjugate).

In particular embodiments, the PEG-lipid conjugate is selected from: 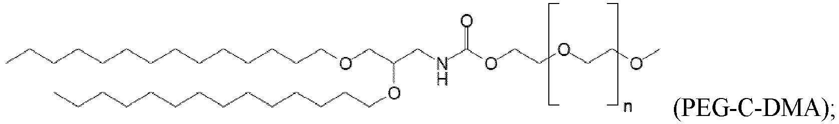 and

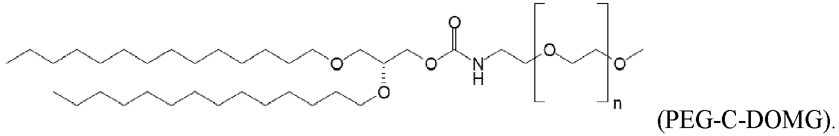

The PEG-DAA conjugates are synthesized using standard techniques and reagents known to those of skill in the art. It will be recognized that the PEG-DAA conjugates will contain various amide, amine, ether, thio, carbamate, and urea linkages. Those of skill in the art will recognize that methods and reagents for forming these bonds are well known and readily available. *See, e.g.,* March, ADVANCED ORGANIC CHEMISTRY (Wiley 1992); Larock, COMPREHENSIVE ORGANIC TRANSFORMATIONS (VCH 1989); and Furniss, VOGEL'S TEXTBOOK OF PRACTICAL ORGANIC CHEMISTRY, 5th ed. (Longman 1989). It will also be appreciated that any functional groups present may require protection and deprotection at different points in the synthesis of the PEG-DAA conjugates. Those of skill in the art will recognize that such techniques are well known. *See, e.g.,* Green and Wuts, PROTECTIVE GROUPS IN ORGANIC SYNTHESIS (Wiley 1991).

Preferably, the PEG-DAA conjugate is a PEG-didecyloxypropyl (Cio) conjugate, a PEG-dilauryloxypropyl (C₁₂) conjugate, a PEG-dimyristyloxypropyl (C₁₄) conjugate, a PEG-dipalmityloxypropyl (C₁₆) conjugate, or a PEG-distearyloxypropyl (C₁₈) conjugate. In these embodiments, the PEG preferably has an average molecular weight of about 750 or about 2,000 daltons. In one particularly preferred embodiment, the PEG-lipid conjugate comprises PEG2000-C-DMA, wherein the "2000" denotes the average molecular weight of the PEG, the "C" denotes a carbamate linker moiety, and the "DMA" denotes dimyristyloxypropyl. In another particularly preferred embodiment, the PEG-lipid conjugate comprises PEG750-C-DMA, wherein the "750" denotes the average molecular weight of the PEG, the "C" denotes a carbamate linker moiety, and the "DMA" denotes dimyristyloxypropyl. In particular embodiments, the terminal hydroxyl group of the PEG is substituted with a methyl group. Those of skill in the art will readily appreciate that other dialkyloxypropyls can be used in the PEG-DAA conjugates of the present invention.

In addition to the foregoing, it will be readily apparent to those of skill in the art that other hydrophilic polymers can be used in place of PEG. Examples of suitable polymers that can be used in place of PEG include, but are not limited to, polyvinylpyrrolidone, polymethyloxazoline, polyethyloxazoline, polyhydroxypropyl methacrylamide, polymethacrylamide and polydimethylacrylamide, polylactic acid, polyglycolic acid, and derivatized celluloses such as hydroxymethylcellulose or hydroxyethylcellulose.

In addition to the foregoing components, the lipid particles (*e.g*., SNALP) of the present invention can further comprise cationic poly(ethylene glycol) (PEG) lipids or CPLs (*see, e.g.,* Chen et al., Bioconj. Chem., 11:433-437 (2000); U.S. Patent No. 6,852,334; PCT Publication No. WO 00/62813).

In some embodiments, the lipid conjugate (*e.g.*, PEG-lipid) comprises from about 0.1 mol % to about 2 mol %, from about 0.5 mol % to about 2 mol %, from about 1 mol % to about 2 mol %, from about 0.6 mol % to about 1.9 mol %, from about 0.7 mol % to about 1.8 mol %, from about 0.8 mol % to about 1.7 mol %, from about 0.9 mol % to about 1.6 mol %, from about 0.9 mol % to about 1.8 mol %, from about 1 mol % to about 1.8 mol %, from about 1 mol % to about 1.7 mol %, from about 1.2 mol % to about 1.8 mol %, from about 1.2 mol % to about 1.7 mol %, from about 1.3 mol % to about 1.6 mol %, from about 1.4 mol % to about 1.5 mol %, or about 1, 1.1, 1.2, 1.3, 1.4, 1.5, 1.6, 1.7, 1.8, 1.9, or 2 mol % (or any fraction thereof or range therein) of the total lipid present in the particle.

In other embodiments, the lipid conjugate (*e.g.,* PEG-lipid) comprises from about 0 mol % to about 20 mol %, from about 0.5 mol % to about 20 mol %, from about 2 mol % to about 20 mol %, from about 1.5 mol % to about 18 mol %, from about 2 mol % to about 15 mol %, from about 4 mol % to about 15 mol %, from about 2 mol % to about 12 mol %, from about 5 mol % to about 12 mol %, or about 2 mol % (or any fraction thereof or range therein) of the total lipid present in the particle.

In further embodiments, the lipid conjugate (*e.g.,* PEG-lipid) comprises from about 4 mol % to about 10 mol %, from about 5 mol % to about 10 mol %, from about 5 mol % to about 9 mol %, from about 5 mol % to about 8 mol %, from about 6 mol % to about 9 mol %, from about 6 mol % to about 8 mol %, or about 5 mol %, 6 mol %, 7 mol %, 8 mol %, 9 mol %, or 10 mol % (or any fraction thereof or range therein) of the total lipid present in the particle.

Additional examples, percentages, and/or ranges of lipid conjugates suitable for use in the lipid particles of the invention are described in PCT Publication No. WO 09/127060, U.S. Publication No. 20110071208, U.S. Publication No. 20110076335, U.S. PublicationNo. 2011-0313017, filed January 13, 2011, and PCT Publication No. WO 2010/006282.

It should be understood that the percentage of lipid conjugate (*e.g.,* PEG-lipid) present in the lipid particles of the invention is a target amount, and that the actual amount of lipid conjugate present in the formulation may vary, for example, by ± 2 mol %. For example, in the 1:57 lipid particle (*e.g.,* SNALP) formulation, the target amount of lipid conjugate is 1.4 mol %, but the actual amount of lipid conjugate may be ± 0.5 mol %, ± 0.4 mol %, ± 0.3 mol %, ± 0.2 mol %, ± 0.1 mol %, or ± 0.05 mol % of that target amount, with the balance of the formulation being made up of other lipid components (adding up to 100 mol % of total lipids present in the particle). Similarly, in the 7:54 lipid particle (*e.g.,* SNALP) formulation, the target amount of lipid conjugate is 6.76 mol %, but the actual amount of lipid conjugate may be ± 2 mol %, ± 1.5 mol %, ± 1 mol %, ± 0.75 mol %, ± 0.5 mol %, ± 0.25 mol %, or ± 0.1 mol % of that target amount, with the balance of the formulation being made up of other lipid components (adding up to 100 mol % of total lipids present in the particle).

One of ordinary skill in the art will appreciate that the concentration of the lipid conjugate can be varied depending on the lipid conjugate employed and the rate at which the lipid particle is to become fusogenic.

By controlling the composition and concentration of the lipid conjugate, one can control the rate at which the lipid conjugate exchanges out of the lipid particle and, in turn, the rate at which the lipid particle becomes fusogenic. For instance, when a PEG-DAA conjugate is used as the lipid conjugate, the rate at which the lipid particle becomes fusogenic can be varied, for example, by varying the concentration of the lipid conjugate, by varying the molecular weight of the PEG, or by varying the chain length and degree of saturation of the alkyl groups on the PEG-DAA conjugate. In addition, other variables including, for example, pH, temperature, ionic strength, *etc.* can be used to vary and/or control the rate at which the lipid particle becomes fusogenic. Other methods which can be used to control the rate at which the lipid particle becomes fusogenic will become apparent to those of skill in the art upon reading this disclosure. Also, by controlling the composition and concentration of the lipid conjugate, one can control the lipid particle (*e.g.*, SNALP) size.

### VI. Preparation of Lipid Particles

The lipid particles of the present invention, *e.g.,* SNALP, in which an active agent such as a nucleic acid (*e.g.,* an interfering RNA such as an siRNA) is entrapped within the lipid portion of the particle and is protected from degradation, can be formed by any method known in the art including, but not limited to, a continuous mixing method, a direct dilution process, and an in-line dilution process. In certain embodiments, one or more antioxidants such as metal chelators (*e.g.*, EDTA), primary antioxidants, and/or secondary antioxidants may be included at any step or at multiple steps in the process (*e.g.*, prior to, during, and/or after lipid particle formation) as described in PCT Publication No. WO/2011/066651.

In particular embodiments, the cationic lipids may comprise at least one, two, three, four, five, or more cationic lipids such as those set forth in Formula I or salts thereof, alone or in combination with other cationic lipid species. In other embodiments, the non-cationic lipids may comprise one, two, or more lipids including egg sphingomyelin (ESM), distearoylphosphatidylcholine (DSPC), dioleoylphosphatidylcholine (DOPC), 1-palmitoyl-2-oleoyl-phosphatidylcholine (POPC), dipalmitoyl-phosphatidylcholine (DPPC), monomethyl-phosphatidylethanolamine, dimethyl-phosphatidylethanolamine, 14:0 PE (1,2-dimyristoyl-phosphatidylethanolamine (DMPE)), 16:0 PE (1,2-dipalmitoyl-phosphatidylethanolamine (DPPE)), 18:0 PE (1,2-distearoyl-phosphatidylethanolamine (DSPE)), 18:1 PE (1,2-dioleoyl-phosphatidylethanolamine (DOPE)), 18:1 trans PE (1,2-dielaidoyl-phosphatidylethanolamine (DEPE)), 18:0-18:1 PE (1-stearoyl-2-oleoyl-phosphatidylethanolamine (SOPE)), 16:0-18:1 PE (1-palmitoyl-2-oleoyl-phosphatidylethanolamine (POPE)), polyethylene glycol-based polymers (*e.g.*, PEG 2000, PEG 5000, PEG-modified diacylglycerols, or PEG-modified dialkyloxypropyls), cholesterol, derivatives thereof, or combinations thereof.

In certain embodiments, the present invention provides nucleic acid-lipid particles (*e.g.,* SNALP) produced via a continuous mixing method, *e.g.,* a process that includes providing an aqueous solution comprising a nucleic acid (*e.g.*, interfering RNA) in a first reservoir, providing an organic lipid solution in a second reservoir (wherein the lipids present in the organic lipid solution are solubilized in an organic solvent, *e.g.,* a lower alkanol such as ethanol), and mixing the aqueous solution with the organic lipid solution such that the organic lipid solution mixes with the aqueous solution so as to substantially instantaneously produce a lipid vesicle (*e.g.,* liposome) encapsulating the nucleic acid within the lipid vesicle. This process and the apparatus for carrying out this process are described in detail in U.S. Patent Publication No. 20040142025.

The action of continuously introducing lipid and buffer solutions into a mixing environment, such as in a mixing chamber, causes a continuous dilution of the lipid solution with the buffer solution, thereby producing a lipid vesicle substantially instantaneously upon mixing. As used herein, the phrase "continuously diluting a lipid solution with a buffer solution" (and variations) generally means that the lipid solution is diluted sufficiently rapidly in a hydration process with sufficient force to effectuate vesicle generation. By mixing the aqueous solution comprising a nucleic acid with the organic lipid solution, the organic lipid solution undergoes a continuous stepwise dilution in the presence of the buffer solution (*i.e.,* aqueous solution) to produce a nucleic acid-lipid particle.

The nucleic acid-lipid particles formed using the continuous mixing method typically have a size of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, less than about 120 nm, 110 nm, 100 nm, 90 nm, or 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm (or any fraction thereof or range therein). The particles thus formed do not aggregate and are optionally sized to achieve a uniform particle size.

In another embodiment, the present invention provides nucleic acid-lipid particles (*e.g.,* SNALP) produced via a direct dilution process that includes forming a lipid vesicle (*e.g.*, liposome) solution and immediately and directly introducing the lipid vesicle solution into a collection vessel containing a controlled amount of dilution buffer. In preferred aspects, the collection vessel includes one or more elements configured to stir the contents of the collection vessel to facilitate dilution. In one aspect, the amount of dilution buffer present in the collection vessel is substantially equal to the volume of lipid vesicle solution introduced thereto. As a non-limiting example, a lipid vesicle solution in 45% ethanol when introduced into the collection vessel containing an equal volume of dilution buffer will advantageously yield smaller particles.

In yet another embodiment, the present invention provides nucleic acid-lipid particles (*e.g.*, SNALP) produced via an in-line dilution process in which a third reservoir containing dilution buffer is fluidly coupled to a second mixing region. In this embodiment, the lipid vesicle (*e.g.*, liposome) solution formed in a first mixing region is immediately and directly mixed with dilution buffer in the second mixing region. In preferred aspects, the second mixing region includes a T-connector arranged so that the lipid vesicle solution and the dilution buffer flows meet as opposing 180° flows; however, connectors providing shallower angles can be used, *e.g.,* from about 27° to about 180° (*e.g.,* about 90°). A pump mechanism delivers a controllable flow of buffer to the second mixing region. In one aspect, the flow rate of dilution buffer provided to the second mixing region is controlled to be substantially equal to the flow rate of lipid vesicle solution introduced thereto from the first mixing region. This embodiment advantageously allows for more control of the flow of dilution buffer mixing with the lipid vesicle solution in the second mixing region, and therefore also the concentration of lipid vesicle solution in buffer throughout the second mixing process. Such control of the dilution buffer flow rate advantageously allows for small particle size formation at reduced concentrations.

These processes and the apparatuses for carrying out these direct dilution and in-line dilution processes are described in detail in U.S. Patent Publication No. 20070042031.

The nucleic acid-lipid particles formed using the direct dilution and in-line dilution processes typically have a size of from about 30 nm to about 150 nm, from about 40 nm to about 150 nm, from about 50 nm to about 150 nm, from about 60 nm to about 130 nm, from about 70 nm to about 110 nm, from about 70 nm to about 100 nm, from about 80 nm to about 100 nm, from about 90 nm to about 100 nm, from about 70 to about 90 nm, from about 80 nm to about 90 nm, from about 70 nm to about 80 nm, less than about 120 nm, 110 nm, 100 nm, 90 nm, or 80 nm, or about 30 nm, 35 nm, 40 nm, 45 nm, 50 nm, 55 nm, 60 nm, 65 nm, 70 nm, 75 nm, 80 nm, 85 nm, 90 nm, 95 nm, 100 nm, 105 nm, 110 nm, 115 nm, 120 nm, 125 nm, 130 nm, 135 nm, 140 nm, 145 nm, or 150 nm (or any fraction thereof or range therein). The particles thus formed do not aggregate and are optionally sized to achieve a uniform particle size.

If needed, the lipid particles of the invention (*e.g.*, SNALP) can be sized by any of the methods available for sizing liposomes. The sizing may be conducted in order to achieve a desired size range and relatively narrow distribution of particle sizes.

Several techniques are available for sizing the particles to a desired size. One sizing method, used for liposomes and equally applicable to the present particles, is described in U.S. Patent No. 4,737,323. Sonicating a particle suspension either by bath or probe sonication produces a progressive size reduction down to particles of less than about 50 nm in size. Homogenization is another method which relies on shearing energy to fragment larger particles into smaller ones. In a typical homogenization procedure, particles are recirculated through a standard emulsion homogenizer until selected particle sizes, typically between about 60 and about 80 nm, are observed. In both methods, the particle size distribution can be monitored by conventional laser-beam particle size discrimination, or QELS.

Extrusion of the particles through a small-pore polycarbonate membrane or an asymmetric ceramic membrane is also an effective method for reducing particle sizes to a relatively well-defined size distribution. Typically, the suspension is cycled through the membrane one or more times until the desired particle size distribution is achieved. The particles may be extruded through successively smaller-pore membranes, to achieve a gradual reduction in size.

In some embodiments, the nucleic acids present in the particles are precondensed as described in, *e.g.,* U.S. Patent Application No. 09/744,103.

In other embodiments, the methods may further comprise adding non-lipid polycations which are useful to effect the lipofection of cells using the present compositions. Examples of suitable non-lipid polycations include, hexadimethrine bromide (sold under the brand name POLYBRENE®, from Aldrich Chemical Co., Milwaukee, Wisconsin, USA) or other salts of hexadimethrine. Other suitable polycations include, for example, salts of poly-L-ornithine, poly-L-arginine, poly-L-lysine, poly-D-lysine, polyallylamine, and polyethyleneimine. Addition of these salts is preferably after the particles have been formed.

In some embodiments, the nucleic acid to lipid ratios (mass/mass ratios) in a formed nucleic acid-lipid particle (*e.g.,* SNALP) will range from about 0.01 to about 0.2, from about 0.05 to about 0.2, from about 0.02 to about 0.1, from about 0.03 to about 0.1, or from about 0.01 to about 0.08. The ratio of the starting materials (input) also falls within this range. In other embodiments, the particle preparation uses about 400 µg nucleic acid per 10 mg total lipid or a nucleic acid to lipid mass ratio of about 0.01 to about 0.08 and, more preferably, about 0.04, which corresponds to 1.25 mg of total lipid per 50 µg of nucleic acid. In other preferred embodiments, the particle has a nucleic acid:lipid mass ratio of about 0.08.

In other embodiments, the lipid to nucleic acid ratios (mass/mass ratios) in a formed nucleic acid-lipid particle (*e.g.,* SNALP) will range from about 1 (1:1) to about 100 (100:1), from about 5 (5:1) to about 100 (100:1), from about 1 (1:1) to about 50 (50:1), from about 2 (2:1) to about 50 (50:1), from about 3 (3:1) to about 50 (50:1), from about 4 (4:1) to about 50 (50:1), from about 5 (5:1) to about 50 (50:1), from about 1 (1:1) to about 25 (25:1), from about 2 (2:1) to about 25 (25:1), from about 3 (3:1) to about 25 (25:1), from about 4 (4:1) to about 25 (25:1), from about 5 (5:1) to about 25 (25:1), from about 5 (5:1) to about 20 (20:1), from about 5 (5:1) to about 15 (15:1), from about 5 (5:1) to about 10 (10:1), or about 5 (5:1), 6 (6:1), 7 (7:1), 8 (8:1), 9 (9:1), 10 (10:1), 11 (11:1), 12 (12:1), 13 (13:1), 14 (14:1), 15 (15:1), 16 (16:1), 17 (17:1), 18 (18:1), 19 (19:1), 20 (20:1), 21 (21:1), 22 (22:1), 23 (23:1), 24 (24:1), or 25 (25:1), or any fraction thereof or range therein. The ratio of the starting materials (input) also falls within this range.

As previously discussed, the conjugated lipid may further include a CPL. A variety of general methods for making SNALP-CPLs (CPL-containing SNALP) are discussed herein. Two general techniques include the "post-insertion" technique, that is, insertion of a CPL into, for example, a pre-formed SNALP, and the "standard" technique, wherein the CPL is included in the lipid mixture during, for example, the SNALP formation steps. The post-insertion technique results in SNALP having CPLs mainly in the external face of the SNALP bilayer membrane, whereas standard techniques provide SNALP having CPLs on both internal and external faces. The method is especially useful for vesicles made from phospholipids (which can contain cholesterol) and also for vesicles containing PEG-lipids (such as PEG-DAAs and PEG-DAGs). Methods of making SNALP-CPLs are taught, for example, in U.S. Patent Nos. 5,705,385; 6,586,410; 5,981,501; 6,534,484; and 6,852,334; U.S. Patent Publication No. 20020072121; and PCT Publication No. WO 00/62813.

### VII. Kits

Lipid particles (*e.g.,* SNALP) in kit form are described herein. In some cases, the kit comprises a container which is compartmentalized for holding the various elements of the lipid particles (*e.g.,* the active agents or therapeutic agents such as nucleic acids and the individual lipid components of the particles). Preferably, the kit comprises a container (*e.g.,* a vial or ampoule) which holds the lipid particles of the invention (*e.g.,* SNALP), wherein the particles are produced by one of the processes set forth herein. In some cases, the kit may further comprise one or more antioxidants such as metal chelators (*e.g.,* EDTA), primary antioxidants, and/or secondary antioxidants. In other cases, the kit may further comprise an endosomal membrane destabilizer (*e.g.,* calcium ions). The kit typically contains the particle compositions of the present invention, either as a suspension in a pharmaceutically acceptable carrier or in dehydrated form, with instructions for their rehydration (if lyophilized) and administration.

The lipid particles of the present invention can be tailored to preferentially target particular tissues, organs, or tumors of interest. In certain instances, preferential targeting of lipid particles such as SNALP may be carried out by controlling the composition of the particle itself. In some instances, the 1:57 lipid particle (*e.g.,* SNALP) formulation can be used to preferentially target the liver (*e.g.,* normal liver tissue). In other instances, the 7:54 lipid particle (*e.g.*, SNALP) formulation can be used to preferentially target solid tumors such as liver tumors and tumors outside of the liver. In preferred cases, the kits of the invention comprise these liver-directed and/or tumor-directed lipid particles, wherein the particles are present in a container as a suspension or in dehydrated form.

In certain instances, it may be desirable to have a targeting moiety attached to the surface of the lipid particle to further enhance the targeting of the particle. Methods of attaching targeting moieties (*e.g.,* antibodies, proteins, *etc.*) to lipids (such as those used in the present particles) are known to those of skill in the art.

### VIII. Administration of Lipid Particles

Once formed, the lipid particles of the invention (*e.g*., SNALP) are useful for the introduction of active agents or therapeutic agents (*e.g*., nucleic acids such as interfering RNA) into cells. Accordingly, the present invention also provides in vitro methods for introducing an active agent or therapeutic agent such as a nucleic acid (*e.g*., interfering RNA) into a cell. In some instances, the cell is a liver cell such as, *e.g.,* a hepatocyte present in liver tissue. In other instances, the cell is a tumor cell such as, *e.g.,* a tumor cell present in a solid tumor. The methods are carried out by first forming the particles as described above and then contacting the particles with the cells for a period of time sufficient for delivery of the active agent or therapeutic agent to the cells to occur.

The lipid particles of the invention (*e.g.*, SNALP) can be adsorbed to almost any cell type with which they are mixed or contacted. Once adsorbed, the particles can either be endocytosed by a portion of the cells, exchange lipids with cell membranes, or fuse with the cells. Transfer or incorporation of the active agent or therapeutic agent (*e.g.,* nucleic acid) portion of the particle can take place via any one of these pathways. In particular, when fusion takes place, the particle membrane is integrated into the cell membrane and the contents of the particle combine with the intracellular fluid.

The lipid particles of the invention (*e.g.*, SNALP) can be administered either alone or in a mixture with a pharmaceutically acceptable carrier (*e.g.*, physiological saline or phosphate buffer) selected in accordance with the route of administration and standard pharmaceutical practice. Generally, normal buffered saline (*e.g.,* 135-150 mM NaCl) will be employed as the pharmaceutically acceptable carrier. Other suitable carriers include, *e.g.,* water, buffered water, 0.4% saline, 0.3% glycine, and the like, including glycoproteins for enhanced stability, such as albumin, lipoprotein, globulin, *etc.* Additional suitable carriers are described in, *e.g*., REMINGTON'S PHARMACEUTICAL SCIENCES, Mack Publishing Company, Philadelphia, PA, 17th ed. (1985). As used herein, "carrier" includes any and all solvents, dispersion media, vehicles, coatings, diluents, antibacterial and antifungal agents, isotonic and absorption delaying agents, buffers, carrier solutions, suspensions, colloids, and the like. The phrase "pharmaceutically acceptable" refers to molecular entities and compositions that do not produce an allergic or similar untoward reaction when administered to a human.

The pharmaceutically acceptable carrier is generally added following lipid particle formation. Thus, after the lipid particle (*e.g.*, SNALP) is formed, the particle can be diluted into pharmaceutically acceptable carriers such as normal buffered saline.

The concentration of particles in the pharmaceutical formulations can vary widely, *i.e.,* from less than about 0.05%, usually at or at least about 2 to 5%, to as much as about 10 to 90% by weight, and will be selected primarily by fluid volumes, viscosities, *etc.,* in accordance with the particular mode of administration selected. For example, the concentration may be increased to lower the fluid load associated with treatment. This may be particularly desirable in patients having atherosclerosis-associated congestive heart failure or severe hypertension. Alternatively, particles composed of irritating lipids may be diluted to low concentrations to lessen inflammation at the site of administration.

The pharmaceutical compositions of the present invention may be sterilized by conventional, well-known sterilization techniques. Aqueous solutions can be packaged for use or filtered under aseptic conditions and lyophilized, the lyophilized preparation being combined with a sterile aqueous solution prior to administration. The compositions can contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, and calcium chloride. Additionally, the particle suspension may include lipid-protective agents which protect lipids against free-radical and lipid-peroxidative damages on storage. Lipophilic free-radical quenchers, such as alphatocopherol, and water-soluble iron-specific chelators, such as ferrioxamine, are suitable.

In some embodiments, the lipid particles of the invention (e.g., SNALP) are particularly useful in methods for the therapeutic delivery of one or more nucleic acids comprising an interfering RNA sequence (*e.g.*, siRNA). In particular, it is an object of this invention to provide lipis particles for use in methods for treatment of a disease or disorder in a mammal (*e.g.*, a rodent such as a mouse or a primate such as a human, chimpanzee, or monkey) by downregulating or silencing the transcription and/or translation of one or more target nucleic acid sequences or genes of interest. As a non-limiting example, the methods of the invention are useful for *in vivo* delivery of interfering RNA (*e.g.,* siRNA) to the liver and/or tumor of a mammalian subject. In certain embodiments, the disease or disorder is associated with expression and/or overexpression of a gene and expression or overexpression of the gene is reduced by the interfering RNA (*e.g.,* siRNA). In certain other embodiments, a therapeutically effective amount of the lipid particle may be administered to the mammal. In some instances, an interfering RNA (*e.g.,* siRNA) is formulated into a SNALP, and the particles are administered to patients requiring such treatment. In other instances, cells are removed from a patient, the interfering RNA is delivered *in vitro* (*e.g.,* using a SNALP described herein), and the cells are reinjected into the patient.

### A. In vivo Administration

Systemic delivery for *in vivo* therapy, *e.g.,* delivery of a therapeutic nucleic acid to a distal target cell via body systems such as the circulation, has been achieved using nucleic acid-lipid particles such as those described in PCT Publication Nos. WO 05/007196, WO 05/121348, WO 05/120152, and WO 04/002453. The present invention also provides fully encapsulated lipid particles that protect the nucleic acid from nuclease degradation in serum, are non-immunogenic, are small in size, and are suitable for repeat dosing.

For *in vivo* administration, administration can be in any manner known in the art, *e.g.,* by injection, oral administration, inhalation (*e.g.,* intransal or intratracheal), transdermal application, or rectal administration. Administration can be accomplished via single or divided doses. The pharmaceutical compositions can be administered parenterally, *i.e.,* intraarticularly, intravenously, intraperitoneally, subcutaneously, or intramuscularly. In some embodiments, the pharmaceutical compositions are administered intravenously or intraperitoneally by a bolus injection (*see, e.g.,* U.S. Patent No. 5,286,634). Intracellular nucleic acid delivery has also been discussed in Straubringer et al., Methods Enzymol., 101:512 (1983); Mannino et al., Biotechniques, 6:682 (1988); Nicolau et al., Crit. Rev. Ther. Drug Carrier Syst., 6:239 (1989); and Behr, Acc. Chem. Res., 26:274 (1993). Still other methods of administering lipid-based therapeutics are described in, for example, U.S. Patent Nos. 3,993,754; 4,145,410; 4,235,871; 4,224,179; 4,522,803; and 4,588,578. The lipid particles can be administered by direct injection at the site of disease or by injection at a site distal from the site of disease (*see, e.g.,* Culver, HUMAN GENE THERAPY, MaryAnn Liebert, Inc., Publishers, New York. pp.70-71(1994)).

In embodiments where the lipid particles of the present invention (*e.g.*, SNALP) are administered intravenously, at least about 5%, 10%, 15%, 20%, or 25% of the total injected dose of the particles is present in plasma about 8, 12, 24, 36, or 48 hours after injection. In other embodiments, more than about 20%, 30%, 40% and as much as about 60%, 70% or 80% of the total injected dose of the lipid particles is present in plasma about 8, 12, 24, 36, or 48 hours after injection. In certain instances, more than about 10% of a plurality of the particles is present in the plasma of a mammal about 1 hour after administration. In certain other instances, the presence of the lipid particles is detectable at least about 1 hour after administration of the particle. In certain embodiments, the presence of a therapeutic agent such as a nucleic acid is detectable in cells of the lung, liver, tumor, or at a site of inflammation at about 8, 12, 24, 36, 48, 60, 72 or 96 hours after administration. In other embodiments, downregulation of expression of a target sequence by an interfering RNA (*e.g.,* siRNA) is detectable at about 8, 12, 24, 36, 48, 60, 72 or 96 hours after administration. In yet other embodiments, downregulation of expression of a target sequence by an interfering RNA (*e.g.,* siRNA) occurs preferentially in liver cells (*e.g.,* hepatocytes), tumor cells, or in cells at a site of inflammation. In further embodiments, the presence or effect of an interfering RNA (*e.g.,* siRNA) in cells at a site proximal or distal to the site of administration or in cells of the lung, liver, or a tumor is detectable at about 12, 24, 48, 72, or 96 hours, or at about 6, 8, 10, 12, 14, 16, 18, 19, 20, 22, 24, 26, or 28 days after administration. In additional embodiments, the lipid particles (e.g., SNALP) of the invention are administered parenterally or intraperitoneally.

The compositions of the present invention, either alone or in combination with other suitable components, can be made into aerosol formulations (*i.e.,* they can be "nebulized") to be administered via inhalation (*e.g.,* intranasally or intratracheally) (*see,* Brigham et al., Am. J. Sci., 298:278 (1989)). Aerosol formulations can be placed into pressurized acceptable propellants, such as dichlorodifluoromethane, propane, nitrogen, and the like.

In certain embodiments, the pharmaceutical compositions may be delivered by intranasal sprays, inhalation, and/or other aerosol delivery vehicles. Methods for delivering nucleic acid compositions directly to the lungs via nasal aerosol sprays have been described, *e.g.,* in U.S. Patent Nos. 5,756,353 and 5,804,212. Likewise, the delivery of drugs using intranasal microparticle resins and lysophosphatidyl-glycerol compounds (U.S. Patent 5,725,871) are also well-known in the pharmaceutical arts. Similarly, transmucosal drug delivery in the form of a polytetrafluoroetheylene support matrix is described in U.S. Patent No. 5,780,045.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions are preferably administered, for example, by intravenous infusion, orally, topically, intraperitoneally, intravesically, or intrathecally.

Generally, when administered intravenously, the lipid particle formulations are formulated with a suitable pharmaceutical carrier. Many pharmaceutically acceptable carriers may be employed in the compositions and methods of the present invention. Suitable formulations for use in the present invention are found, for example, in REMINGTON'S PHARMACEUTICAL SCIENCES, Mack Publishing Company, Philadelphia, PA, 17th ed. (1985). A variety of aqueous carriers may be used, for example, water, buffered water, 0.4% saline, 0.3% glycine, and the like, and may include glycoproteins for enhanced stability, such as albumin, lipoprotein, globulin, *etc.* Generally, normal buffered saline (135-150 mM NaCl) will be employed as the pharmaceutically acceptable carrier, but other suitable carriers will suffice. These compositions can be sterilized by conventional liposomal sterilization techniques, such as filtration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, *etc.* These compositions can be sterilized using the techniques referred to above or, alternatively, they can be produced under sterile conditions. The resulting aqueous solutions may be packaged for use or filtered under aseptic conditions and lyophilized, the lyophilized preparation being combined with a sterile aqueous solution prior to administration.

In certain applications, the lipid particles disclosed herein may be delivered via oral administration to the individual. The particles may be incorporated with excipients and used in the form of ingestible tablets, buccal tablets, troches, capsules, pills, lozenges, elixirs, mouthwash, suspensions, oral sprays, syrups, wafers, and the like (*see, e.g.,* U.S. Patent Nos. 5,641,515, 5,580,579, and 5,792,451). These oral dosage forms may also contain the following: binders, gelatin; excipients, lubricants, and/or flavoring agents. When the unit dosage form is a capsule, it may contain, in addition to the materials described above, a liquid carrier. Various other materials may be present as coatings or to otherwise modify the physical form of the dosage unit. Of course, any material used in preparing any unit dosage form should be pharmaceutically pure and substantially non-toxic in the amounts employed.

Typically, these oral formulations may contain at least about 0.1% of the lipid particles or more, although the percentage of the particles may, of course, be varied and may conveniently be between about 1% or 2% and about 60% or 70% or more of the weight or volume of the total formulation. Naturally, the amount of particles in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

Formulations suitable for oral administration can consist of: (a) liquid solutions, such as an effective amount of a packaged therapeutic agent such as nucleic acid (*e.g.,* interfering RNA) suspended in diluents such as water, saline, or PEG 400; (b) capsules, sachets, or tablets, each containing a predetermined amount of a therapeutic agent such as nucleic acid (*e.g*., interfering RNA), as liquids, solids, granules, or gelatin; (c) suspensions in an appropriate liquid; and (d) suitable emulsions. Tablet forms can include one or more of lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise a therapeutic agent such as nucleic acid (*e.g.,* interfering RNA) in a flavor, *e.g.,* sucrose, as well as pastilles comprising the therapeutic agent in an inert base, such as gelatin and glycerin or sucrose and acacia emulsions, gels, and the like containing, in addition to the therapeutic agent, carriers known in the art.

In another example of their use, lipid particles can be incorporated into a broad range of topical dosage forms. For instance, a suspension containing nucleic acid-lipid particles such as SNALP can be formulated and administered as gels, oils, emulsions, topical creams, pastes, ointments, lotions, foams, mousses, and the like.

When preparing pharmaceutical preparations of the lipid particles of the invention, it is preferable to use quantities of the particles which have been purified to reduce or eliminate empty particles or particles with therapeutic agents such as nucleic acid associated with the external surface.

The methods may be practiced in a variety of hosts. Preferred hosts include mammalian species, such as primates (*e.g.*, humans and chimpanzees as well as other nonhuman primates), canines, felines, equines, bovines, ovines, caprines, rodents (*e.g.*, rats and mice), lagomorphs, and swine.

The amount of particles administered will depend upon the ratio of therapeutic agent (*e.g.,* nucleic acid) to lipid, the particular therapeutic agent (*e.g.,* nucleic acid) used, the disease or disorder being treated, the age, weight, and condition of the patient, and the judgment of the clinician, but will generally be between about 0.01 and about 50 mg per kilogram of body weight, preferably between about 0.1 and about 5 mg/kg of body weight, or about 10⁸-10¹⁰ particles per administration (*e.g.*, injection).

### B. In vitro Administration

For *in vitro* applications, the delivery of therapeutic agents such as nucleic acids (*e.g.,* interfering RNA) can be to any cell grown in culture, whether of plant or animal origin, vertebrate or invertebrate, and of any tissue or type. In preferred embodiments, the cells are animal cells, more preferably mammalian cells, and most preferably human cells (*e.g.,* tumor cells or hepatocytes).

Contact between the cells and the lipid particles, when carried out *in vitro,* takes place in a biologically compatible medium. The concentration of particles varies widely depending on the particular application, but is generally between about 1 µmol and about 10 mmol. Treatment of the cells with the lipid particles is generally carried out at physiological temperatures (about 37°C) for periods of time of from about 1 to 48 hours, preferably of from about 2 to 4 hours.

In one group of preferred embodiments, a lipid particle suspension is added to 60-80% confluent plated cells having a cell density of from about 10³ to about 10⁵ cells/ml, more preferably about 2 x 10⁴ cells/ml. The concentration of the suspension added to the cells is preferably of from about 0.01 to 0.2 µg/ml, more preferably about 0.1 µg/ml.

To the extent that tissue culture of cells may be required, it is well-known in the art. For example, Freshney, Culture of Animal Cells, a Manual of Basic Technique, 3rd Ed., Wiley-Liss, New York (1994), Kuchler et al., Biochemical Methods in Cell Culture and Virology, Dowden, Hutchinson and Ross, Inc. (1977), and the references cited therein provide a general guide to the culture of cells. Cultured cell systems often will be in the form of monolayers of cells, although cell suspensions are also used.

Using an Endosomal Release Parameter (ERP) assay, the delivery efficiency of the SNALP or other lipid particle of the invention can be optimized. An ERP assay is described in detail in U.S. Patent Publication No. 20030077829. More particularly, the purpose of an ERP assay is to distinguish the effect of various cationic lipids and helper lipid components of SNALP or other lipid particle based on their relative effect on binding/uptake or fusion with/destabilization of the endosomal membrane. This assay allows one to determine quantitatively how each component of the SNALP or other lipid particle affects delivery efficiency, thereby optimizing the SNALP or other lipid particle. Usually, an ERP assay measures expression of a reporter protein (*e.g.*, luciferase, β-galactosidase, green fluorescent protein (GFP), *etc.*), and in some instances, a SNALP formulation optimized for an expression plasmid will also be appropriate for encapsulating an interfering RNA. In other instances, an ERP assay can be adapted to measure downregulation of transcription or translation of a target sequence in the presence or absence of an interfering RNA (*e.g.,* siRNA). By comparing the ERPs for each of the various SNALP or other lipid particles, one can readily determine the optimized system, *e.g*., the SNALP or other lipid particle that has the greatest uptake in the cell.

### C. Cells for Delivery of Lipid Particles

The compositions and methods of the present invention are used to treat a wide variety of cell types, *in vivo* and *in vitro.* Suitable cells include, but are not limited to, hepatocytes, reticuloendothelial cells (*e.g.,* monocytes, macrophages, *etc.*), fibroblast cells, endothelial cells, platelet cells, other cell types infected and/or susceptible of being infected with viruses, hematopoietic precursor (stem) cells, keratinocytes, skeletal and smooth muscle cells, osteoblasts, neurons, quiescent lymphocytes, terminally differentiated cells, slow or noncycling primary cells, parenchymal cells, lymphoid cells, epithelial cells, bone cells, and the like.

In particular embodiments, an active agent or therapeutic agent such as a nucleic acid (*e.g.,* an interfering RNA) is delivered to cancer cells (*e.g.,* cells of a solid tumor) including, but not limited to, liver cancer cells, lung cancer cells, colon cancer cells, rectal cancer cells, anal cancer cells, bile duct cancer cells, small intestine cancer cells, stomach (gastric) cancer cells, esophageal cancer cells, gallbladder cancer cells, pancreatic cancer cells, appendix cancer cells, breast cancer cells, ovarian cancer cells, cervical cancer cells, prostate cancer cells, renal cancer cells, cancer cells of the central nervous system, glioblastoma tumor cells, skin cancer cells, lymphoma cells, choriocarcinoma tumor cells, head and neck cancer cells, osteogenic sarcoma tumor cells, and blood cancer cells.

*In vivo* delivery of lipid particles such as SNALP encapsulating a nucleic acid (*e.g.,* an interfering RNA) is suited for targeting cells of any cell type. The methods and compositions can be employed with cells of a wide variety of vertebrates, including mammals, such as, *e.g,* canines, felines, equines, bovines, ovines, caprines, rodents (*e.g.,* mice, rats, and guinea pigs), lagomorphs, swine, and primates (*e.g*. monkeys, chimpanzees, and humans).

### D. Detection of Lipid Particles

In some embodiments, the lipid particles of the present invention (*e.g*., SNALP) are detectable in the subject at about 1, 2, 3, 4, 5, 6, 7, 8 or more hours. In other embodiments, the lipid particles of the present invention (e.g., SNALP) are detectable in the subject at about 8, 12, 24, 48, 60, 72, or 96 hours, or about 6, 8, 10, 12, 14, 16, 18, 19, 22, 24, 25, or 28 days after administration of the particles. The presence of the particles can be detected in the cells, tissues, or other biological samples from the subject. The particles may be detected, *e.g.,* by direct detection of the particles, detection of a therapeutic nucleic acid such as an interfering RNA (*e.g.,* siRNA) sequence, detection of the target sequence of interest (*i.e.,* by detecting expression or reduced expression of the sequence of interest), or a combination thereof.

### 1. Detection of Particles

Lipid particles of the invention such as SNALP can be detected using any method known in the art. For example, a label can be coupled directly or indirectly to a component of the lipid particle using methods well-known in the art. A wide variety of labels can be used, with the choice of label depending on sensitivity required, ease of conjugation with the lipid particle component, stability requirements, and available instrumentation and disposal provisions. Suitable labels include, but are not limited to, spectral labels such as fluorescent dyes (*e.g*., fluorescein and derivatives, such as fluorescein isothiocyanate (FITC) and Oregon Green™; rhodamine and derivatives such Texas red, tetrarhodimine isothiocynate (TRITC), *etc.,* digoxigenin, biotin, phycoerythrin, AMCA, CyDyes™, and the like; radiolabels such as ³H, ¹²⁵I, ³⁵S, ¹⁴C, ³²P, ³³P, *etc.*; enzymes such as horse radish peroxidase, alkaline phosphatase, *etc.*; spectral colorimetric labels such as colloidal gold or colored glass or plastic beads such as polystyrene, polypropylene, latex, *etc.* The label can be detected using any means known in the art.

### 2. Detection of Nucleic Acids

Nucleic acids (*e.g*., interfering RNA) are detected and quantified herein by any of a number of means well-known to those of skill in the art. The detection of nucleic acids may proceed by well-known methods such as Southern analysis, Northern analysis, gel electrophoresis, PCR, radiolabeling, scintillation counting, and affinity chromatography. Additional analytic biochemical methods such as spectrophotometry, radiography, electrophoresis, capillary electrophoresis, high performance liquid chromatography (HPLC), thin layer chromatography (TLC), and hyperdiffusion chromatography may also be employed.

The selection of a nucleic acid hybridization format is not critical. A variety of nucleic acid hybridization formats are known to those skilled in the art. For example, common formats include sandwich assays and competition or displacement assays. Hybridization techniques are generally described in, *e.g.,* "Nucleic Acid Hybridization, A Practical Approach," Eds. Hames and Higgins, IRL Press (1985).

The sensitivity of the hybridization assays may be enhanced through the use of a nucleic acid amplification system which multiplies the target nucleic acid being detected. *In vitro* amplification techniques suitable for amplifying sequences for use as molecular probes or for generating nucleic acid fragments for subsequent subcloning are known. Examples of techniques sufficient to direct persons of skill through such *in vitro* amplification methods, including the polymerase chain reaction (PCR), the ligase chain reaction (LCR), Qβ-replicase amplification, and other RNA polymerase mediated techniques (e.g., NASBA™) are found in Sambrook et al., In Molecular Cloning: A Laboratory Manual, Cold Spring Harbor Laboratory Press (2000); and Ausubel et al., SHORT PROTOCOLS IN MOLECULAR BIOLOGY, eds., Current Protocols, Greene Publishing Associates, Inc. and John Wiley & Sons, Inc. (2002); as well as U.S. Patent No. 4,683,202; PCR Protocols, A Guide to Methods and Applications (Innis et al. eds.) Academic Press Inc. San Diego, CA (1990); Arnheim & Levinson (October 1, 1990), C&EN 36; The Journal Of NIH Research, 3:81 (1991); Kwoh et al., Proc. Natl. Acad. Sci. USA, 86:1173 (1989); Guatelli et al., Proc. Natl. Acad. Sci. USA, 87:1874 (1990); Lomell et al., J. Clin. Chem., 35:1826 (1989); Landegren et al., Science, 241:1077 (1988); Van Brunt, Biotechnology, 8:291 (1990); Wu and Wallace, Gene, 4:560 (1989); Barringer et al., Gene, 89:117 (1990); and Sooknanan and Malek, Biotechnology, 13:563 (1995). Improved methods of cloning *in vitro* amplified nucleic acids are described in U.S. Pat. No. 5,426,039. Other methods described in the art are the nucleic acid sequence based amplification (NASBA™, Cangene, Mississauga, Ontario) and Qβ-replicase systems. These systems can be used to directly identify mutants where the PCR or LCR primers are designed to be extended or ligated only when a select sequence is present. Alternatively, the select sequences can be generally amplified using, for example, nonspecific PCR primers and the amplified target region later probed for a specific sequence indicative of a mutation.

Nucleic acids for use as probes, *e.g.,* in *in vitro* amplification methods, for use as gene probes, or as inhibitor components are typically synthesized chemically according to the solid phase phosphoramidite triester method described by Beaucage et al., Tetrahedron Letts., 22:1859 1862 (1981), *e.g.,* using an automated synthesizer, as described in Needham VanDevanter et al., Nucleic Acids Res., 12:6159 (1984). Purification of polynucleotides, where necessary, is typically performed by either native acrylamide gel electrophoresis or by anion exchange HPLC as described in Pearson et al., J. Chrom., 255:137 149 (1983). The sequence of the synthetic polynucleotides can be verified using the chemical degradation method of Maxam and Gilbert (1980) in Grossman and Moldave (eds.) Academic Press, New York, Methods in Enzymology, 65:499.

An alternative means for determining the level of transcription is *in situ* hybridization. *In situ* hybridization assays are well-known and are generally described in Angerer et al., Methods Enzymol., 152:649 (1987). In an *in situ* hybridization assay, cells are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters.

### IX. Examples

The present invention will be described in greater detail by way of specific examples. The following examples are offered for illustrative purposes, and are not intended to limit the invention in any manner. Those of skill in the art will readily recognize a variety of noncritical parameters which can be changed or modified to yield essentially the same results. The non-SI unit inch used to define in particular the size of the columns in the reference examples and the examples can be converted into the SI unit cm as follows: 1 inch = 2,54 cm.

### Reference Example 1. Synthesis of MC3.

MC3 (Compound **1**) having the structure shown below was synthesized as described in Scheme 1 below.

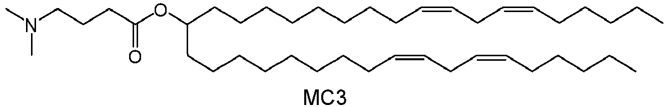

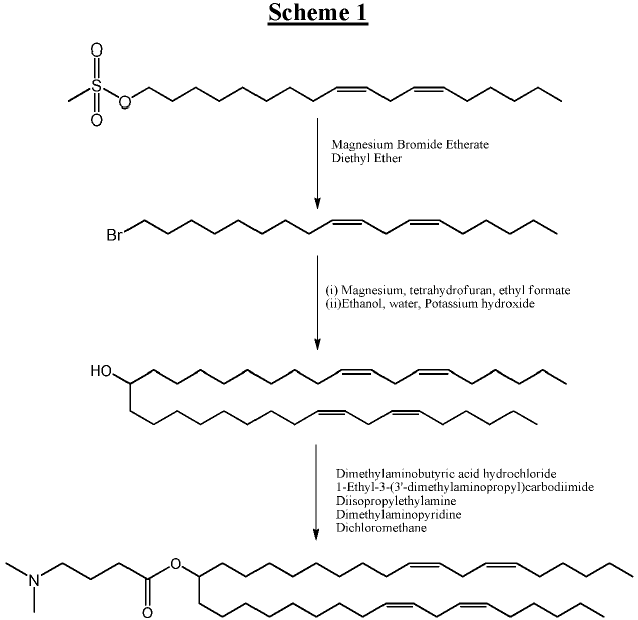

STEP 1: Magnesium bromide etherate (34 g, 110 mmol) and a stir bar were added to a 2000 mL round bottom flask. The flask was sealed and flushed with nitrogen. Anhydrous diethyl ether (400 mL) was added via canulla. A solution of linolenyl mesylate (20 g, 58 mmol) in anhydrous ether (300 mL) was then added, and the suspension stirred overnight. The suspension was poured into 500 mL of chilled water and transferred to a 2000-mL separating funnel. After shaking, the organic phase was separated. The aqueous phase was then extracted with ether (2 x 250 mL) and all ether phases combined. The ether phase was washed with water (2 x 250 mL), brine (250 mL) and dried over anhydrous Mg₂SO₄. The solution was filtered, concentrated and purified by flash chromatography. Final yield 18.9 g, 99%.

STEP 2: A 1 liter RBF was charged with magnesium turnings (11.1 g, 463 mmol), anhydrous THF (65 mL) and stir-bar and flushed with nitrogen. In a separate flask, a solution of linoleyl bromide (140 g, 425 mL) in anhydrous THF (150 mL) was prepared, and 20 mL of this solution added to the magnesium. When most of the heat had dissipated, the remainder of the bromide was added over a period of 15 minutes. Temperature was then maintained at 45°C for 4 h. The reaction was then cooled (0°C). Using a dropping funnel, a solution of ethyl formate (32.4 g, 438 mmol) in anhydrous THF (150 mL) was added over a 40 minute period. The reaction was stirred overnight at room temperature. The reaction was cooled to -15°C and 5N HCl (185 mL) added slowly. The mixture was transferred to a 2 L separating funnel separated. Water (150 mL) and hexane (150 mL) were added, the mixture washed, and again the aqueous removed. The organic was washed a final time with water (150 mL) and then concentrated to a yellow oil. The yellow oil was stirred with a mixture of EtOH (210 mL), water (30 mL) and KOH (15.8 g) for 1.5 h at room temp. The EtOH was evaporated and the residue treated with hexane (50 mL). 5N HCl (200 mL) was added via dropping funnel. The organic was washed with water (2 x 50 mL) evaporated, dried and purified by chromatography (0 - 5% EtOAc in hexane, 91 g, 81%).

STEP 3: Dilinoleylmethanol (7.8 g, 14.9 mmol), dimethylaminobutyric acid hydrochloride (2.99 g, 17.8 mmol) and a stir bar were added to 500 mL RBF. The flask was flushed with nitrogen and anh. DCM (120 mL) added, followed by EDCI (3.6 g, 18.8 mmol), DIPEA (6.3 mL, 36.3 mmol) and DMAP (450 mg, 3.69 mmol). The reaction was stirred overnight. The reaction was diluted with DCM (300 mL) and washed with sat. NaHCO₃ (200 mL), water (300 mL) and sat. NaCL (200 mL). Each aq. wash was extracted once with DCM (50 mL). Organics were combined, dried (MgSO₄) and concentrated to yield a yellow oil with some crystalline matter. This was purified by chromatography (0-2% MeOH in CHCl₃) to yield Lin-MC3 as a pale yellow oil (9.0 g, 14.1 mmol, 95%).

### Reference Example 2. Synthesis of LenMC3 and CP-LenMC3.

LenMC3 (Compound **4**) and CP-LenMC3 (Compound **5**) having the structures shown below were synthesized as described in Scheme 2 below. LenMC3 is also known as linolenyl-MC3 and DLen-MC3. CP-LenMC3 is also known as CP-linolenyl-MC3 and CP-DLen-MC3.

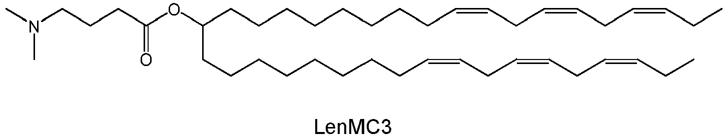

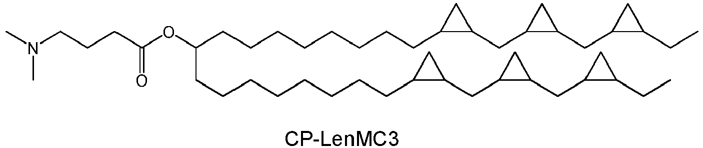

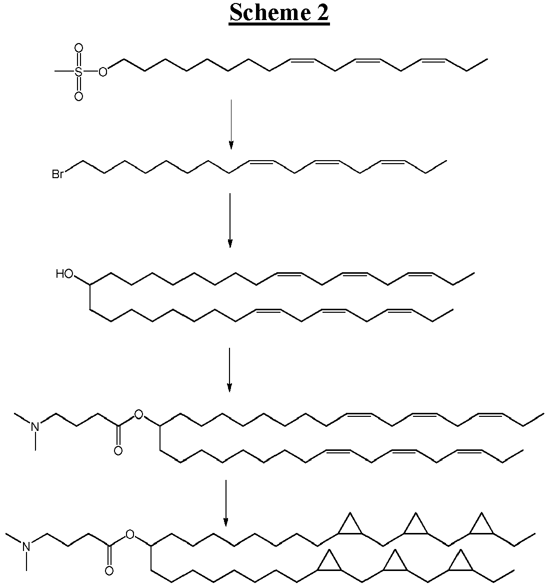

### Synthesis of linolenyl bromide (Compound 2)

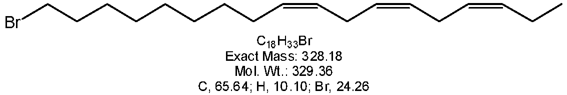

Magnesium bromide etherate (34 g, 110 mmol) and a stir bar were added to a 2000 mL round bottom flask. The flask was sealed and flushed with nitrogen. Anhydrous diethyl ether (400 mL) was added via canulla. A solution of linolenyl mesylate (20 g, 58 mmol) in anhydrous ether (300 mL) was then added, and the suspension stirred overnight. The suspension was poured into 500 mL of chilled water and transferred to a 2000-mL separating funnel. After shaking, the organic phase was separated. The aqueous phase was then extracted with ether (2 x 250 mL) and all ether phases combined. The ether phase was washed with water (2 x 250 mL), brine (250 mL) and dried over anhydrous Mg₂SO₄. The solution was filtered, concentrated and purified by flash chromatography. Final yield 19.1 g, 100%.

### Synthesis of dilinolenyl methanol (Compound 3)

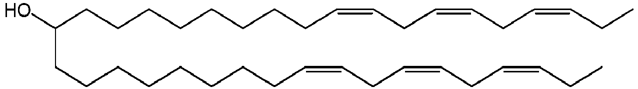

Magnesium turnings (2.1 g, 87 mmol), 5 crystals of iodine and a stirbar were added to a 1000 mL round-bottom flask. The flask was flushed with nitrogen and a solution of linolenyl bromide (Compound **2**) (19.1 g, 58 mmol) in anhydrous diethyl ether (500 mL) added via cannula. The mixture turned cloudy and was refluxed overnight. The mixture was cooled to RT and ethyl formate (4.66 mL, 58 mmol) added via syringe. The addition was made dropwise, directly into the reaction mixture, and the cloudy suspension again stirred overnight. During this time the reaction turned bright yellow. The R.M. was transferred to a 2000-mL sep. funnel with ether (50 mL), and washed with 10% H₂SO₄ (200 mL), water (2 x 200 mL) and brine (200 mL). The organic was dried over anhydrous MgSO₄, filtered and concentrated. Crude yield was 14.5 g. TLC indicated that majority of product was the methyl formate, which was purified by column chromatography. The purified formate (9.3 g, 16.7 mmol) was transferred to a 1000 mL round bottom flask and EtOH (600 mL) and a stirbar added. With stirring, water (25 mL - forming ∼5% aqueous solution) was slowly added, followed by KOH (2.0 g, 35.7 mmol). After 1 hour, the solution had turned pale yellow. TLC indicated reaction had gone to completion. The solution was concentrated by rotovap to 50% of its volume and then poured into 200 mL of 5% HCl. The aqueous phase was extracted with ether (3 x 200 mL). The ether fractions were combined and washed with water (3 x 200 mL), dried (MgSO₄) and concentrated to yield 8.9 g of dilinolenyl methanol (16.8 mmol, 58%).

### Synthesis of Len-MC3 (Compound 4)

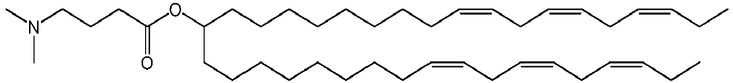

Dilinolenyl methanol (Compound 3) (2.5 g, 4.76 mmol), dimethylaminobutyric acid hydrochloride (970 mg, 5.77 mmol) and a stir bar were added to 100 mL RBF. The flask was flushed with nitrogen and anhydrous DCM (40 mL) added, followed by EDCI (FW 191.7, 1.2 g, 6.26 mmol), DIPEA (2.1 mL, 12.1 mmol) and DMAP (150 mg, 1.23 mmol). The reaction was stirred overnight, whereupon TLC indicated >80% conversion. Reaction was diluted with DCM (100 mL) and washed with sat. NaHCO₃ (100 mL), water (200 mL) and sat. NaCL (100 mL). Aqueous washes were combined and extracted with DCM (2 x 50 mL). Organics were then combined, dried (MgSO₄) and concentrated to yield a yellow oil with some crystalline matter. This was purified by chromatography to yield Len-MC3 as a pale yellow oil (2.3 g, 3.6 mmol, 76%).

### Synthesis of CP-LenMC3 (Compound 5)

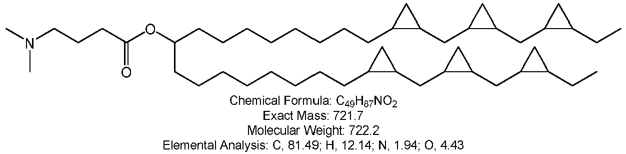

To a 250 mL RBF was added Len-MC3 (Compound **4**) (1.1 g, 1.72 mmol), a stirbar and anhydrous DCM (40 mL). The flask was flushed with N₂ and cooled to 0°C, then a 1M solution of diethylzinc in hexanes added (30 mL, 30 mmol). The solution was stirred for 1 hour at 0°C, then diiodomethane (2.4 mL 30 mmol) added and the reaction stirred overnight at RT. The reaction mixture was concentrated and then redissolved in EtOAc (50 mL). The EtOAc was washed successively with 5% HCl (2 x 50 mL), water (50 mL), NaHCO₃ (50 mL), water (50 mL), and brine (50 mL). The aqueous washes were combined and extracted with DCM (2 x 50 mL). All organics were combined, dried and concentrated to yield crude CP-Len-MC3. ¹H-NMR indicated some olefins still to be present, so the compound was treated again, using the same procedures and amounts outlined above. This time, after chromatography, ¹H-NMR indicated total conversion of the olefins. Final yield 1.0 g, 1.39 mmol, 81%.

### Reference Example 3. Synthesis of γ-LenMC3 and CP-γ-LenMC3.

γ-LenMC3 (Compound **8**) and CP-γ-LenMC3 (Compound **9**) having the structures shown below were synthesized as described in Scheme 3 below. γ-LenMC3 is also known as γlinolenyl-MC3, γDLen-MC3, and D-γ-Len-MC3. CP-γ-LenMC3 is also known as CP-γlinolenyl-MC3, CP-γDLen-MC3, and CP-D-γ-Len-MC3.

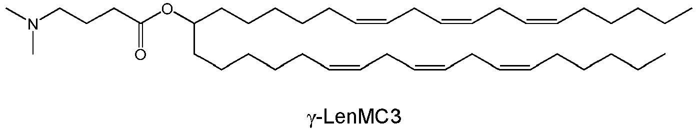

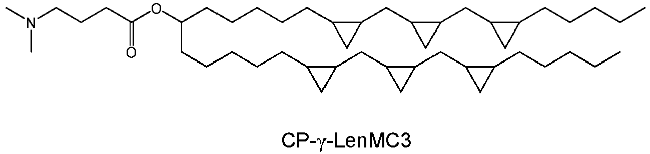

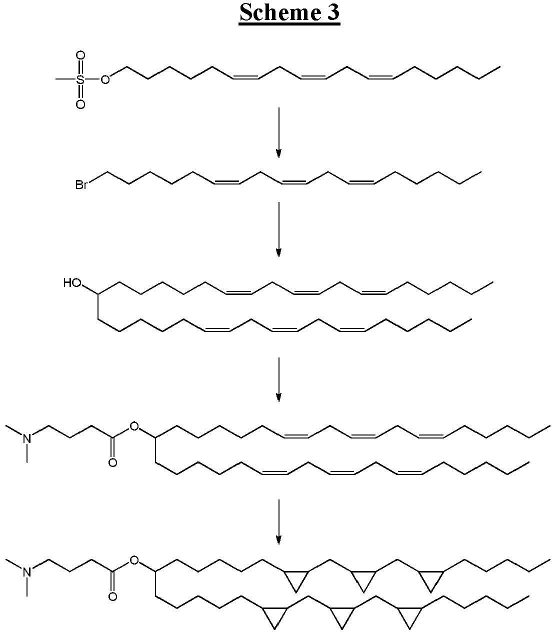

### Synthesis of γ-linolenyl bromide (Compound 6)

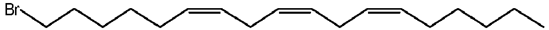

Magnesium bromide etherate (34 g, 110 mmol) and a stir bar were added to a 2000 mL round bottom flask. The flask was sealed and flushed with nitrogen. Anhydrous diethyl ether (400 mL) was added via canulla. A solution of γ-linolenyl mesylate (20 g, 58 mmol) in anhydrous ether (300 mL) was then added, and the suspension stirred overnight. The suspension was poured into 500 mL of chilled water and transferred to a 2000-mL separating funnel. After shaking, the organic phase was separated. The aqueous phase was then extracted with ether (2 x 250 mL) and all ether phases combined. The ether phase was washed with water (2 x 250 mL), brine (250 mL) and dried over anhydrous Mg₂SO₄. The solution was filtered, concentrated and purified by flash chromatography. Final yield 18.9 g, 99%.

### Synthesis of di-γ-linolenyl methanol (Compound 7)

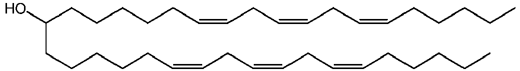

Magnesium turnings (2.1 g, 87 mmol), 5 crystals of iodine and a stirbar were added to a 1000 mL round-bottom flask. The flask was flushed with nitrogen and a solution of γ-linolenyl bromide (Compound **6**) (18.9 g, 57 mmol) in anhydrous diethyl ether (500 mL) added via cannula. The mixture turned cloudy and was refluxed overnight. The mixture was cooled to RT and ethyl formate (4.66 mL, 58 mmol) added dropwise. The suspension was stirred overnight, turning bright yellow. The R.M. was transferred to a 2000-mL sep. funnel with ether (50 mL), and washed with 10% sulphuric acid (200 mL), water (2 x 200 mL) and brine (200 mL). The organic was dried over anhydrous MgSO₄, filtered and concentrated. Crude yield was 14.5 g. TLC indicated that majority of product was the methyl formate, which was purified by column chromatography. The purified formate was transferred to a 1000 mL round bottom flask and EtOH (600 mL) and a stirbar added. With stirring, water (25 mL - forming ∼5% aqueous solution) was slowly added, followed by KOH (2.0 g, 35.7 mmol). After 1 hour, solution had turned pale yellow. TLC indicated reaction had gone to completion. The solution was concentrated by rotovap to 50% of its volume and then poured into 200 mL of 5% HCl. The aqueous phase was extracted with ether (3 x 200 mL). The ether fractions were combined and washed with water (3 x 200 mL), dried (MgSO₄) and concentrated to yield 8.8 g of di-γ-linolenyl methanol (16.8 mmol, 58%).

### Synthesis of γ-LenMC3 (Compound 8)

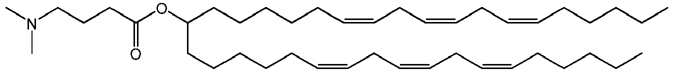

Di-γ-linolenyl methanol (Compound 7) (2.5 g, 4.76 mmol), dimethylaminobutyric acid hydrochloride (970 mg, 5.77 mmol) and a stir bar were added to 100 mL RBF. The flask was flushed with nitrogen and anhydrous DCM (40 mL) added, followed by EDCI (1.2 g, 6.26 mmol), DIPEA (2.1 mL, 12.1 mmol) and DMAP (150 mg, 1.23 mmol). The reaction was stirred overnight. The reaction was diluted with DCM (100 mL) and washed with sat. NaHCO₃ (100 mL), water (200 mL) and sat. NaCL (100 mL). Aqueous washes were combined and extracted with DCM (2 x 50 mL). Organics were then combined, dried (MgSO₄) and concentrated to yield a yellow oil. This was purified by chromatography to yield γ-Len-MC3 as a pale yellow oil (2.6 g, 4.1 mmol, 86%).

### Synthesis of CP-γ-LenMC3 (Compound 9)

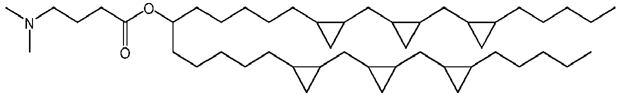

To a 250 mL RBF was added γ-LenMC3 (Compound **8**) (1.28 g, 2.0 mmol), a stirbar and anhydrous DCM (40 mL). The flask was flushed with N₂ and cooled to 0°C, then a 1M solution of diethylzinc in hexanes added (30 mL, 30 mmol, ∼ 5 equivalents per olefin). The solution was stirred for 1 hour at 0°C, then diiodomethane (2.4 mL 50 mmol) added and the reaction stirred overnight at RT. The reaction mixture was concentrated and then redissolved in EtOAc (50 mL). The EtOAc was washed successively with 5% HCl (2 x 50 mL), water (50 mL), NaHCO₃ (50 mL), water (50 mL), and brine (50 mL). The aqueous washes were combined and extracted with DCM (2 x 50 mL). All organics were combined, dried and concentrated to yield crude CP-γ-LenMC3. ¹H-NMR indicated some olefins still to be present, so the compound was treated again, using the same procedures and amounts outlined above. This time ¹H-NMR indicated total conversion of the olefins. Final yield after chromatography was 1.3 g, 1.8 mmol, 90%.

### Reference Example 4. Synthesis of MC3MC.

MC3MC (Compound **10**) having the structure shown below was synthesized as described in Schemes 4 and 5 below.

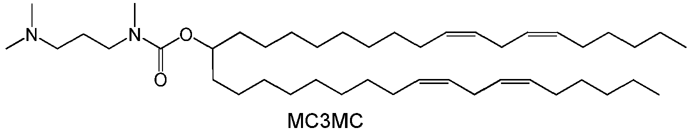

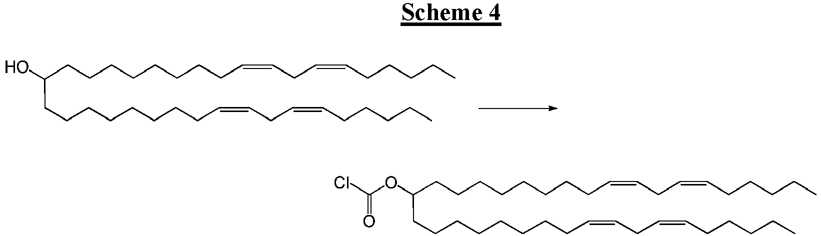

A 50 mL round bottom flask was charged with dilinoleyl methanol (3.06 g, 5.78 mmol) and a stir bar and flushed with nitrogen. Anhydrous DCM (30 mL) was added, followed by diphosgene (1.75 mL, 14.46 mmol, 2.5 eqv.). The reaction was stirred overnight and then concentrated by rotovap and purified by chromatography. This yielded the product as a colourless oil (2.6 g, 4.4 mmol, 76%).

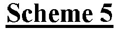

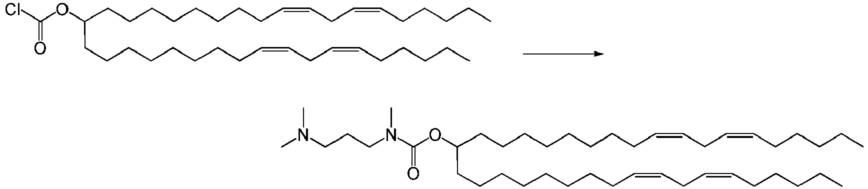

A 50 mL r.b.f. containing the chloroformate (350 mg, 0.59 mmol) and a stir bar was flushed with nitrogen and sealed. Anhydrous DCM (10 mL) and *N,N,N*'-trimethyl-1,3-propanediamine (580 mg, 5 mmol) were added and the reaction stirred for 4 h. TLC indicated the reaction to have gone to completion. The mixture was diluted to a volume of 40 mL with DCM and washed with sat. NaHCO₃ (30 mL), water (30 mL) and brine (30 mL). The aqueous phases were combined and extracted once with DCM (20 mL). Organics were then combined, dried over MgSO₄, and concentrated by rotovap. Purification yielded the product as a pale oil, 350 mg, 0.52 mmol, 89%.

### Reference Example 5. Synthesis of MC2MC.

MC2MC (Compound **11**) having the structure shown below was synthesized as described in Scheme 6 below.

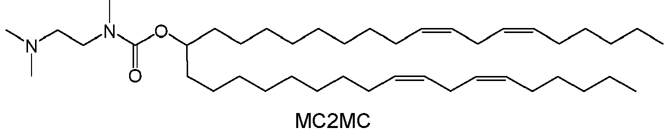

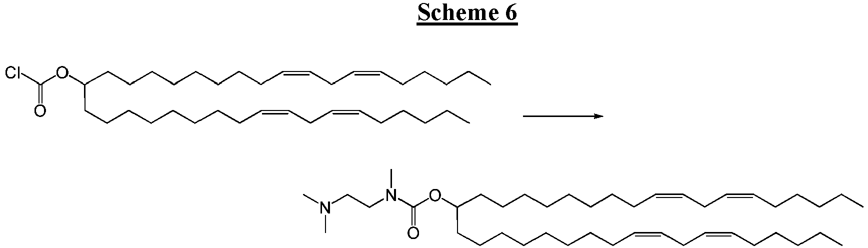

A 50 mL round bottom flask containing the chloroformate (400 mg, 0.68 mmol) and a stir bar was flushed with nitrogen and sealed. Anhydrous DCM (10 mL) and *N,N,N'-*trimethyl-1,2-ethanediamine (510 mg, 5 mmol) were added and the reaction stirred for overnight. TLC indicated the reaction to have gone to completion. The mixture was concentrated by rotovap and purified by column chromatography to yield the product as a pale oil (350 mg, 0.53 mmol, 78%).

### Reference Example 6. Synthesis of MC2C.

MC2C (Compound **12**) having the structure shown below was synthesized as described in Scheme 7 below.

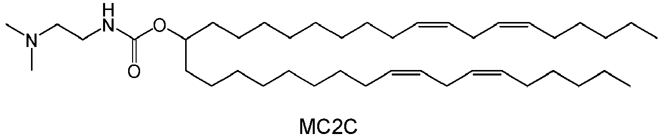

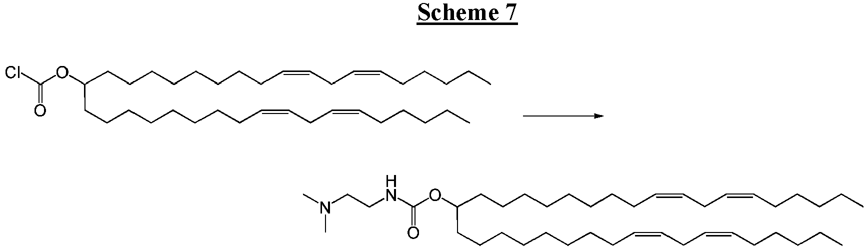

A 50 mL round bottom flask containing the chloroformate (400 mg, 0.68 mmol) and a stir bar was flushed with nitrogen and sealed. Anhydrous DCM (10 mL) and *N,N,-*dimethylethylenediamine (440 mg, 5 mmol) were added and the reaction stirred for overnight. TLC indicated the reaction to have gone to completion. The mixture was concentrated by rotovap and purified by column chromatography to yield the product as a pale yellow oil (350 mg, 0.54 mmol, 80%).

### Reference Example 7. Synthesis of MC3 Ether.

MC3 Ether (Compound **13**) having the structure shown below was synthesized as described in Scheme 8 below.

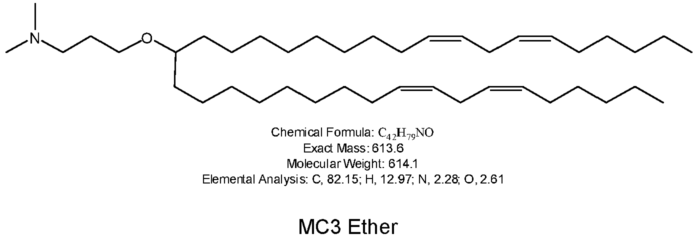

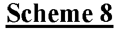

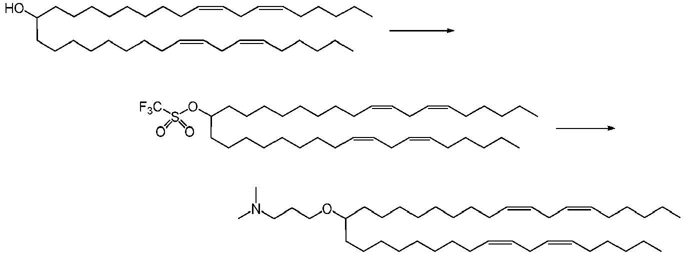

A 50 mL RBF with stir-bar was flushed with nitrogen and anhydrous DCM (4 mL). Triflic anhydride (0.7 g, 420 µL, 2.5 mmol) was added and the flask cooled to -15°C. Anhydrous pyridine (198 mg, 202 µL, 2.5 mmol) was slowly added, causing fuming and a white precipitate to form. A solution of dlinoleyl methanol (1.06 g, 2 mmol) in anhydrous DCM (2 mL) was added slowly over a period of 2 minutes. After stirring for 2 h at ∼ -15°C the reaction was off-white in color. TLC showed triflate formation and water (2 mL) was added to quench the reaction. DCM (10 mL) was added and the mixture washed with water (2 x 20 mL), dried (MgSO₄), filtered and transferred to a 25 mL round bottom flask. Proton Sponge (1.07 g, 5 mmol, min 2.5 eqv.), dimethylaminopropanol (515 mg, 5 mmol, min. 2.5 eqv) and a stir bar added and the vessel flushed with nitrogen, fitted with a condenser and refluxed for 48 h. Water (10 mL) was added, and after stirring vigorously for several minutes, separated in a 30 mL sep funnel. The organic was washed again with water (10 mL), dried over MgSO₄, concentrated and purified by chromatography (MeOH/CHCl₃) to yield the product as a pale yellow oil (400 mg, 33%).

Alternatively, MC3 Ether (Compound **13**) was synthesized starting from dilinoleyl methanol (DLinMeOH) as follows:

### Synthesis of Compound 14

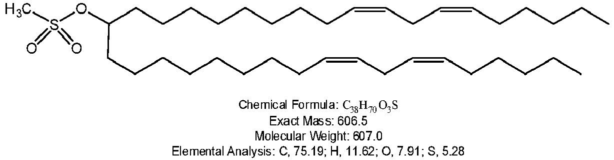

A 50 mL RBF with stir-bar was flushed with nitrogen, and DLinMeOH (1060 mg, 2 mmol), TEA (6 mmol, 834 µL) and anh. DCM (20 mL) added. Flask was cooled to 0°C and either MsCl (6 mmol) added. Reaction was stirred overnight. Reaction was diluted to 70 mL with DCM, washed with sat. NaHCO₃ (2 x 50 mL) and sat. NaCl (50 mL), dried (MgSO₄), filtered, concentrated and purified by column chromatography (1-4% EtOAc in hexane). Yield 900 mg, 75%.

### Synthesis of MC3 Ether

A 50 mL RBF with stir-bar were flushed with nitrogen, and NaH (220 mg, 9 mmol), dimethylaminopropanol (927 mg, 1.06 mL, 9 mmol) and anh. benzene (10 mL) added. After effervescence subsided, Compound **14** (440 mg, 0.75 mmol) was added and RM refluxed overnight at 90°C. TLC indicated 30-50% product formation. RM was refluxed a second night, but TLC did not appear to indicate further reaction. The reaction was diluted to 40 mL with benzene, and quenched with ethanol (25 mL). It was then washed with water (40 mL), dried and concentrated. The crude product was purified to yield product as a pale yellow oil, 157 mg, 33%.

### Reference Example 8. Synthesis of MC4 Ether.

MC4 Ether (Compound **15**) having the structure shown below was synthesized as described below.

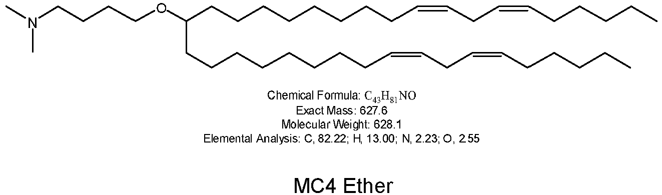

A 50 mL RBF with stir-bar were flushed with nitrogen, and NaH (220 mg, 9 mmol), dimethylaminobutanol (1.05g, 9 mmol) and anh. benzene (10 mL) added. After effervescence subsided, Compound **14** (440 mg, 0.75 mmol) was added and RM refluxed overnight at 90°C. TLC indicated some product formation. The reaction was diluted to 40 mL with benzene, and quenched with ethanol (25 mL). It was then washed with water (40 mL), dried and concentrated. The crude product was purified to yield product as a pale yellow oil, 145 mg, 31%.

### Reference Example 9. Synthesis of MC3 Amide.

MC3 Amide (Compound **16**) having the structure shown below was synthesized as described in Schemes 9-11 below.

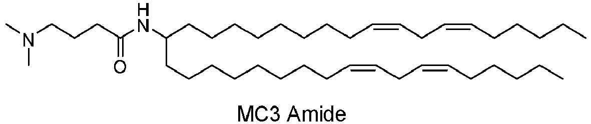

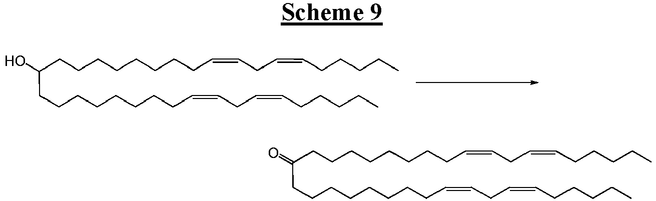

To a 500 mL RBF containing a solution of dilinoleyl methanol (10 g, 18.9 mmol) in DCM (200 mL) was added pyridinium chlorochromate (12.24 g, 56.7 mmol), anh. sodium carbonate (1.0 g, 9.5 mmol) and a stir bar. The resulting suspension was stirred under nitrogen at RT for 3 h, after which time TLC indicated all SM to have been consumed. Ether (300 mL) was then added to the mixture and the resulting brown suspension filtered through a pad of silica (300 mL), washing the pad with ether (3 x 100 mL). The ether phases were combined, concentrated and purified to yield 9.0 g (17.1 mmol, 90%) of ketone.

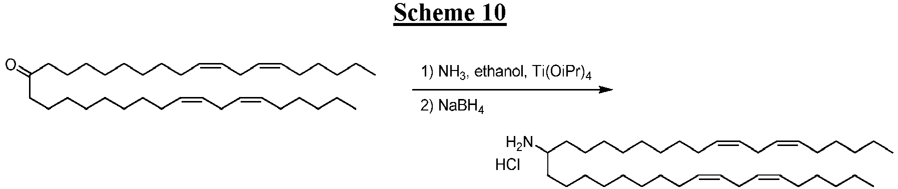

To a solution of dilinoleyl ketone (1.0 g, 1.9 mmol) in 2M ammonia in ethanol (5 mL) was added titanium(IV) isopropoxide (1.15 mL, 3.8 mmol). The solution was stirred under nitrogen at room temperature for 6 hours then sodium borohydride (110 mg, 3.8 mmol) was added. The solution effervesced for approximately 5 minutes, and then a colorless precipitate began to form. The solution was stirred for 16 hours at room temperature, quenched with 10% NH₄OH (25 mL) and diluted with ethyl acetate (50 mL). The inorganic solids were filtered and the aqueous phase was washed with ethyl acetate (2 x 75 mL). The combine ethyl acetate extracts were washed with 2M HCl (2 x 50 mL), dried on magnesium sulfate, filtered and concentrated to dryness to afford the product as a pale yellow HCl salt (1.1 g, quantitative).

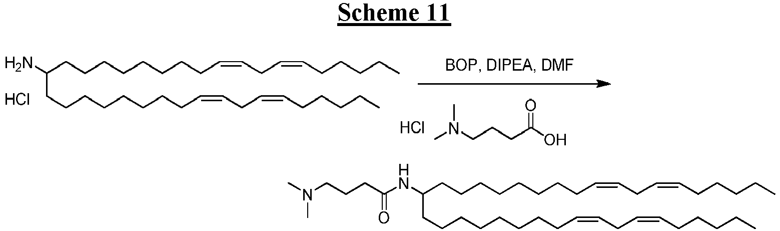

To a solution of dilinoleyl methylamine hydrochloride (1.1 g, 1.95 mmol), BOP (1.1 g, 2.4 mmol) and 4-(dimethylamino)butanoic acid hydrochloride (402 mg, 2.4 mmol) in anhydrous DMF (20 mL) was added diisopropylethylamine (1.4 mL, 7.8 mmol). The solution was stirred for 16 hours at room temperature. The solution was concentrated *in vacuo* to dryness and dissolved in ethyl acetate (100 mL). The ethyl acetate was washed with brine (3 x 50 mL), dried on magnesium sulfate, filtered and concentrated *in vacuo* to dryness. The residue was purified by column chromatography (1% to 2.5 % MeOH in CHCl₃) to afford the product as an orange oil. Decolorization through a pad of silica gel (eluted with 50% hexanes ethyl acetate to 100% ethyl acetate) afforded the product as a pale yellow oil (151 mg, 12%).

### Reference Example 10. Synthesis of Pan-MC3.

Phytanyl-MC3 ("Pan-MC3") (Compound **17**) having the structure shown below was synthesized as described in Scheme 12 below.

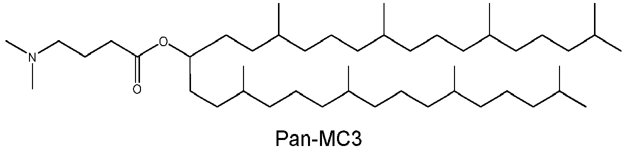

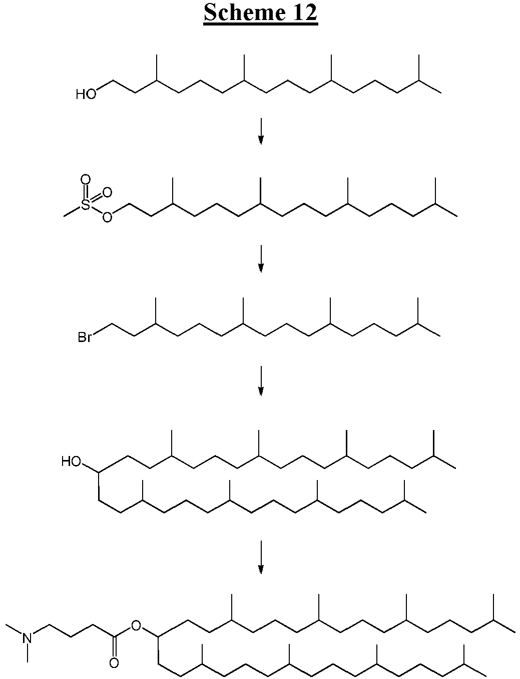

### Synthesis of Phytanyl Mesylate

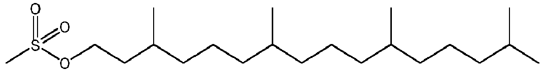

To a solution of phytanol (14.98 g, 50.2 mmol) in anhydrous dichloromethane (150 mL) under nitrogen was added triethylamine (7.7 mL, 55.2 mmol). The solution was cooled to -10°C and then a solution of methanesulfonyl chloride (11.51 g, 100.5 mmol) in anhydrous dichloromethane (100 mL) was added dropwise over 30 minutes. Upon completion, the solution was diluted to 500 mL using dichloromethane. The solution was washed twice with saturated NaHCO₃, dried over MgSO₄, filtered, and concentrated to dryness to afford the product as a colorless oil (18.9 g, 100%).

### Synthesis of Phytanyl Bromide

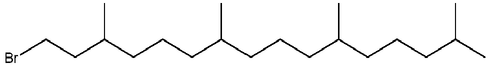

To a suspension of magnesium bromide diethyl etherate (25.9 g, 100.3 mmol) in anhydrous diethyl ether (250 mL) under nitrogen at room temperature was added a solution of phytanyl mesylate (18.9 g, 50.2 mmol) in anhydrous diethyl ether (200 mL) dropwise over 15 minutes. The resulting slurry was stirred for 72 hours at room temperature. Upon completion, the reaction mixture was cooled to 0°C and ice cold water was added dropwise until all solid dissolved and bubbling stopped. Diethyl ether (300 mL) was added, and the organic and aqueous layers separated. The aqueous layer was back-extracted with diethyl ether (200 mL). The combined diethyl ether extracts were dried on MgSO₄, filtered, and concentrated. The resulting oil was purified by column chromatography (column 10"L x 2"W; eluted with 100% hexanes) to afford the product as a pale yellow oil (16.3 g, 90%).

### Synthesis of Diphytanyl Methanol

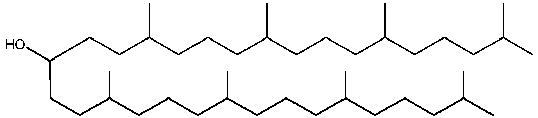

Magnesium turnings (1.18g, 48.5 mmol) were heated at 250°C in an oven for 1 hour and then stirred at room temperature under nitrogen for 2 hours. Anhydrous diethyl ether (300 mL) and a single crystal of iodine were added, followed by a solution of phytanyl bromide (15.2 g, 42.1 mmol) in anhydrous diethyl ether (30 mL). The resulting cloudy mixture was heated to reflux overnight. The solution was cooled (0°C) and a solution of ethyl formate (3.9 mL, 48.5 mmol) in anhydrous diethyl ether (15 mL) was added dropwise over 25 minutes. The resulting yellow solution was again stirred overnight. The yellow solution was cooled (0°C) and quenched using 5M HCl (15 mL), and then hexanes (100 mL) and water (150 mL) were added. The aqueous and organic layers were separated and the aqueous layer back-extracted twice with hexanes. The combined organics were washed with water, dried on MgSO₄, filtered, and concentrated *in vacuo* to dryness.

The resulting pale yellow oil was dissolved in ethanol (25 mL) and transferred to a flask containing a solution of potassium hydroxide (2.2 g, 39.2 mmol) in water (5 mL). The resulting biphasic solution was stirred at 10°C for 2.5 hours. Ethanol was removed *in vacuo* and hexanes (25 mL) and 5M HCl (35 mL) were added. The organic and aqueous layers were separated and the organic layer washed twice with water. The combined organics were dried over MgSO₄, filtered, and concentrated. The resulting pale yellow oil was purified by column chromatography (column 12"L x 2"W; eluted with a gradient of 100% hexanes → 2% → 4% ethyl ether in hexanes) to afford the product as a pale yellow oil (6.4 g, 49%).

### Synthesis of Phytanyl-MC3

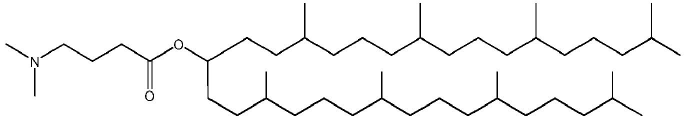

To a solution of diphytanyl methanol (6.4 g, 10.3 mmol) and 4-(dimethylamino) butyric acid hydrochloride (2.25 g, 13.4 mmol) in anhydrous dichloromethane (60 mL) under nitrogen at room temperature was added EDC (2.77 g, 18.0 mmol), diisopropylethylamine (5.4 mL, 31.0 mmol), and 4-dimethylaminopyridine (45 mg, 0.37 mmol). After 16 hours the reaction mixture was diluted with dichloromethane (75 mL). The organic layer was washed with saturated NaHCO₃, water, and brine, and then dried on MgSO₄, filtered, and concentrated. The resulting yellow oil was purified by column chromatography (column 10"L x 2"W; eluted with a gradient of 100% hexanes → 10% →- 50% ethyl acetate in hexanes) to afford the product as a pale yellow oil (3.53 g, 49%) with recovery of some phytanyl methanol (2.81 g, 44%).

### Reference Example 11. Synthesis of Pan-MC4.

Phytanyl-MC4 ("Pan-MC4") (Compound **18**) having the structure shown below was synthesized as described in Scheme 13 below.

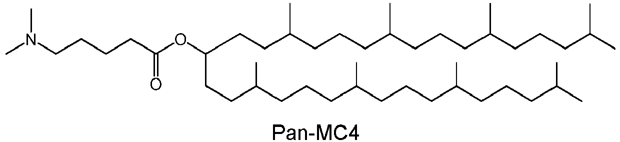

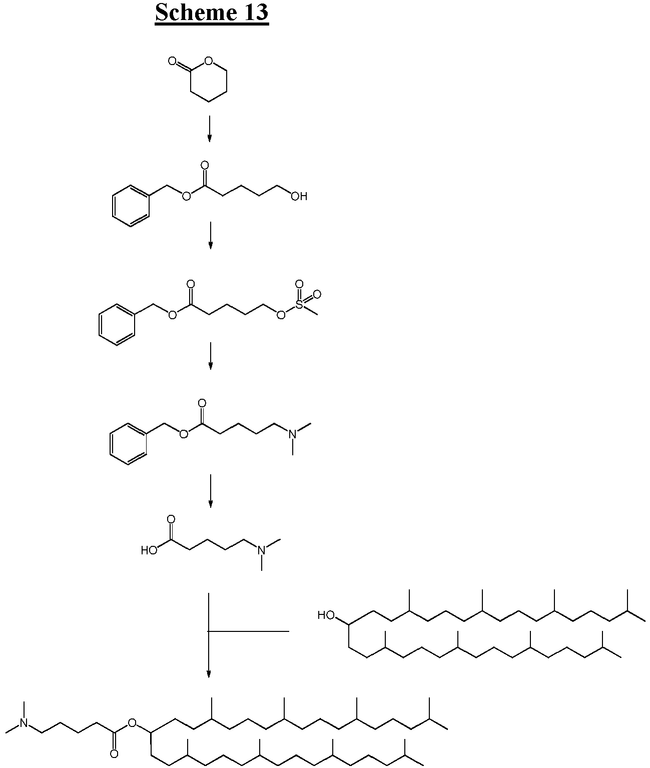

### Synthesis of Benzyl 5-hydroxypentanoate

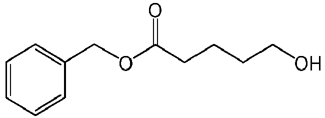

A solution of δ-valerolactone (10 g, 100 mmol) in 1M aqueous sodium hydroxide (100 mL) was heated overnight with stirring at 65°C. The solution was concentrated *in vacuo* to dryness and any residual water removed under high vacuum at -190°C. The resulting white powder was broken up and suspended in acetone (40 mL). With stirring, benzyl bromide (17 g, 101.4 mmol) and tetrabutylammonium bromide (0.82 g, 2.539 mmol) were added. The mixture was heated at 45°C with stirring for 72 hours, cooled, and concentrated. The resulting white oily powder was dissolved in ethyl acetate (300 mL) and washed twice each with saturated NaHCO₃ and brine. The organic portion was dried over anhydrous MgSO₄, filtered, and then concentrated. The result was a yellow oil, which was purified by column chromatography (column 10"L x 2"W; eluted with a gradient of 100% hexanes → 30% → 50% ethyl acetate in hexanes) to afford the product as a pale yellow oil (3.11 g, 15%).

### Synthesis of Benzyl 5-(methanesulfonyl)pentanoate

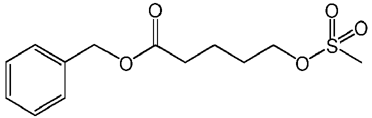

To a solution of benzyl 5-hydroxypentanoate (2.01 g, 9.65 mmol) in anhydrous dichloromethane (30 mL) under nitrogen at -15°C was added triethylamine (2.7 mL, 19.3 mmol) followed by a solution of methanesulfonyl chloride (1.5 mL, 19.3 mmol) dropwise over 20 minutes. The reaction was stirred at room temperature overnight and then diluted to 75 mL using dichloromethane. The organic layer was washed three times with saturated NaHCO₃ and the combined aqueous layers backextracted with dichloromethane. The combined organic phases were dried over MgSO₄, filtered, and concentrated. The resulting dark orange oil was purified by column chromatography (column 5"L x 1"W; eluted with a gradient of 100% hexanes → 10% → 20% → 25% diethyl ether in hexanes) to afford the product as a pale yellow oil (1.39 g, 50%).

### Synthesis of Benzyl 5-(dimethylamino)pentanoate

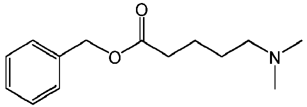

Benzyl 5-(methanesulfonyl)pentanoate (1.39 g, 4.85 mmol) was allowed to react in a 5.6M solution of dimethylamine in ethanol (100 mL) for 20 hours. The solution was then concentrated *in vacuo* to dryness. The resulting brown oil was purified by column chromatography (column 10"L x 1"W; eluted with a gradient of 100% dichloromethane → 2%/0.5% → 4%/0.5% MeOH/NH₄OH in dichloromethane) to afford the product as a yellow oil (0.79 g, 69%).

### Synthesis of 5-(dimethylamino)pentanoic acid

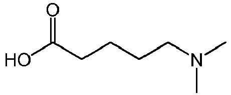

To a solution of 5-(dimethylamino)benzyl pentanoate (0.79 g, 33.6 mmol) in anhydrous ethyl acetate (20 mL) under nitrogen at room temperature was added 10% palladium on carbon (250 mg). The solution was stirred vigorously under a hydrogen atmosphere. After 16 hours additional palladium on carbon (100 mg) was added to encourage the reaction, and at 24 hours hydrogen gas was bubbled through the solution. At 40 hours the solution was filtered through celite and concentrated *in vacuo* to dryness to afford the product as a yellow oil (295mg, 60.4%).

### Synthesis of Phytanyl-MC4

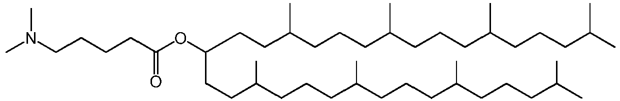

A solution of diphytanyl methanol (0.8 g, 1.3 mmol) and 4-(dimethylamino) pentanoic acid (0.24 g, 1.7 mmol) in anhydrous dichloromethane (10 mL) under nitrogen at room temperature was added EDC (0.347 g, 1.8 mmol), diisopropylethylamine (0.67 mL, 3.9 mmol), and 4-dimethylaminopyridine (45 mg, 0.37 mmol). After 20 hours additional 5-(dimethylamino)pentanoic acid (0.05 g, 0.34 mmol) was added to encourage the reaction. The reaction was stirred for an additional 52 hours and then diluted to 50 mL using dichloromethane. The organic phase was washed with saturated NaHCO₃, water, and brine, and the combined aqueous layers backextracted with dichloromethane. The combined organic layers were dried on MgSO₄, filtered, and concentrated. The resulting yellow oil was purified by column chromatography (column 10"L x 1¼" W; eluted with a gradient of 100% hexanes → 10% → 50% ethyl acetate in hexanes) to afford the product as a pale yellow oil (474 mg, 51%) with recovery of some diphytanyl methanol (348 mg, 43.5%).

### Reference Example 12. Synthesis of Pan-MC5.

Phytanyl-MC5 ("Pan-MC5") (Compound **19**) having the structure shown below was synthesized as described in Scheme 14 below.

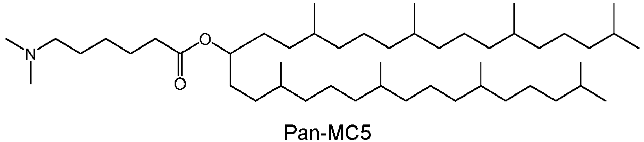

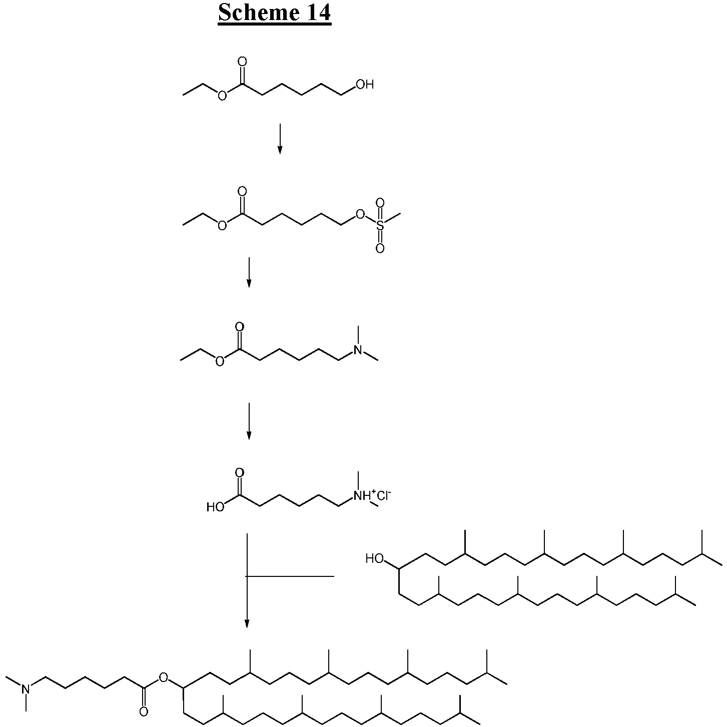

### Synthesis of Ethyl 6-(methanesulfonyl)hexanoate

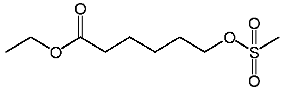

To a solution of ethyl 6-hydroxyhexanoate (5 g, 31.2 mmol) in anhydrous dichloromethane (115 mL) under nitrogen at -10°C was added triethylamine (8.7 mL, 62.5 mmol) followed by methanesulfonyl chloride (4.8 mL, 62.5 mmol) dropwise over 1 hour. The resulting solution was stirred at room temperature for 6 hours and then diluted to 300 mL using dichloromethane. The solution was washed with twice saturated NaHCO₃, and the aqueous layers backextracted with dichloromethane. The combined organics were dried over MgSO₄, filtered, and concentrated. The resulting dark orange oil was purified by column chromatography (column 5"L x 2"W; eluted with a gradient of 100% hexanes → 10% → 20% ethyl acetate in hexanes) to afford the product as a pale yellow oil.

### Synthesis of Ethyl 6-(dimethylamino)hexanoate

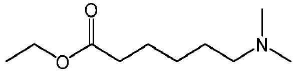

Ethyl 6-(methanesulfonyl)hexanoate was allowed to react in a 5.6M solution of dimethylamine in ethanol (100mL) for 17 hours. The solution was then concentrated *in vacuo* to dryness. The resulting bright orange paste was purified by column chromatography (column 5"L x 2"W; eluted with a gradient of 100% dichloromethane → 1%/0.25% → 2%/0.5% MeOH/NH₄OH in dichloromethane) to afford the product as a yellow oil.

### Synthesis of 6-(dimethylamino)hexanoic acid hydrochloride

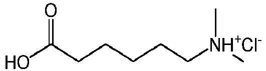

To a solution of Ethyl 6-(dimethylamino)hexanoate (5.85g, 31.2mmol) in dioxane (200mL) was added 1M NaOH (200mL). The solution was stirred vigorously at room temperature for 2 hours and then dioxane was removed *in vacuo.* The resulting aqueous solution was made slightly acidic using concentrated HCl (15mL). At this point, dichloromethane and ether were used in an attempt to extract the product from solution. However, all attempts failed. Instead, water was removed under high vacuum to afford the product as an off-white solid, a mixture of approximately 35% 6-(dimethylamino)hexanoic acid hydrochloride in NaCl by weight.

### Synthesis of Phytanyl-MC5

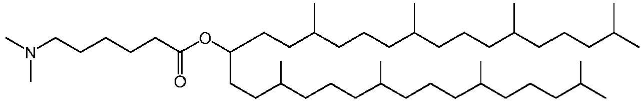

To a solution of diphytanyl methanol (1.5 g, 2.4 mmol) and 35% 6-(dimethylamino) hexanoic acid hydrochloride (1.79 g, 3.2 mmol) in anhydrous dichloromethane (15 mL) under nitrogen at room temperature was added EDC (0.65 g, 3.4 mmol), diisopropylethylamine (1.26 mL, 7.2 mmol) and 4-dimethylaminopyridine (10 mg). After 48 hours additional 35% 6-(dimethylamino)hexanoic acid (1 g, 1.8 mmol), EDC (0.32 g, 1.7 mmol) and 4-dimethylaminopyridine (15 mg) were added. After an additional 72 hours the reaction mixture was diluted to 75 mL using dichloromethane and then washed with water, saturated NaHCO₃, and brine. The combined aqueous layers were backextracted twice with dichloromethane and the combined organic layers dried over MgSO₄, filtered, and concentrated. The resulting yellow oil was purified by column chromatography (column 1¼"W x 10"L; eluted with a gradient of 100% hexanes → 10% → 50% ethyl acetate in hexanes) to afford the product as a yellow oil (175 mg, 10%) with some recovery of diphytanyl methanol.

### Reference Example 13. Synthesis of MC3 Thioester.

MC3 Thioester (Compound **20**) having the structure shown below was synthesized as described in Scheme 15 below.

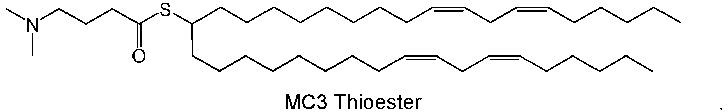

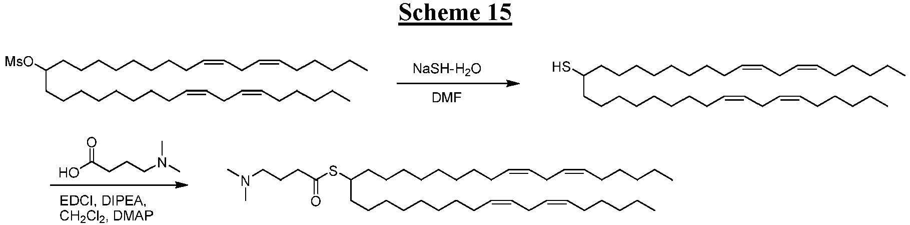

### Synthesis of (6Z,9Z,28Z,31Z)-heptatriaconta-6,9,28,31-tetraene-1 9-thiol

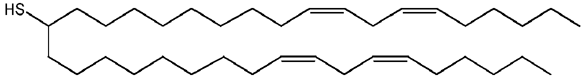

A solution of (6Z,9Z,28Z,31Z)-heptatriaconta-6,9,28,31-tetraen-19-yl methanesulfonate (2.0g, 3.3mmol) in anhydrous DMF (15mL) was treated with NaSH·H₂O (925mg, 16.5mmol) and heated (70°C, 2h). The mixture was cooled (rt), diluted with H₂O and extracted with Et₂O (3x). The organic extract was washed with brine, then dried (Na₂SO₄), filtered and concentrated. The crude material was subjected to chromatography (hexanes → 2% EtOAc-hexanes) to yield (6Z,9Z,28Z,31Z)-heptatriaconta-6,9,28,31-tetraene-19-thiol (1.31g, 73%) as a pale yellow oil. Rf 0.9 (10% EtOAc-hexanes), FW 544.50, C₃₇H₆₈S.

### Synthesis of S-(6Z,9Z,28Z,31Z)-heptatriaconta-6,9,28,31-tetraen-19-yl 4 (dimethylamino) butanethioate (MC3 Thioester)

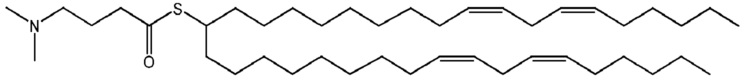

A solution of (6Z,9Z,28Z,31Z)-heptatriaconta-6,9,28,31-tetraene-19-thiol (500mg, 0.92mmol) and N,N-dimethylamino-butyric acid hydrochloride (200mg, 1.2mmol) in anhydrous CH₂Cl₂ (3mL) was treated with EDC (229mg, 1.2mmol), Hünig's base (481µL, 2.8mmol) and DMAP (18mg). After stirring (2h) the solution was diluted with CH₂Cl₂, washed with NaHCO₃ (sat. aq.) and brine then dried (Na₂SO₄), filtered and concentrated. The crude material was subjected to chromatography (CH₂Cl₂ → 3% CH₃OH-CH₂Cl₂) to yield S-(6Z,9Z,28Z,31Z)-heptatriaconta-6,9,28,31-tetraen-19-yl 4 (dimethylamino) butanethioate (197mg, 33%) as a colorless oil. Rf 0.4 (8% CH₃OH-CHCl₃), ¹H NMR (400MHz, CDCl₃, δ_{H}) 5.41-5.28 (m, 8 H, HC=CH x 8), 3.53-3.45 (m, 1 H, CHSCO), 2.77 (app. t, 4 H, C=CHCH₂HC=C x 2), 2.56 (t, 2 H, CH₂COS), 2.27 (t, 2 H, CH₂N(CH₃)₂), 2.21 (s, 6 H, N(CH₃)₂), 2.10-1.97 (m, 8 H, CH₂HC=C x 4), 1.85-1.76 (m, 2 H, CH₂), 1.63-1.43 (m, 4 H, CH₂ x 2), 1.42-1.20 (m, 36 H, CH₂ x 18), 0.89 (t, 6 H, CH₃ x 2). FW 659.14, C₄₃H₇₉NOS.

### Reference Example 14. Synthesis of Compounds 21-24.

(6Z,9Z,27Z,30Z)-19-oxohexatriaconta-6,9,27,30-tetraen-18-yl 3-(dimethylamino)propanoate (Compound **21**), (6Z,9Z,27Z,30Z)-19-hydroxyhexatriaconta-6,9,27,30-tetraen-18-yl 3-(dimethylamino)propanoate (Compound **22**), (6Z,9Z,27Z,30Z)-19-oxohexatriaconta-6,9,27,30-tetraen-18-yl 4-(dimethylamino)butanoate (Compound **23**), and (6Z,9Z,27Z,30Z)-19-hydroxyhexatriaconta-6,9,27,30-tetraen-18-yl 4-(dimethylamino)butanoate (Compound **24**) having the structures shown below were synthesized as described in Scheme 16 below.

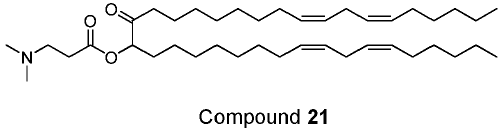

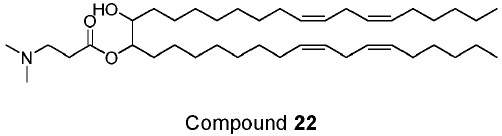

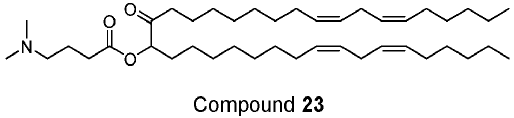

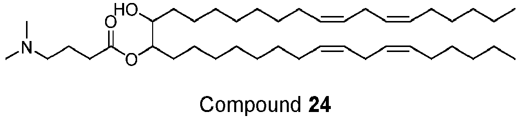

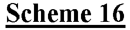

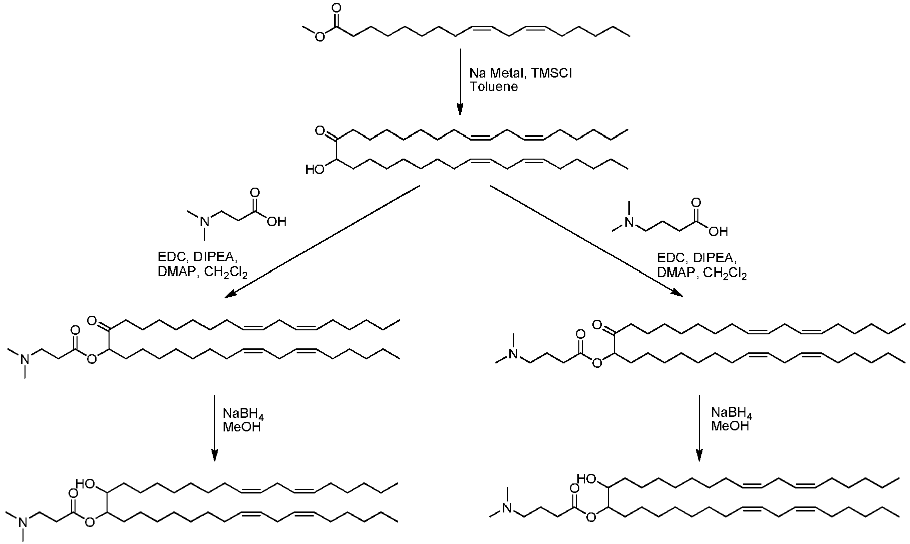

### Synthesis of (6Z,9Z,27Z,30Z)-19-hydroxyhexatriaconta-6,9,27,30-tetraen-18-one

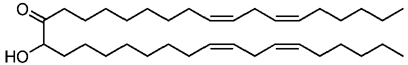

To a 500 mL round bottom flask, purged with nitrogen, was added anhydrous toluene (70 mL) followed by finely sliced pieces of sodium metal (4.25 g, 185 mmol). chlorotrimethylsilane (17 mL, 136.1 mmol) was added slowly and the reaction was heated to 40°C. To this solution was added methyl linoleate (10 g, 32.4 mmol) drop wise over 45 minutes. The solution was brought to reflux for 2 hours where upon the sodium changed from a large mass to small 1-2 mm beads (reaction turned purple after ∼1.5 hours). The reaction was cooled to room temperature and slowly quenched with methanol (25 mL) at 0°C over 30 minutes. Once the unreacted sodium metal had dissolved, the reaction mixture was filtered through celite with ether rinses (400 mL). The filtrate (∼400-500 mL) was stirred vigorously with saturated ammonium chloride (300 mL) for 16 hours. Upon completion, the ether/toluene layer was separated and washed with brine (1 x 100 mL). The organic layer was dried on magnesium sulfate, filtered and concentrated *in vacuo* to dryness. The yellow oil was purified by column chromatography (gradient: 100% Hexanes to 5% EtOAc in hexanes) to afford the title compound as a pale yellow oil (4.8 g, 56%).

### Synthesis of (6Z,9Z,27Z,30Z)-19-oxohexatriaconta-6,9,27,30-tetraen-18-yl 3-(dimethylamino)propanoate (Compound 21)

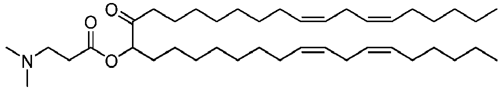

To a solution of (6*Z*,9*Z*,27*Z*,30*Z*)-19-hydroxyhexatriaconta-6,9,27,30-tetraen-18-one (1.0 g, 1.9 mmol), 3-(dimethylamino)propanoic acid hydrogen chloride (876 mg, 5.7 mmol) and EDCI (1.2 g, 6.3 mmol), in dichloromethane (20 mL) was added DIPEA (1.0 mL, 5.7 mmol) and DMAP (20 mg). The solution was stirred at room temperature for 16 hours under nitrogen. Upon completion, the reaction was diluted with dichloromethane (50 mL) and washed with sodium bicarbonate solution (50 mL). The dichloromethane layer was dried on magnesium sulfate, filtered and concentrated *in vacuo* to dryness. The residue was purified by column chromatography (100% ethyl acetate) to afford the title compound as a colorless oil (675 mg, 57%). ¹H NMR (400 MHz, CDCl₃): *δ*5.43-5.28 (m, 8H), 5.03-4.98 (m, 1H), 2.80-2.75 (m, 4H), 2.74-2.56 (m, 4H), 2.54-2.36 (m, 2H), 2.28 (s, 6H), 2.09-2.01 (m, 8H), 1.82-1.64 (m, 2H), 1.63-1.52 (m, 2H), 1.42-1.23 (m, 30H), 0.93-0.86 (m, 6H).

### Synthesis of (6Z,9Z,27Z,30Z)-19-hydroxyhexatriaconta-6,9,27,30-tetraen-18-yl 3-(dimethylamino)propanoate (Compound 22)

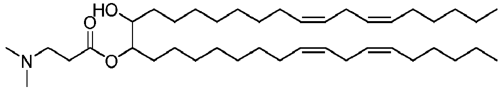

To a solution of (6Z,9Z,27Z,30Z)-19-oxohexatriaconta-6,9,27,30-tetraen-18-yl 3-(dimethylamino)propanoate (450 mg, 0.72 mmol) in anhydrous methanol (20 mL) was slowly added sodium borohydride (222 mg, 5.9 mmol). The solution was stirred for 16 hours at room temperature then quenched with water (20 mL). The solution was washed with dichloromethane (3 x 50 mL) and the combined extracts were dried on magnesium sulfate, filtered and concentrated *in vacuo* to dryness. The residue was purified by column chromatography (100% ethyl acetate) to afford the title compound as a colorless oil (416 mg, 91%). ¹H NMR (400 MHz, CDCl₃): *δ* 5.45-5.28 (m, 8H), 5.02-4.82 (m, 1H), 3.69-3.43 (m, 1H), 2.84-2.45 (m, 8H), 2.30 (s, 6H), 2.09-2.02 (m, 8H), 1.67-1.20 (m, 36H), 0.93-0.86 (m, 6H).

### Synthesis of (6Z,9Z,27Z,30Z)-19-oxohexatriaconta-6,9,27,30-tetraen-18-yl 4-(dimethylamino)butanoate (Compound 23)

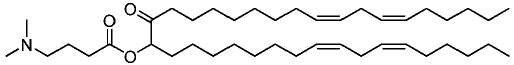

Using an analogous procedure to that described for the synthesis of (6*Z*,9*Z*,27*Z*,30*Z*)-19-oxohexatriaconta-6,9,27,30-tetraen-18-yl 3-(dimethylamino)propanoate, (6*Z*,9*Z*,27*Z*,30*Z*)-19-oxohexatriaconta-6,9,27,30-tetraen-18-yl 4-(dimethylamino)butanoate was obtained as a colorless oil (980 mg, 80%). ¹H NMR (400 MHz, CDCl₃): *δ* 5.43-5.29 (m, 8H), 5.02-4.97 (m, 1H), 2.81-2.75 (m, 4H), 2.54-2.33 (m, 6H), 2.39 (s, 6H), 2.09-2.01 (m, 8H), 1.92-1.82 (m, 2H), 1.80-1.64 (m, 2H), 1.62-1.53 (m, 2H), 1.42-1.24 (m, 30H), 0.93-0.86 (m, 6H).

### Synthesis of (6Z,9Z,27Z,30Z)-19-hydroxyhexatriaconta-6,9,27,30-tetraen-18-yl 4-(dimethylamino)butanoate (Compound 24)

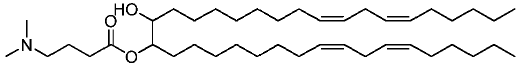

Using an analogous procedure to that described for the synthesis of (6*Z*,9*Z*,27*Z*,30*Z*)-19-hydroxyhexatriaconta-6,9,27,30-tetraen-18-yl 3-(dimethylamino)propanoate, 6*Z*,9*Z*,27*Z*,30*Z*)-19-hydroxyhexatriaconta-6,9,27,30-tetraen-18-yl 4-(dimethylamino)butanoate was obtained as a colorless oil (218 mg, 31%). ¹H NMR (400 MHz, CDCl₃): *δ* 5.44-5.29 (m, 8H), 4.92-4.81 (m, 1H), 3.71-3.54 (m, 1H), 2.81-2.75 (m, 4H), 2.46-2.34 (m, 1H), 2.28 (s, 3H), 2.27 (s, 3H), 2.09-2.02 (m, 8H), 1.94-1.78 (m, 2H), 1.69-1.20 (m, 36H), 0.93-0.86 (m, 6H).

### Reference Example 15. Synthesis of Compound 25.

(6Z,9Z,28E,31E)-heptatriaconta-6,9,28,31-tetraen-19-yl 4-(dimethylamino)butanoate (Compound **25**) having the structure shown below was synthesized as described in Scheme 17 below.

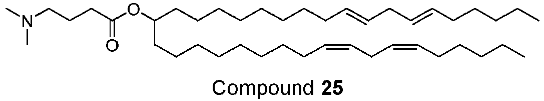

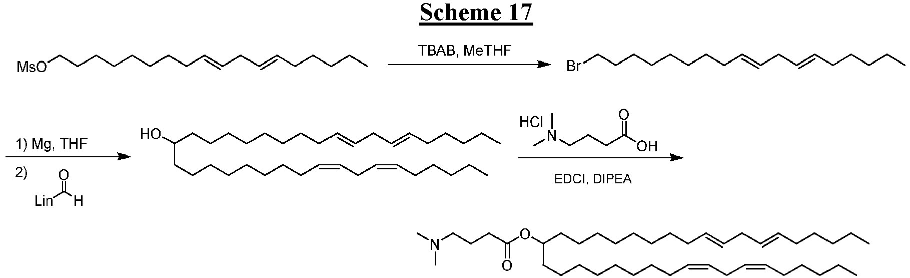

### Synthesis of (6E,9E)-18-bromooctadeca-6,9-diene

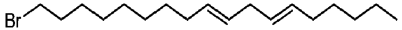

Using an analogous procedure to that described for the synthesis of linoleyl bromide, (6*E*,9*E*)-18-bromooctadeca-6,9-diene was obtained as a colorless oil (9.1 g, 87%).

### Synthesis of (10Z,13Z)-nonadeca-10,13-dienal

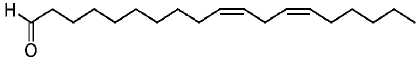

A 100 mL round bottom flask charged with magnesium turnings (462 mg, 19.0 mmol) and a stir bar was dried with a high temperature heat gun for 5 minutes. The flask was cooled to room temperature under nitrogen then charged with THF (25 mL). Linoleyl bromide (1.9 g, 5.76 mmol) was added drop wise and the solution was heated to 45 °C for 3 hours under nitrogen. Upon completion, DMF (1.3 mL, 16.7 mmol) was added slowly and the solution was stirred for 1 hour at room temperature. The solution was diluted with ether (75 mL) and washed with 5% HCl (3 x 50 mL). The ether solution was dried on magnesium sulfate, filtered and concentrated *in vacuo* to dryness. The residue was purified by column chromatography (column: 2" x 10"; eluted with 2% ether/hexanes) to afford the title compound as a colorless oil (3.5 g, 83%).

### Synthesis of (6Z,9Z,28E,31E)-heptatriaconta-6,9,28,31-tetraen-19-ol

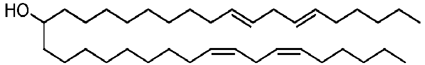

A 100 mL round bottom flask, charged with magnesium turnings (355 mg, 14.6 mmol) and a stir bar, was dried with a high temperature heat gun for 5 minutes. Upon cooling under nitrogen, THF (6 mL) was added followed by linolaidyl bromide (6.02 g, 18.3 mmol). The mixture was heated to 45 °C and a single grain of iodine was added. The reaction was refluxed for 2.5 hours then cooled to room temperature where upon a solution of (10*Z*,13*Z*)-nonadeca-10,13-dienal (3.4 g, 12.4 mmol) in THF (10 mL) was added. The solution was stirred for 1.5 hours at room temperature then poured into water (150 mL) and 5% HCl (50 mL) was added. Upon dissolution of the magnesium, the solution was extracted with ether (2 x 150 mL). The combined ether extracts were dried on magnesium sulfate, filtered and concentrated *in vacuo* to dryness. The residue was purified by column chromatography (100% hexanes) to afford the title compound as a colorless oil (4.57 g, 71%).

### Synthesis of (6Z,9Z,28E,31E)-heptatriaconta-6,9,28,31-tetraen-19-yl 4-(dimethylamino)butanoate (Compound 25)

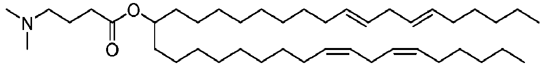

Using an analogous procedure to that described for the synthesis of (6*Z*,9*Z*,28*Z*,31*Z*)-heptatriaconta-6,9,28,31-tetraen-19-yl 4-(dimethylamino)butanoate, (6*Z*,9*Z*,28*E*,31*E*)-heptatriaconta-6,9,28,31-tetraen-19-yl 4-(dimethylamino)butanoate was obtained as a pale yellow oil (1.12 g, 93%). ¹H NMR (400 MHz, CDCl₃): *δ* 5.47-5.29 (m, 8H), 4.91-4.83 (m, 1H), 2.81-2.75 (m, 2H), 2.70-2.65 (m, 2H), 2.58-2.30 (m, 10H), 2.10-1.85 (m, 10H), 1.57-1.45 (m, 4H), 1.41-1.20 (m, 36H), 0.93-0.86 (m, 6H).

### Reference Example 16. Synthesis of Compound 26.

(6*R*,8*Z*,27*Z*,30*Z*)-6-hydroxyhexatriaconta-8,27,30-trien-18-yl 4-(dimethylamino)butanoate (Compound **26**) having the structure shown below was synthesized as described in Scheme 18 below.

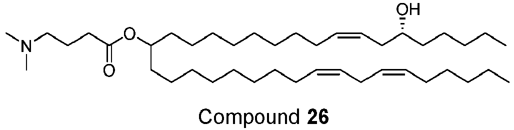

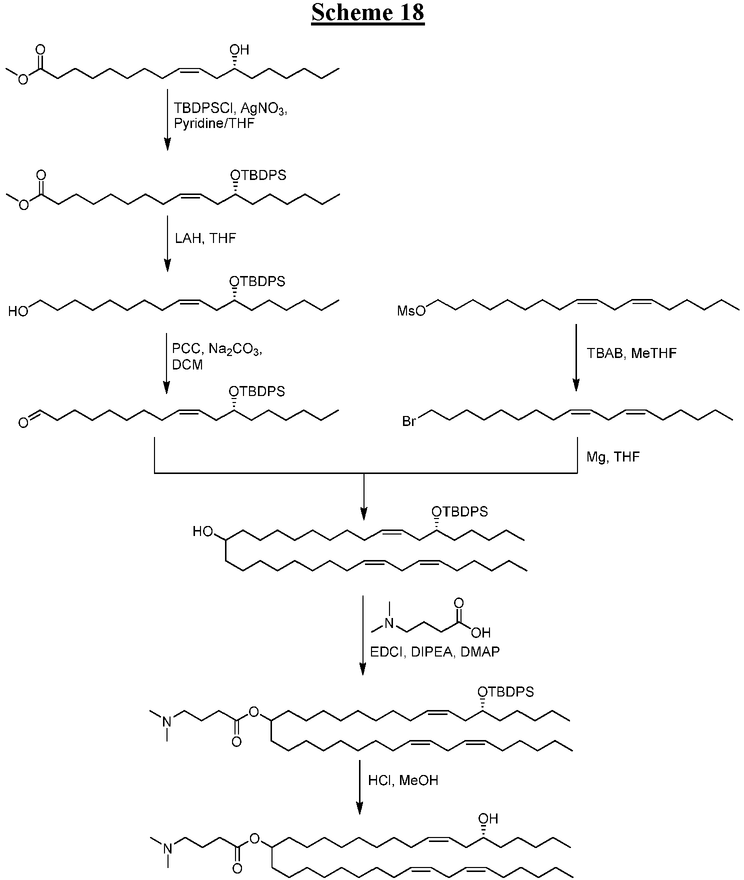

### Synthesis of (R,Z)-Methyl 12-(tert-butyldiphenylsilyloxy)octadec-9-enoate

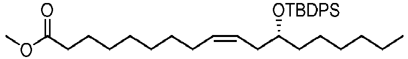

To a solution of methyl ricinoleate (8.0 g, 32 mmol) in anhydrous pyridine (90 mL) and anhydrous THF (45 mL) was added t-butylchlorodimethyl silane (8.5 mL, 33.3 mmol) and silver nitrate (5.65 g, 33.3 mmol). The solution was stirred at room temperature for 90 minutes under nitrogen. The solution was filtered through celite and the filter cake was rinsed with THF (400 mL). The filtrate was concentrated *in vacuo* and the residue was dissolved in dichloromethane (300 mL) and washed with 5% HCl (150 mL). The aqueous layer was separated and washed with dichloromethane (2 x 200 mL). The combined dichloromethane extracts were washed with brine (350 mL), dried on magnesium sulphate, filtered and concentrated *in vacuo* to dryness. The residue was purified by column chromatography (gradient: 100 % hexanes to 10% ethyl acetate in hexanes) to afford the title compound as a colourless oil (13.1 g, 93%).

### Synthesis of (R,Z)-12-(tert-butyldiphenylsilyloxy)octadec-9-en-1-ol

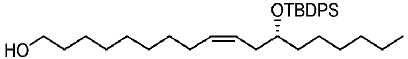

To a suspension of lithium aluminium hydride (1.8 g, 47.5 mmol) in anhydrous THF (30 mL) was added drop wise a solution of (*R*,*Z*)-methyl 12-(tert-butyldiphenylsilyloxy)octadec-9-enoate (13.1 g, 23.7 mmol) in THF (30 mL) over 30 minutes. The reaction was stirred for 1 hour at room temperature under nitrogen. Upon completion, 5M NaOH (4 mL) was added drop wise at 0°C. The mixture was dried on magnesium sulfate, filtered and concentrated *in vacuo* to dryness to afford the title compound as a light yellow oil. The product was used in the next step without further purification (11.1 g, 89%).

### Synthesis of (R,Z)-12-(tert-butyldiphenylsilyloxy)octadec-9-enal

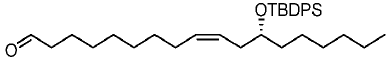

To a solution of (*R*,*Z*)-12-(tert-butyldiphenylsilyloxy)octadec-9-en-1-ol (11.1 g, 21.2 mmol) in anhydrous dichloromethane (160 mL) was added pyridinium chlorochromate (215.6 g, 110.3 mmol) and sodium carbonate (1.1 g, 18.4 mmol). The mixture was stirred at room temperature for 3 hours then filtered through a pad of silica (eluted with 100% ethyl acetate). The filtrate was concentrated *in vacuo* to dryness and the residue was purified by column chromatography (gradient: 100% hexanes to 10% ethyl acetate in hexanes) to afford the title compound as a yellow oil (8.9 g, 81%).

### Synthesis of (6R,8Z,27Z,30Z)-6-(tert-butyldiphenylsilyloxy)hexatriaconta-8,27,30-trien-18-ol

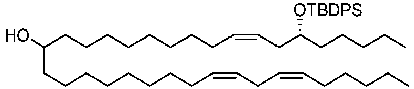

A 100 mL round bottom flask charged with magnesium turnings (0.16 g, 6.53 mmol) and a stir bar was dried with a high temperature heat gun for 5 minutes. The flask was cooled to room temperature under nitrogen then charged with THF (1.1 mL). A solution of linoleyl bromide (1.9 g, 5.76 mmol) in THF (1.9 mL) was added drop wise and the solution was heated to 55 °C for 2 hours under nitrogen. Upon completion, the reaction mixture was cooled to room temperature and a solution of (*R*,*Z*)-12-(tert-butyldiphenylsilyloxy)octadec-9-enal (2.0 g, 3.84 mmol) in THF (20 mL) was added slowly over 10 minutes. The solution was stirred for 1 hour at room temperature and then quenched with 5M HCl (20 mL) and water (100 mL). The remaining solution was extracted with diethyl ether (3 x 150 mL) and the combined diethyl ether extracts were dried on magnesium sulfate, filtered and concentrated *in vacuo* to dryness. The residue was purified by column chromatography (gradient: 100% Hexanes to 5% ethyl acetate hexanes) to afford the title compound as a yellow oil (1.8 g, 60%).

### Synthesis of (6R,8Z,27Z,30Z)-6-(tert-butyldiphenylsilyloxy)hexatriaconta-8,27,30-trien-18-yl 4-(dimethylamino)butanoate

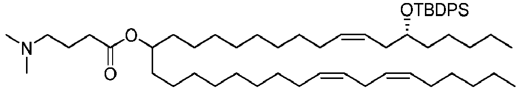

Using an analogous procedure to that described for the synthesis of (6*Z*,9*Z*,28*Z*,31*Z*)-heptatriaconta-6,9,28,31-tetraen-19-yl 4-(dimethylamino)butanoate, (6*R*,8*Z*,27*Z*,30*Z*)-6-(tert-butyldiphenylsilyloxy)hexatriaconta-8,27,30-trien-18-yl 4-(dimethylamino)butanoate was obtained as a colorless oil (800 mg, 78%).

### Synthesis of (6R,8Z,27Z,30Z)-6-hydroxyhexatriaconta-8,27,30-trien-18-yl 4-(dimethylamino)butanoate (Compound 26)

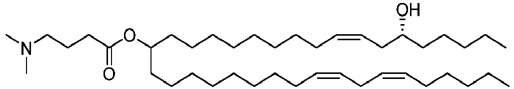

A solution of (6*R*,8*Z*,27*Z*,30*Z*)-6-(tert-butyldiphenylsilyloxy)hexatriaconta-8,27,30-trien-18-yl 4-(dimethylamino)butanoate (800 mg, 0.95 mmol) in the saturated HCl_{(g)}/MeOH (50 mL) was stirred for 10 minutes. The starting material did not dissolve so anhydrous DCM (12 mL) was added, and the mixture was stirred for 30 minutes. Upon completion, the solution was concentrated *in vacuo,* saturated sodium bicarbonate (75 mL) was added, and the solution was extracted with ethyl acetate (3 x 100 mL). The combined ethyl acetate extracts were washed with brine (100 mL), dried on magnesium sulfate, filtered and concentrated *in vacuo* to dryness. The residue was purified by column chromatography (100% ethyl acetate) to afford the title compound as a colorless oil (0.228 g, 39%). ¹H NMR (400 MHz, CDCl₃): *δ* 5.61-5.52 (m, 1H), 5.45-5.29 (m, 4H), 4.91-4.83 (m, 1H), 3.66-3.58 (m, 1H), 2.81-2.75 (m, 2H), 2.75-2.44 (m, 7H), 2.42-2.36 (m, 2H), 2.24-2.18 (m, 2H), 2.10-1.93 (m, 7H), 1.56-1.41 (m, 6H), 1.40-1.20 (m, 36H), 0.93-0.85 (m, 6H).

### Reference Example 17. Synthesis of Compounds 27 and 28.

(6*Z*,9*Z*,28*Z*,31*Z*)-heptatriaconta-6,9,28,31-tetraen-18-yl 3-(dimethylamino)propyl(hexyl)carbamate (Compound **27**) and (6Z,9Z,28Z,31Z)-heptatriaconta-6,9,28,31-tetraen-18-yl 3-(dimethylamino)propyl((Z)-hept-4-enyl)carbamate (Compound **28**) having the structures shown below were synthesized as described in Scheme 19 below.

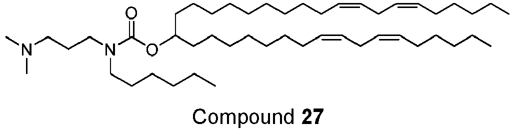

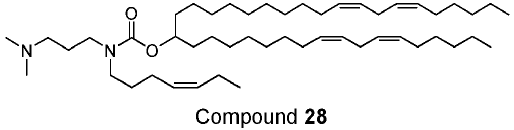

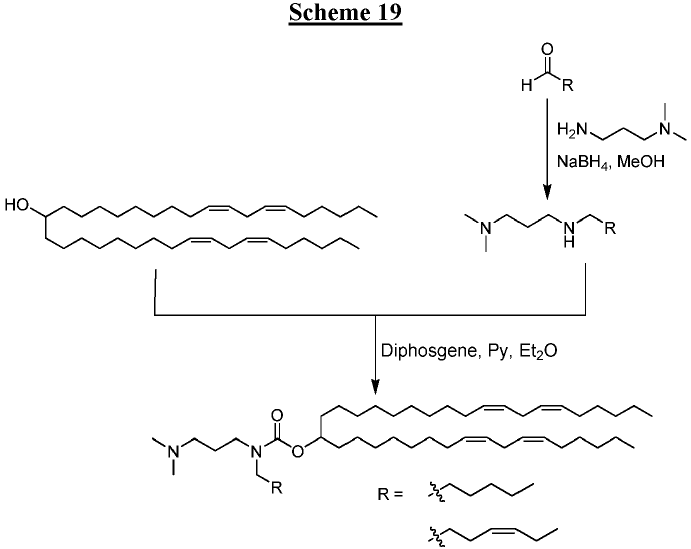

### Synthesis of N-hexyl-N',N'-dimethylpropane-1,3-diamine

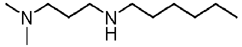

A solution of n-capronaldehyde (3.79 g, 37.8 mmol) and N,N-dimethylpropane-1,3-diamine (5 mL, 39.7 mmol) in anhydrous methanol (140 mL) was stirred at room temperature for 3 hours. To this solution was added slowly sodium borohydride (2.15 g, 56.8 mmol) over 5 minutes. The solution was stirred for 30 minutes then quenched with 1M NaOH (75 mL), diluted with water (125 mL) and extracted with ethyl acetate (3 x 150 mL). The combined ethyl acetate extracts were dried on magnesium sulfate, filtered and concentrated *in vacuo* to dryness. The residue was purified by column chromatography (gradient: 100% DCM to 10% MeOH in DCM with 1% NH₄OH) to afford the title compound as a pale yellow oil (3.79 g, 54%).

### Synthesis of (6Z,9Z,28Z,31Z)-heptatriaconta-6,9,28,31-tetraen-18-yl 3-(dimethylamino)propyl(hexyl)carbamate (Compound 27)

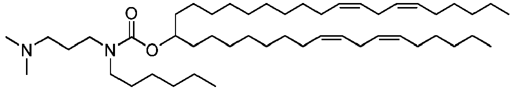

To a solution of dilinoleyl methanol (1.0 g, 1.9 mmol) and pyridine (230 µL, 4.7 mmol) in anhydrous ether (10 mL) cooled to -15°C under nitrogen was slowly added diphosgene (380 µL, 3.1 mmol). The reaction was stirred for 20 minutes at -15 °C, then N-hexyl-*N'*,*N'*-dimethylpropane-1,3-diamine (3.8 g, 20.3 mmol) was added as a solution in 4:1 ether / dichloromethane (10 mL). The solution was warmed to room temperature and stirring was continued for 20 minutes. Upon completion, the solution was diluted with diethyl ether (100mL) and washed with saturated sodium bicarbonate (2 x 50mL). The ether extract was dried on magnesium sulphate, filtered, concentrated *in vacuo* to dryness. The residue was purified by column chromatography (100% ethyl acetate) to afford the title compound as a colorless oil (1.1 g, 76%). ¹H NMR (400 MHz, CDCl₃): δ 5.43-5.26 (m, 8H), 4.80-4.68 (m, 1H), 3.35-3.15 (m, 4H), 2.81-2.75 (m, 4H), 2.66-2.25 (m, 8H), 2.10-1.70 (m, 14H), 1.64-1.46 (m, 6H), 1.40-1.20 (m, 38H), 1.00-0.93 (m, 3H), 0.92-0.86 (m, 6H).

### Synthesis of (Z)-N-(hept-4-enyl)-N',N'-dimethylpropane-1,3-diamine

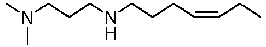

Using an analogous procedure to that described for the synthesis of *N-*hexyl-*N*',*N*'-dimethylpropane-1,3-diamine, (*Z*)-*N*-(hept-4-enyl)-*N'*,*N'*-dimethylpropane-1,3-diamine was obtained as a pale yellow oil (5.11 g, 68%).

### Synthesis of (6Z,9Z,28Z,31Z)-heptatriaconta-6,9,28,31-tetraen-18-yl 3-(dimethylamino)propyl((Z)-hept-4-enyl)carbamate (Compound 28)

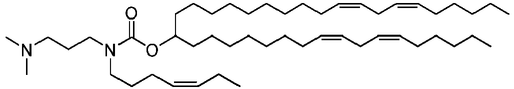

Using an analogous procedure to that described for the synthesis of (6*Z*,9*Z*,28*Z*,31*Z*)-heptatriaconta-6,9,28,31-tetraen-18-yl 3-(dimethylamino)propyl(hexyl)carbamate, (6*Z*,9*Z*,28*Z*,31*Z*)-heptatriaconta-6,9,28,31-tetraen-18-yl 3-(dimethylamino)propyl((Z)-hept-4-enyl)carbamate was obtained as a colorless oil (1.31 g, 92%). ¹H NMR (400 MHz, CDCL₃): *δ*5.44-5.27 (m, 10H), 4.80-4.67 (m, 1H), 3.35-3.15 (m, 4H), 2.81-2.74 (m, 4H), 2.69-2.26 (m, 8H), 2.10-1.98 (m, 12H), 1.98-1.72 (m, 2H), 1.64-1.46 (m, 6H), 1.41-1.22 (m, 36H), 0.96 (t, J = 7.5 Hz, 3H), 0.92-0.86 (m, 6H).

### Reference Example 18. Synthesis of Compound 29.

(6*Z*,9*Z*,28*Z*,31*Z*)-heptatriaconta-6,9,28,31-tetraen-18-yl 2-(dimethylamino)ethyl(ethyl)carbamate (Compound **29**) having the structure shown below was synthesized as described in Scheme 20 below.

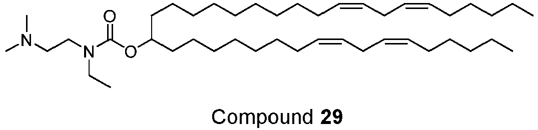

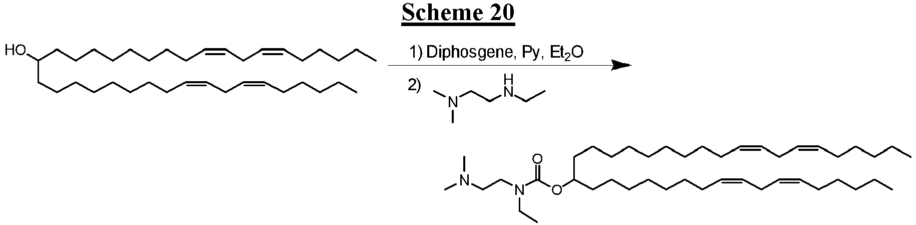

### Synthesis of (6Z,9Z,28Z,31Z)-heptatraconta-6,9,28,31-tetraen-18-yl 2-(dimethylamino)ethyl(ethyl)carbamate (Compound 29)

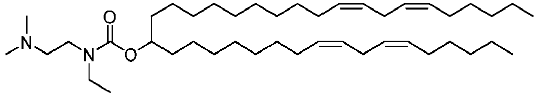

To a solution of dilinoleyl methanol (1.0 g, 1.9 mmol) and pyridine (230 µL, 4.7 mmol) in anhydrous ether (10 mL) cooled to -15°C under nitrogen was slowly added diphosgene (0.38 mL, 3.14 mmol). The reaction was stirred for 1 hour at -15°C, then *N,N-*dimethyl-*N*'-ethyl-ethylenediamine (2.2 mL, 14.2 mmol) was added. The solution was warmed to room temperature and stirred for 30 minutes. The solution was diluted with diethyl ether (50 mL) and filtered to remove the urea and ammonium salts. The ether filtrate was concentrated *in vacuo* to dryness and the residue was purified by column chromatography (100% ethyl acetate) to afford the title compound as a colorless oil (990 mg, 78%). ¹H NMR (400 MHz, CDCl₃): *δ*5.41-5.27 (m, 8H), 4.77-4.69 (m, 1H), 3.55-3.20 (m, 4H), 2.79-2.72 (m, 4H), 2.72-2.56 (m, 1H), 2.55-2.21 (m, 7H), 2.08-1.96 (m, 8H), 1.58-1.44 (m, 4H), 1.39-1.15 (m, 36H), 1.10 (t, *J* = 7.0 Hz, 3H), 0.90-0.82 (m, 6H).

### Reference Example 19. Synthesis of Compounds 30 and 31.

(6*Z*,9*Z*,28*Z*,31*Z*)-heptatriaconta-6,9,28,3 1-tetraen-19-yl (3-(ethyl(methyl)amino)propyl)(methyl)carbamate (Compound 30) and (6Z,9Z,28Z,31Z)-heptatriaconta-6,9,28,31-tetraen-19-yl (3-(diethylamino)propyl)(methyl)carbamate (Compound 31) having the structures shown below were synthesized as described in Scheme 21 below.

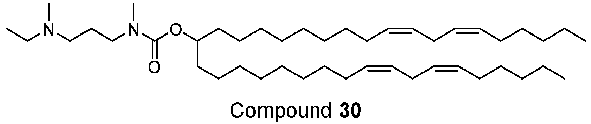

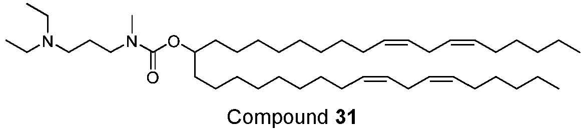

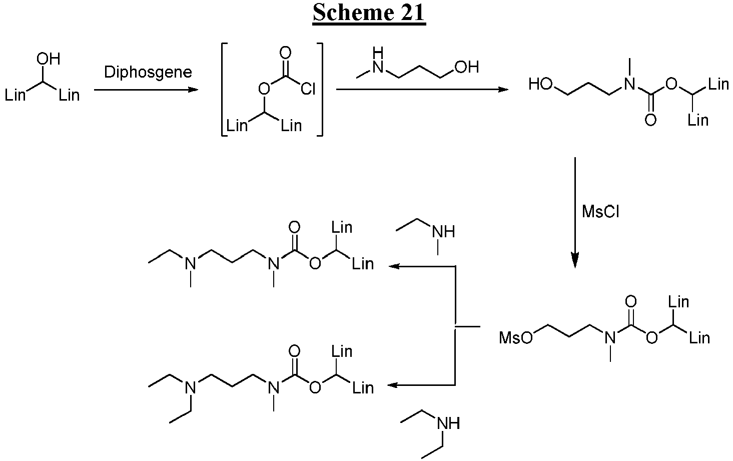

### Synthesis of (6Z,9Z,28Z,31Z)-heptatriaconta-6,9,28,31-tetraen-19-yl (3-hydroxypropyl)(methyl(carbamate

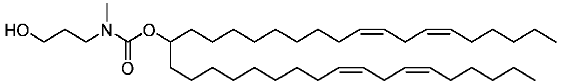

A solution of trichloromethyl chloroformate (340 µL, 2.8 mmol) in anhydrous Et₂O (5 mL) was cooled (-15°C) and treated with a solution of dilinoleyl methanol (1.0 g, 1.9 mmol) and pyridine (230 µL, 2.8 mmol) in Et₂O (5mL). After stirring (1 h) the reaction mixture was filtered through a frit and the filtrate was added, dropwise, to a cool (0°C) solution of 3-methylamino-propan-1-ol (1.1 mL, 11.3 mmol) in Et₂O (5 mL). After stirring (5 min) the reaction mixture was filtered and concentrated. The crude material was subjected to chromatography (12% → 20% EtOAc-hexanes) to yield (6Z,9Z,28Z,31Z)-heptatriaconta-6,9,28,3 1-tetraen-19-yl (3-hydroxypropyl)(methyl)carbamate (960 mg, 79%) as a colorless oil. Rf 0.17 (10% EtOAc-hexanes), FW 644.07, C₄₂H₇₇NO₃.

### Synthesis of 3-((((6Z,9Z,28Z,31Z)-heptatriaconta-6,92831-tetraen-19-yloxy)carbonyl)(methyl)amino)propyl methanesulfonate

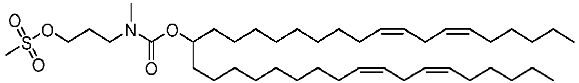

A solution of the alcohol (6*Z*,9*Z*,28*Z*,31*Z*)-heptatriaconta-6,9,28,31-tetraen-19-yl (3-hydroxypropyl)(methyl)carbamate (960 mg, 1.5 mmol) and triethylamine (0.7 mL) in anhydrous CH₂Cl₂ (7 mL) was cooled (-15°C) and treated with a solution of methanesulphonyl chloride (230 µL) in CH₂Cl₂ (5 mL) over 5min. After stirring (45 min) the solution was diluted with CH₂Cl₂ and washed with NaHCO₃ (Sat. aq.) and brine, dried (Na₂SO₄), filtered and concentrated. The crude material was subjected to chromatography (20% EtOAc-hexanes) to yield 3-((((6Z,9Z,28Z,31Z)-heptatriaconta-6,9,28,31-tetraen-19-yloxy)carbonyl)(methyl)amino)propyl methanesulfonate (1.0 g, 95%) as a colorless oil. Rf 0.5 (CH₂Cl₂), FW 722.16, C₄₃H₇₉NO₅S.

### Synthesis of (6Z,9Z,28Z,31Z)-heptatriaconta-6,92831-tetraen-19-yl (3-(ethyl(methyl)amino)propyl)(methyl)carbamate (Compound 30)

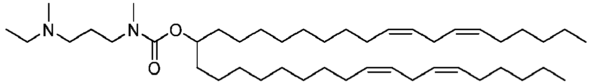

A solution of ethylmethylamine (2mL) in EtOH (10mL) was treated with 3-((((6*Z*,9*Z*,28*Z*,31*Z*)-heptatriaconta-6,9,28,31-tetraen-19-yloxy)carbonyl)(methyl)amino)propyl methanesulfonate (500 mg, 0.7 mmol) in CH₂Cl₂ (2.5 mL). The solution was stirred (50 h), concentrated and subjected to chromatography (EtOAc) to yield (6*Z*,9*Z*,28*Z*,31*Z*)-heptatriaconta-6,9,28,31-tetraen-19-yl (3-(ethyl(methyl)amino)propyl)(methyl)carbamate (305 mg, 64%) as a pale yellow oil. Rf 0.41 (10% CH₃OH-CH₂Cl₂), ¹H NMR (400MHz, CDCl₃, δ_{H}) 5.47-5.34 (m, 8 H, C=CH x 8), 4.81-4.75 (m, 1 H, CHO₂CNR₂), 3.33 (br. s, 2 H, CH₂NCH₃), 2.94 (br. s, 3 H, CO₂NCH₃), 2.83 (app. t, 4 H), 2.45 (q, 2 H, N(CH₃)CH₂CH₃), 2.37 (t, 2 H, CH₂N(CH₃)CH₂CH₃), 2.25 (s, 3 H, N(CH₃)CH₂CH₃), 2.14-2.07 (m, 8 H, CH₂HC=C x 4), 1.81-1.70 (m, 4 H, CH₂ x 2), 1.62-1.50 (m, 4 H, CH₂ x 2), 1.46-1.25 (m, 34 H, CH₂ x 17), 1.09 (t, 3 H, CH₃NCH₂CH₃), 0.93 (t, 6 H, CH₃ x 2), FW 685.16, C₄₅H₈₄N₂O₂.

### Synthesis of (6Z,9Z,28Z,31Z)-heptatiiaconta-6,9,28,31-tetraen-19-yl (3-(diethylamino)propyl)(methyl)carbamate (Compound 31)

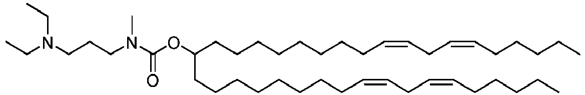

A solution of diethylamine (2 mL) in EtOH (10 mL) was treated with 3-((((6*Z*,9*Z*,28*Z*,31*Z*)-heptatriaconta-6,9,28,31-tetraen-19-yloxy)carbonyl)(methyl)amino)propyl methanesulfonate (500 mg, 0.7 mmol) in CH₂Cl₂ (2.5 mL). The solution was stirred (50 h), concentrated and subjected to chromatography (EtOAc) to yield (6*Z*,9*Z*,28*Z*,31*Z*)-heptatriaconta-6,9,28,31-tetraen-19-yl (3-(diethylamino)propyl)(methyl)carbamate (207 mg, 42%) as a pale yellow oil. Rf 0.43 (10% CH₃OH-CH₂Cl₂), ¹H NMR (400MHz, CDCl₃, δ_{H}) 5.41-5.28 (m, 8 H, C=CH x 8), 4.79-4.70 (m, 1 H, CHO₂CNR₂), 3.28 (br. s, 2 H, CH₂NCH₃), 2.94-2.83 (br. s, 3 H, CO₂NCH₃), 2.79 (app. t, 4 H, bis-allylic CH₂ x 2), 2.52 (q, 4 H, N(CH₂CH₃)₂), 2.09-2.01 (m, 8 H, CH₂HC=C x 4), 1.75-1.65 (m 4 H, CH₂ x 2), 1.59-1.45 (m, 4 H, CH₂ x 2), 1.42-1.22 (m, 36 H, CH₂ x 18), 1.15 (t, 6 H, N(CH₂CH₃)₂), 0.90 (t, 6 H, CH₃ x 2). FW 699.19, C₄₆H₈₆N₂O₂.

### Reference Example 20. Synthesis of Compound 32.

(6*Z*,9*Z*,28*Z*,31*Z*)-heptatriaconta-6,9,28,31-tetraen-19-yl 2-(1H-imidazol-1-yl)acetate (Compound **32**) having the structure shown below was synthesized as described in Scheme 22 below.

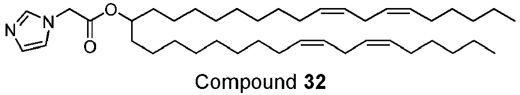

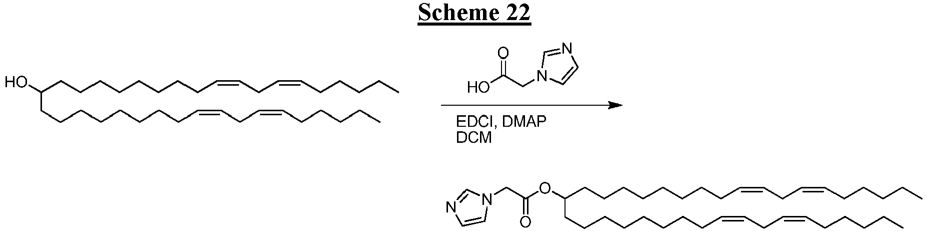

A solution of dilinoleyl methanol (1.5 g, 2.8 mmol), 2-(1H-imidazol-1-yl)acetic acid (1.07 g, 8.5 mmol), EDCI hydrochloride (1.8 g, 9.4 mmol) in anhydrous dichloromethane (25 mL) was added DMAP (10 mg). The solution was refluxed for 16 hours under nitrogen. Upon completion, the solution was diluted with dichloromethane (100 mL) and washed with saturated sodium bicarbonate (100 mL). The sodium bicarbonate solution was back extracted with dichloromethane (2 x 100 mL). The combined dichloromethane extracts were dried on magnesium sulfate, filtered and concentrated *in vacuo* to dryness. The residue was purified by column chromatography on silica gel 60 (column: 2" x 6" L; eluted with 1:1 hexanes/ethyl acetate) to afford the title compound as a colorless oil (700 mg, 39%). ¹H NMR (400 MHz, CDCl₃): *δ*7.91 (s, 1H), 7.16 (s, 1H), 7.00 (s, 1H), 5.44-5.28 (m, 8H), 4.99-4.90 (m, 1H), 4.77 (s, 2H), 2.81-2.75 (m, 4H), 2.09-2.02 (m, 8H), 1.58-1.49 (m, 4H), 1.41-1.20 (m, 36H), -.93-0.86 (m, 6H).

### Reference Example 21. Synthesis of Compound 33.

1-(2-((6*Z*,9*Z*,28*Z*,31*Z*)-heptatriaconta-6,9,28,31-tetraen-19-yloxy)ethyl)-1*H-*imidazole (Compound **33**) having the structure shown below was synthesized as described in Scheme 23 below.

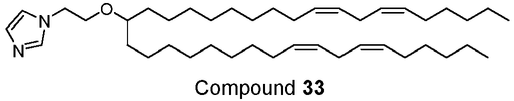

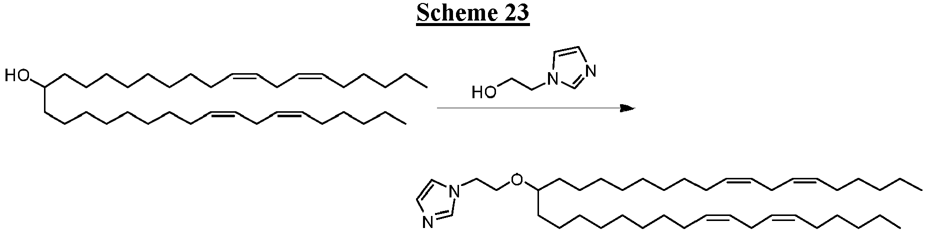

Using an analogous procedure to that described for the synthesis of 3-((6*Z*,9*Z*,28*Z*,31*Z*)-heptatriaconta-6,9,28,31-tetraen-19-yloxy)-N,N-dimethylpropan-1-amine (MC3 Ether), 1-(2-((6*Z*,9*Z*,28*Z*,31*Z*)-heptatriaconta-6,9,28,31-tetraen-19-yloxy)ethyl)-1*H-*imidazole was obtained as a pale yellow oil (600 mg, 24%). ¹H NMR (400 MHz, CDCl₃): *δ* 8.12 (s, 1H), 7.15 (s, 1H), 7.09 (s, 1H), 5.43-5.30 (m, 8H), 4.20 (t, *J* = 4.9 Hz, 2H), 3.70 (t, *J* = 5Hz, 2H), 3.26-3.18 (m, 1H), 2.82-2.75 (m, 4H), 2.10-2.04 (m, 8H), 1.46-1.12 (m, 40H), 0.93-0.86 (m, 6H).

### Example 1. Synthesis of Compounds 3-35.

3-((3*Z*,6*Z*,9*Z*,28*Z*,31*Z*,34*Z*)-heptatriaconta-3,6,9,28,31,34-hexaen-19-yloxy)-*N,N* dimethylpropan-1-amine (Compound **34**), and 4-((3Z,6Z,9Z,28Z,31Z,34Z)-heptatriaconta-3,6,9,28,31,34-hexaen-19-yloxy)-N,N-dimethylbutan-1-amine (Compound **35**) of the invention, as well as 2-((6Z,9Z,28Z,31Z)-heptatriaconta-6,9,28,31-tetraen-19-yloxy)-N,N-dimethylethanamine (Compound **36**), and 3-((6E,9E,28E,31E)-heptatriaconta-6,9,28,31-tetraen-19-yloxy)-N,N-dimethylpropan-1-amine (Compound **37**) having the structures shown below were synthesized as described in Scheme 24 below.

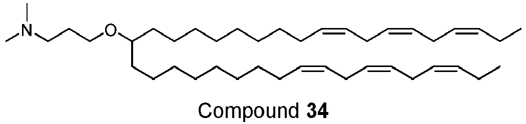

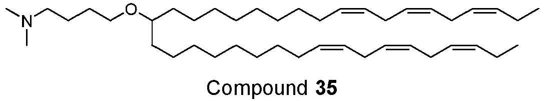

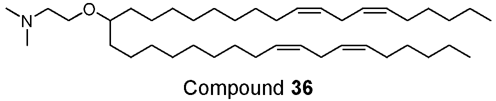

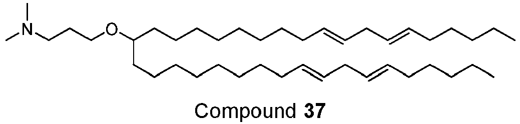

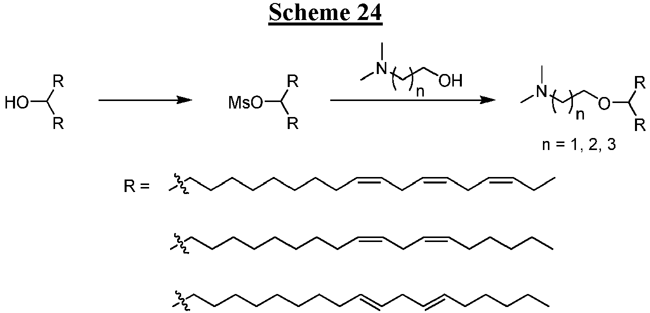

### Synthesis of 3-((3Z,6Z,9Z,28Z,31Z,34Z)-hei)tatriaconta-3,6,9,28,31,34-hexaen-19-yloxy)-N,N-dimethylpropan-1-amine (Compound 34)

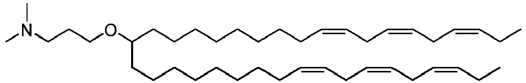

In the same fashion as 3-((6*Z*,9*Z*,28*Z*,31*Z*)-heptatriaconta-6,9,28,31-tetraen-19-yloxy)-*N,N*-dimethylpropan-1-amine (MC3 Ether), 3-((3Z,6Z,9Z,28Z,31Z,34Z)-heptatriaconta-3,6,9,28,31,34-hexaen-19-yloxy)-*N,N-*dimethylpropan-1-amine (2.26 g, 54%) was prepared as a pale yellow oil. ¹H NMR (400MHz, CDCl₃, δ_{H}) 5.43-5.27 (m, 12 H), 3.46 (t, 2 H), 3.18 (app. p, 1 H), 3.07-2.91 (m, 3 H), 2.84-2.77 (m, 8 H), 2.61-2.45 (m, 2 H), 2.44-2.30 (br. s, 6 H), 2.11-2.04 (m, 8 H), 1.89-1.78 (br. s, 2 H), 1.45-1.20 (m, 28 H), 0.95 (t, 6 H). UPLC 95.6%. FW 610.05, C₄₂H₇₅NO.

### Synthesis of 4-((3Z,6Z,9Z,28Z,31Z,34Z)-heptatriaconta-3,6,9,28,31,34-hexaen-19-yloxy)-N,N-dimethylbutan-1-amine (Compound 35)

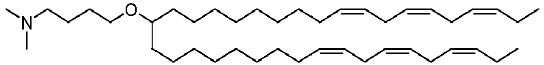

In the same fashion as 3-((6*Z*,9*Z*,28*Z*,31*Z*)-heptatriaconta-6,9,28,31-tetraen-19-yloxy)-*N,N*-dimethylpropan-1-amine (MC3 Ether), 4-((3*Z*,6*Z*,9*Z*,28*Z*,31*Z*,34*Z*)-heptatriaconta-3,6,9,28,31,34-hexaen-19-yloxy)-*N,N-*dimethylbutan-1-amine (1.33 g, 74%) was prepared as a pale yellow oil. ¹H NMR (400MHz, CDCl₃, δ_{H}) 5.48-5.27 (m, 12 H), 3.41 (t, 2 H), 3.21-3.17 (m, 1 H), 2.86-2.79 (m, 8 H), 2.50-2.40 (m, 2 H), 2.35 (s, 6 H), 2.12-2.02 (m, 8 H), 1.70-1.56 (m, 4 H), 1.50-1.20 (m, 28 H), 0.98 (t, 6 H). FW 624.08, C₄₃H₇₇NO.

### Synthesis of 2-((6Z,9Z,28Z,31Z)-hei)tatriaconta-6,9,28,31-tetraen-19-yloxy)-N,N-dimethylethanamine (Compound 36)

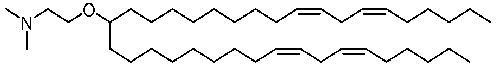

In the same fashion as 3-((6*Z*,9*Z*,28*Z*,31*Z*)-heptatriaconta-6,9,28,31-tetraen-19-yloxy)-*N,N*-dimethylpropan-1-amine (MC3 Ether), 2-((6Z,9Z,28Z,31Z)-heptatriaconta-6,9,28,31-tetraen-19-yloxy)-*N,N*-dimethylethanamine (1.30 g, 52%) was prepared as a pale yellow oil. ¹H NMR (400MHz, CDCl₃, δ_{H}) 5.42-5.26 (m, 8 H), 3.58 (t, 2 H), 3.23-3.18 (m, 1 H), 2.77 (app. t, 4 H), 2.63-2.58 (m, 2 H), 2.37 (s, 6 H), 2.10-2.00 (m, 8 H), 1.56-1.21 (m, 40 H), 0.90 (t, 6 H). FW 600.06, C₄₁H₇₇NO.

### Synthesis of 3-((6E,9E28E31E)-heptatriaconta-6,92831-tetraen-19-yloxy)-N,N-dimethylpropan-1-amine (Compound 37)

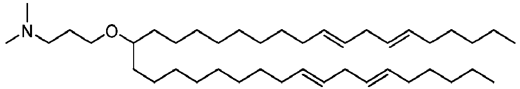

In the same fashion as 3-((6*Z*,9*Z*,28*Z*,31*Z*)-heptatriaconta-6,9,28,31-tetraen-19-yloxy)-*N,N*-dimethylpropan-1-amine (MC3 Ether), 3-((6*E*,9*E*,28*E*,31*E*)-heptatriaconta-6,9,28,31-tetraen-19-yloxy)-*N,N*-dimethylpropan-l-amine (0.22 g, 56%) was prepared as a colorless oil. ¹H NMR (400MHz, CDCl₃, δ_{H}) 5.50-5.35 (m, 8 H), 3.43 (t, 2 H), 3.21-3.18 (m, 1 H), 2.74-2.60 (m, 4 H), 2.48-2.40 (m, 2 H), 2.30 (s, 6 H), 2.16-1.91 (m, 8 H), 1.82-1.73 (m, 2 H), 1.51-1.20 (m, 40 H), 0.92 (t, 6 H). FW 614.08, C₄₂H₇₉NO.

### Reference Example 22. Synthesis of Compound 38.

3-((6Z,9Z,28E,31E)-heptatriaconta-6,9,28,31-tetraen-19-yloxy)-N,N-dimethylpropan-1-amine (Compound **38**) having the structure shown below was synthesized as described in Scheme 25 below.

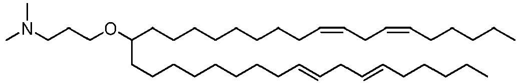

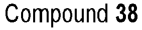

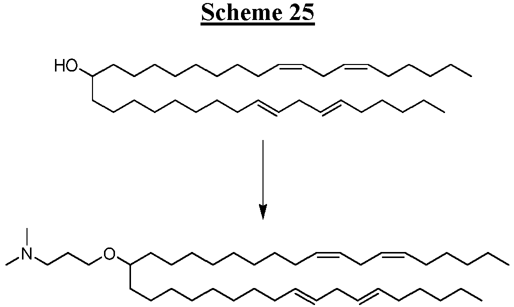

In the same fashion as 3-((6*Z*,9*Z*,28*Z*,31*Z*)-heptatriaconta-6,9,28,31-tetraen-19-yloxy)-*N,N*-dimethylpropan-1-amine (MC3 Ether), 3-((6*Z*,9*Z*,28*E*,31*E*)-heptatriaconta-6,9,28,3 1-tetraen-19-yloxy)-*N,N-*dimethylpropan-1-amine (1.23 g, 50%) was prepared as a colorless oil from (6*Z*,9*Z*,28*E*,31*E*)-heptatriaconta-6,9,28,31-tetraen-19-ol. ¹H NMR (400MHz, CDCl₃, δ_{H}) 5.50-5.32 (m, 8 H), 3.49 (t, 2 H), 3.22-3.18 (m, 1 H), 2.79 (app, t, 2 H), 2.76-2.62 (m, 4 H), 2.45 (br. s, 6 H), 2.30-2.15 (m, 4 H), 2.15-1.96 (m, 4 H), 1.94-1.83 (m, 2 H), 1.51-1.20 (m, 40 H), 0.95-0.88 (m, 6 H). FW 614.08, C₄₂H₇₉NO.

### Reference Example 23. Synthesis of Compound 39.

N,N-diethyl-4-((6*Z*,9*Z*,28*Z*,31*Z*)-heptatriaconta-6,9,28,31-tetraen-19-yloxy)but-2-yn-1-amine (Compound 39) having the structure shown below was synthesized as described in Scheme 26 below.

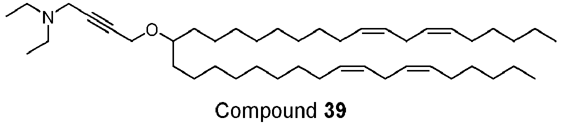

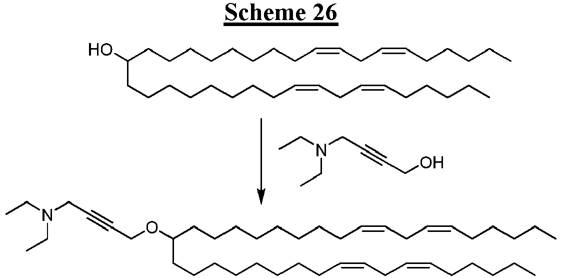

In the same fashion as 3-((6*Z*,9*Z*,28*Z*,31*Z*)-heptatriaconta-6,9,28,31-tetraen-19-yloxy)-N,N-dimethylpropan-l-amine (MC3 Ether), *N,N*-diethyl-4-((6*Z*,9*Z*,28*Z*,31*Z*)-heptatriaconta-6,9,28,31-tetraen-19-yloxy)but-2-yn-1-amine (511 mg, 48%) was prepared from di-linoleyl mesylate (1.0 g, 1.6 mmol) and 4-diethylaminobutyn-1-ol (1.2 mL, 8.2 mmol). ¹H NMR (400MHz, CDCl₃, δ_{H}) 5.46-5.35 (m, 8 H), 4.20 (s, 2 H), 3.50-3.39 (m, 3 H), 2.80 (app. t, 4 H), 2.59 (q, 4 H), 2.15-2.00 (m, 8 H), 1.65-1.58 (br. s, 2 H), 1.52-1.25 (m, 42 H), 1.10 (t, 6 H), 0.88 (t, 6 H). FW 652.13, C₄₅H₈₁NO.

### Reference Example 24. Synthesis of Compound 40.

(6*E*,9*E*,28*E*,31*E*)-heptatriaconta-6,9,28,31-tetraen-19-yl 4-(dimethylamino)butanoate (Compound **40**) having the structure shown below was synthesized as described in Scheme 27 below.

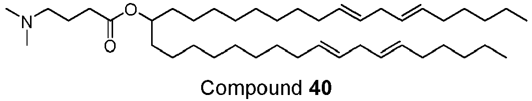

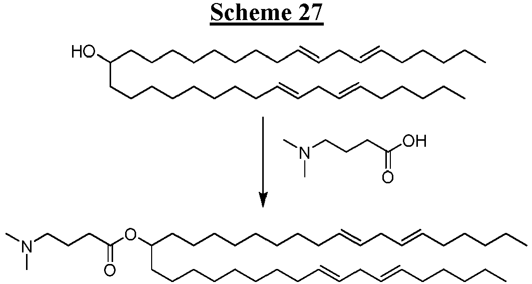

In the same fashion as (6*Z*,9*Z*,28*Z*,31*Z*)-heptatriaconta-6,9,28,31-tetraen-19-yl 4-(dimethylamino)butanoate (MC3), (6*E*,9*E*,28*E*,31*E*)-heptatnaconta-6,9,28,31-tetraen-19-yl 4-(dimethylamino)butanoate (2.9 g, 59%) was prepared as a colorless oil. ¹H NMR (400MHz, CDCl₃, δ_{H}) 5.49-5.35 (m, 8 H), 4.87 (app. p, 1 H), 2.75-2.61 (m, 4 H), 2.36-2.24 (m, 4 H), 2.22 (s, 6 H), 2.04-1.91 (m, 8 H), 1.83-1.72 (m, 3 H), 1.56-1.44 (m, 4 H), 1.40-1.19 (m, 38 H), 0.89 (t, 6 H). FW 642.09, C₄₃H₇₉NO₂.

### Reference Example 25. Synthesis of Compound 41.

3-((7*E*,9*Z*,28*Z*,30*E*)-heptatriaconta-7,9,28,30-tetraen-19-yloxy)-*N,N-*dimethylpropan-1-amine (Compound **41**) having the structure shown below was synthesized as described in Scheme 28 below.

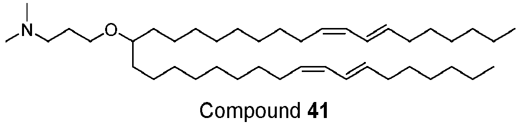

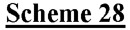

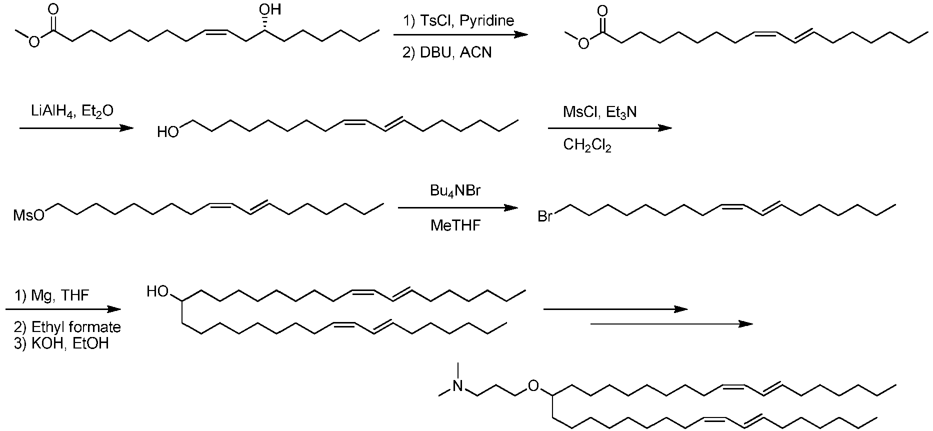

### Synthesis of (9Z,11E)-methyl octadeca-9,11-dienoate

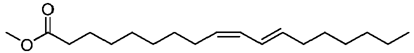

(9*Z*,11*E*)-methyl octadeca-9,11 -dienoate was synthesized according to the procedure described in US Patent No. 5,892,074.

### Synthesis of (9Z, 11E)-octadeca-9,11-dien-1-ol

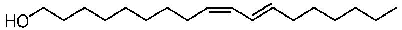

To a suspension of lithium aluminum hydride (1.5 g, 38.7 mmol) in anhydrous ether (50 mL) cooled to 0°C under nitrogen was slowly added a solution of (9*Z*,11*E*)-methyl octadeca-9,11-dienoate (11.6 g, 38.7 mmol) in anhydrous diethyl ether (50 mL + 25 mL rinse) via cannula transfer. The solution was stirred for 2 hours at 0°C then quenched slowly with 1M NaOH (3.5 mL). The solution was dried on magnesium sulfate, filtered and concentrated *in vacuo* to dryness to afford the product as a colorless oil (9.5 g, 92%).

### Synthesis of (9Z,11E)-octadeca-9,11-dienyl methanesulfonate

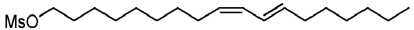

Using an analogous procedure to that described for the synthesis of dilinoleyl mesylate, (9*Z*,11*E*)-octadeca-9,11-dienyl methanesulfonate was obtained as a pale yellow oil (11.8 g, 96%).

### Synthesis of (7E,9Z)-18-bromooctadeca-7,9-diene

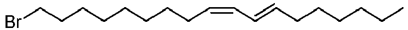

Using an analogous procedure to that described for the synthesis of linoleyl bromide, (7*E*,9*Z*)-18-bromooctadeca-7,9-diene was obtained as a pale yellow oil (11.0 g, 97%).

### Synthesis of (7E,9Z,28Z,30E)-heptatriaconta-7,9,28,30-tetraen-19-ol

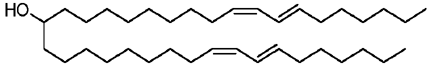

Using an analogous procedure to that described for the synthesis of dilinoleyl methanol, (7*E*,9*Z*,28*Z*,30*E*)-heptatriaconta-7,9,28,30-tetraen-19-ol was obtained as a pale yellow oil (4.7 g, 53%).

### Synthesis of 3-((7E,9Z,28Z,30E)-heptatriaconta-7,9,28,30-tetraen-19-yloxy)-N,N-dimethylpropan-1-amine (Compound 41)

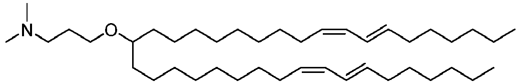

Using an analogous procedure to that described for the synthesis of 3-((6*Z*,9*Z*,28*Z*,31*Z*)-heptatriaconta-6,9,28,31-tetraen-19-yloxy)-*N,N*-dimethylpropan-1-amine, 3-((7*E*,9*Z*,28*Z*,30*E*)-heptatriaconta-7,9,28,30-tetraen-19-yloxy)-*N,N*-dimethylpropan-1-amine was obtained as a pale yellow oil (1.3 g). ¹H NMR (400 MHz, CDCl₃): *δ*6.35-5.26 (m, 8H), 3.49-3.44 (m, 2H), 3.23-3.15 (m, 1H), 2.60-2.50 (m, 2H), 2.38 (s, 6H), 2.21-1.95 (m, 8H), 1.88-1.77 (m, 2H), 1.50-1.21 (m, 44H), 0.92-0.86 (m, 6H).

### Reference Example 26. Synthesis of Compound 42.

3-((9*Z*,28*Z*)-heptatriaconta-9,28-dien-19-yloxy)-N,N-dimethylpropan-1-amine (Compound **42**) having the structure shown below was synthesized as described in Scheme 29 below.

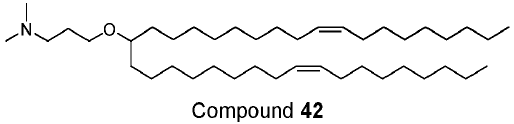

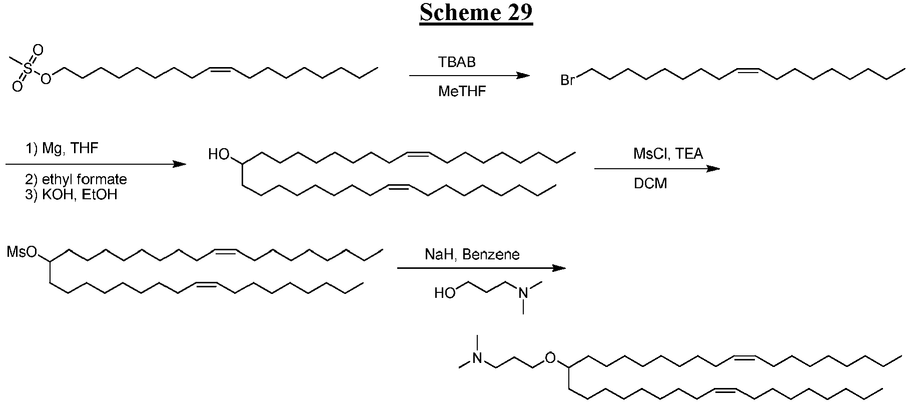

### Synthesis of Oleyl bromide

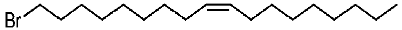

Using an analogous procedure to that described for the synthesis of linoleyl bromide, oleyl bromide was obtained as a colorless oil (70.2 g, quantitative).

### Synthesis of Diolevl methanol

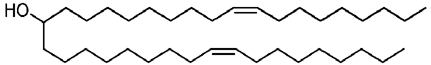

Using an analogous procedure to that described for the synthesis of dilinoleyl methanol, dioleyl methanol was obtained as a colorless oil (46.6 g, 82%).

### Synthesis of Dioleyl mesylate

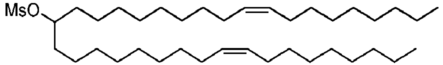

Using an analogous procedure to that described for the synthesis of dilinoleyl mesylate, dioleyl mesylate was obtained as a pale yellow oil (51.7 g, 97%).

### Synthesis of 3-((9Z,28Z)-heptatriaconta-9,28-dien-19-yloxy)-N,N-dimethylpropan-1-amine

### (Compound 42)

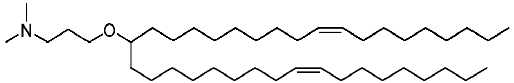

Using an analogous procedure to that described for the synthesis of 3-((6*Z*,9*Z*,28*Z*,31*Z*)-heptatriaconta-6,9,28,31-tetraen-19-yloxy)-*N,N*-dimethylpropan-1-amine, 3-((9*Z*,28*Z*)-heptatriaconta-9,28-dien-19-yloxy)-*N,N*-dimethylpropan-1-amine was obtained as a colorless oil (14.7 g, 28%). ¹H NMR (400 MHz, CDCl₃): *δ*5.41-5.30 (m, 4H), 3.46 (t, *J* = 6.4 Hz, 2H), 3.23-3.16 (m, 1H), 2.46-2.38 (m, 2H), 2.29 (s, 6H), 2.08-1.96 (m, 8H), 1.81-1.72 (m, 2H), 1.52-1.20 (m, 52H), 0.93-0.86 (m, 6H).

### Reference Example 27. Synthesis of Compound 43.

1-(1-((6*Z*,9*Z*,28*Z*,31*Z*)-heptatriaconta-6,9,28,31-tetraen-19-yl)-1H-1,2,3-triazol-4-yl)-N,N-dimethylmethanamine (Compound **43**) having the structure shown below was synthesized as described in Scheme 30 below.

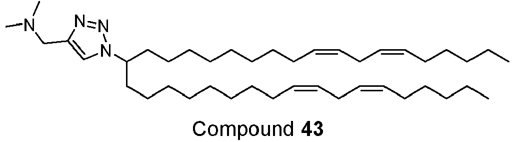

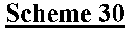

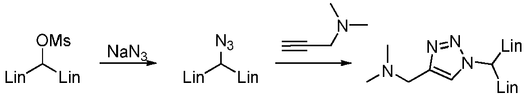

### Synthesis of Dilinoleyl methyl azide

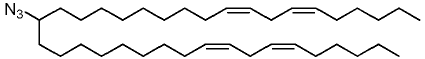

A solution of dilinoleyl mesylate (6.27 g, 10.3 mmol) in anhydrous DMF (110 mL) was treated with NaN₃ (3.35 g, 51.6 mmol) and subsequently heated (80°C, 18h). The DMF was then removed under reduced pressure. The residue was taken up in CH₂Cl₂ and washed with sat. aq. NaHCO₃ (2x) and brine, dried (Na₂SO₄), filtered, concentrated, and purified via column chromatography (0.5% → 1% ethyl acetate/hexanes) to yield dilinoleyl methyl azide (4.89 g, 86%) as a colorless oil. FW 553.95, C₃₇H₆₇N₃.

### Synthesis of 1-(1-((6Z,9Z,28Z,31Z)-heptatriaconta-6,9,28,31-tetraen-19-yl)-1H-1,2,3-triazol-4-yl)-N,N-dimethylmethanamine (Compound 43)

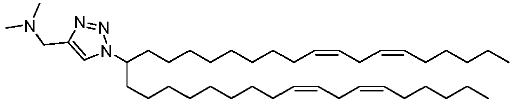

A solution of dilinoleyl methyl azide (500 mg, 0.90 mmol) and 3-dimethylamino-1-propyne (75 mg, 0.90 mmol) in H₂O and tert-butyl alcohol (1:1, 12 mL) was treated with sodium ascorbate (0.090 mmol, 17.9µL taken from a 1M solution in water), followed by CuSO₄·5H₂O (2.3 mg, dissolved in 30µL of water) and stirred (96 h). The solution was then diluted with H₂O (50 mL) and extracted with CH₂Cl₂ (3x), dried (Na₂SO₄), filtered, concentrated and purified via column chromatography (2% → 4% MeOH/CH₂Cl₂) to yield the title compound as a colorless oil (524 mg, 91%). ¹H NMR (400MHz, CDCl₃, δ_{H}) 7.76 (br. s, 1 H), 5.47-5.25 (m, 8 H), 4.48 (p, 1 H), 3.80 (br. s, 2 H), 2.75 (app. t, 4 H), 2.49 (s, 6 H), 2.15-2.00 (m, 8 H), 1.91-1.75 (m ,4 H), 1.40-1.12 (m, 34 H), 1.11-0.98 (m, 2 H), 0.85 (t, 6 H). FW 637.08, C₄₂H₇₆N₄.

### Reference Example 28. Synthesis of Compound 44.

3-((6*Z*,9*Z*,28*Z*,31*Z*)-heptatriaconta-6,9,28,31-tetraen-19-yloxy)-N,N,N-trimethylpropan-1-aminium chloride (Compound **44**) having the structure shown below was synthesized as described in Scheme 31 below.

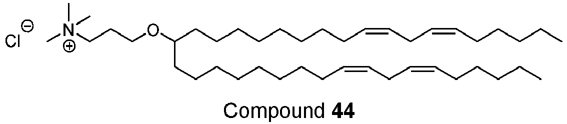

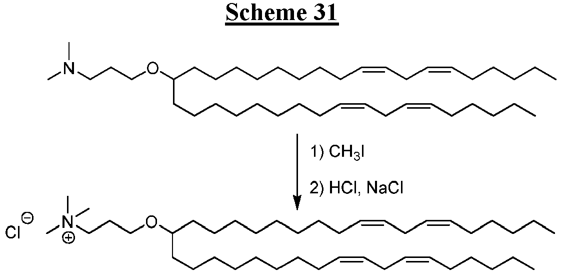

To a solution of Compound **13** (3.0 g, 4.9 mmol) in dichloromethane (10 mL) was added iodomethane (4.5 mL, 73.5 mmol). The solution was stirred at room temperature for 16 hours at room temperature under nitrogen. Upon completion, the solution was concentrated *in vacuo* to dryness and dissolved in dichloromethane (150 mL). The solution was transferred to a separatory funnel and washed with 1M HCl in methanol solution (40 mL). To this solution was added brine (50 mL) and the mixture was shaken well. The aqueous phase was removed and previous wash procedure was repeated four more times to complete the ion exchange process. The dichloromethane solution was dried on magnesium sulfate, filtered and concentrated *in vacuo* to dryness. The yellow residue was purified by column chromatography on silica gel (100% ethyl acetate to 15% MeOH in ethyl acetate) to afford the title compound as a colorless oil (2.3 g, 71%). ¹H NMR (400 MHz, CDCl₃): *δ* 5.43-5.28 9m, 8H), 3.63-3.56 (m, 2H), 3.54-3.45 (m, 11H), 3.23-3.16 (m, 1H), 2.81-2.76 (m, 4H), 2.09-2.01 (m, 10H), 1.45-1.20 (m, 40H), 0.92-0.86 (m, 6H).

### Reference Example 29. Lipid Encapsulation of siRNA.

All siRNA molecules used in these studies were chemically synthesized and annealed using standard procedures.

In some embodiments, siRNA molecules were encapsulated into serum-stable nucleic acid-lipid particles (SNALP) composed of the following lipids: (1) the lipid conjugate PEG2000-C-DMA (3-N-[(-methoxypoly(ethylene glycol)2000)carbamoyl]-1,2-dimyristyloxypropylamine); (2) one or more cationic lipids or salts thereof (e.g., cationic lipids of Formula I of the invention and/or other cationic lipids described herein); (3) the phospholipid DPPC (1,2-dipalmitoyl-sn-glycero-3-phosphocholine) (Avanti Polar Lipids; Alabaster, AL); and (4) synthetic cholesterol (Sigma-Aldrich Corp.; St. Louis, MO) in the molar ratio 1.4:57.1:7.1:34.3, respectively. In other words, siRNA molecules were encapsulated into SNALP of the following "1:57" formulation: 1.4% PEG2000-C-DMA; 57.1% cationic lipid; 7.1% DPPC; and 34.3% cholesterol. It should be understood that the 1:57 formulation is a target formulation, and that the amount of lipid (both cationic and non-cationic) present and the amount of lipid conjugate present in the formulation may vary. Typically, in the 1:57 formulation, the amount of cationic lipid will be 57.1 mol % ± 5 mol %, and the amount of lipid conjugate will be 1.4 mol % ± 0.5 mol %, with the balance of the 1:57 formulation being made up of non-cationic lipid (e.g., phospholipid, cholesterol, or a mixture of the two).

In other embodiments, siRNA were encapsulated into SNALP composed of the following lipids: (1) the lipid conjugate PEG750-C-DMA (3-N-[(-Methoxypoly(ethylene glycol)750)carbamoyl]-1,2-dimyristyloxypropylamine); (2) one or more cationic lipids or salts thereof (*e.g.,* cationic lipids of Formula I of the invention and/or other cationic lipids described herein); (3) the phospholipid DPPC; and (4) synthetic cholesterol in the molar ratio 6.76:54.06:6.75:32.43, respectively. In other words, siRNA were encapsulated into SNALP of the following "7:54" formulation: 6.76 mol % PEG750-C-DMA; 54.06 mol % cationic lipid; 6.75 mol % DPPC; and 32.43 mol % cholesterol. Typically, in the 7:54 formulation, the amount of cationic lipid will be 54.06 mol % ± 5 mol %, and the amount of lipid conjugate will be 6.76 mol % ± 1 mol %, with the balance of the 7:54 formulation being made up of non-cationic lipid (e.g., phospholipid, cholesterol, or a mixture of the two).

For vehicle controls, empty particles with identical lipid composition may be formed in the absence of siRNA.

### Reference Example 30. Characterization of SNALP Formulations Containing

### Novel Cationic Lipids.

This example demonstrates the efficacy of 1:57 SNALP formulations containing various novel cationic lipids of Formula I described herein with an siRNA targeting ApoB in a mouse liver model. The ApoB siRNA sequence used in this study is provided in Table 1.

**Table 1**

| **siRNA** | **ApoB siRNA Sequence** | | **% 2'OMe-Modified** | **% Modified in DS Region** |
|---|---|---|---|---|
| ApoB-10164 | 5'-AGU**G**UCA**U**CACAC**U**GAAUACC-3' | (SEQ ID NO:1) | 7/42 = 16.7% | 7/38 = 18.4% |
| | 3'-GU**U**CACAGUAGU**G**U**G**AC**U**UAU-5' | (SEQ ID NO:2) | | |

| | | | | |
|---|---|---|---|---|
| Column 1: The number after "ApoB" refers to the nucleotide position of the 5' base of the sense strand relative to the human ApoB mRNA sequence NM _000384. Column 2: 2'OMe nucleotides are indicated in bold and underlined. The 3' -overhangs on one or both strands of the siRNA molecule may alternatively comprise 1-4 deoxythymidine (dT) nucleotides, 1-4 modified and/or unmodified uridine (U) ribonucleotides, or 1-2 additional ribonucleotides having complementarity to the target sequence or the complementary strand thereof. Column 3: The number and percentage of 2'OMe-modified nucleotides in the siRNA molecule are provided. Column 4: The number and percentage of modified nucleotides in the double-stranded (DS) region of the siRNA molecule are provided. | | | | |

1:57 SNALP formulations containing encapsulated ApoB siRNA were prepared as described in Section VI above with DLin-C2K-DMA ("C2K") or Compound 1 (DLin-M-C3-DMA ("MC3")), 4, 5, 8, 9, 10, 11, 13, 15, 17, 18, 20, 22, 23, 25, 27, 28, 29, 30, 31, 34, 35, 40, or 41.

SNALP formulations were administered by IV injection at 0.033 mg/kg or at 0.05 mg/kg into Balb/c mice (n = 3 per group). Liver ApoB mRNA levels were evaluated at 48 hours after SNALP administration by a branched DNA assay (QuantiGene assay) to assess ApoB mRNA relative to the housekeeping gene GAPDH.

Table 2 shows a comparison of the liver ApoB mRNA knockdown activity of each of these SNALP formulations. As non-limiting examples, Table 2 illustrates that a SNALP formulation containing Compound 1, 4, 8, 13, 15, 27, 28, or 35 displayed unexpectedly improved ApoB silencing activity compared to a SNALP formulation containing the C2K benchmark cationic lipid when administered at the same dose.

**Table 2. Effect on ApoB:GAPD of 1:57 SNALP Containing Novel Cationic Lipids Administered at 0.05 or 0.033 mg/kg to Balb/c Mice (48 h, n=3)**

| **Compound** | **Dose (mg/kg)** | **ApoB knockdown relative to PBS control (%)** |
|---|---|---|
| C2K | 0.033 | -51 |
| | 0.05 | -66 |
| **1** (MC3) | 0.033 | -71 |
| | 0.05 | -81 |
| **4** | 0.033 | -78 |
| **5** | 0.033 | -56 |
| **8** | 0.033 | -71 |
| **9** | 0.033 | -54 |
| **10** | 0.033 | -50 |
| **11** | 0.05 | -54 |
| **13** | 0.033 | -69 |
| **15** | 0.05 | -77 |
| **17** | 0.05 | -14 |
| **18** | 0.05 | -46 |
| **20** | 0.05 | -50 |
| **22** | 0.033 | -30 |
| **23** | 0.033 | -33 |
| **25** | 0.05 | -66 |
| **27** | 0.033 | -66 |
| **28** | 0.033 | -68 |
| **29** | 0.05 | -59 |
| **30** | 0.05 | -65 |
| **31** | 0.05 | -62 |
| **34** | 0.033 | -41 |
| **35** | 0.033 | -78 |
| **40** | 0.05 | -58 |
| **41** | 0.033 | -52 |

It is to be understood that the above description is intended to be illustrative and not restrictive. Many embodiments will be apparent to those of skill in the art upon reading the above description. The scope of the invention should, therefore, be determined not with reference to the above description, but should instead be determined with reference to the appended claims.

## Claims

1. A lipid particle comprising a cationic lipid of Formula I having the following structure: 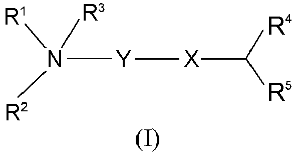 or salts thereof, wherein R¹ and R² are either the same or different and are independently hydrogen (H) or an optionally substituted C₁-C₆ alkyl, C₂-C₆ alkenyl, or C₂-C₆ alkynyl, or R¹ and R² may join to form an optionally substituted heterocyclic ring;
R³ is either absent or is hydrogen (H) or a C₁-C₆ alkyl to provide a quaternary amine; R⁴ and R⁵ are independently selected from the group consisting of a dodecatrienyl moiety, a tetradectrienyl moiety, a hexadecatrienyl moiety, an octadecatrienyl moiety, and an icosatrienyl moiety;
X is O, S, N(R⁶), C(O), C(O)N(R⁶), N(R⁶)C(O), OC(O)N(R⁶), N(R⁶)C(O)O, C(O)S, C(S)O, S(O), S(O)(O), C(S), wherein R⁶ is hydrogen (H) or an optionally substituted C₁-C₁₀ alkyl, C₂-C₁₀ alkenyl, or C₂-C₁₀ alkynyl; and
Y is either absent or is an optionally substituted C₁-C₆ alkyl, C₂-C₆ alkenyl, or C₂-C₆ alkynyl.

2. The lipid particle of claim 1, wherein the cationic lipid is: 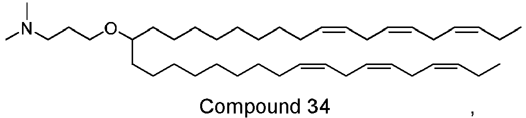 or 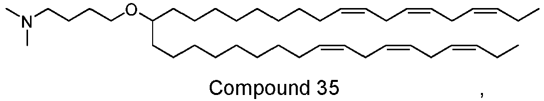 or a salt thereof.

3. The lipid particle of claim 1 or claim 2, wherein:
(a) the particle further comprises a non-cationic lipid, optionally wherein the non-cationic lipid is selected from the group consisting of a phospholipid, cholesterol, or a mixture of a phospholipid and cholesterol, wherein the phospholipid optionally comprises dipalmitoylphosphatidylcholine (DPPC), distearoylphosphatidylcholine (DSPC), or a mixture thereof, and wherein the cholesterol is optionally a cholesterol derivative; and/or
(b) the particle further comprises a conjugated lipid that inhibits aggregation of particles, optionally wherein the conjugated lipid that inhibits aggregation of particles comprises a polyethyleneglycol (PEG)-lipid conjugate, such as a PEG-diacylglycerol (PEG-DAG) conjugate, a PEG-dialkyloxypropyl (PEG-DAA) conjugate, or a mixture thereof; and/or
(c) the particle further comprises a therapeutic agent, optionally wherein the therapeutic agent is a nucleic acid, such as an interfering RNA, optionally wherein the interfering RNA is selected from the group consisting of a small interfering RNA (siRNA), an asymmetrical interfering RNA (aiRNA), a microRNA (miRNA), a Dicer-substrate dsRNA, a small hairpin RNA (shRNA), and mixtures thereof, and/or
(d) the particle has a median diameter of from about 30 nm to about 150 nm.

4. The lipid particle of claim 3 (c) or (d), wherein:
(i) the therapeutic agent is not substantially degraded after incubation of the particle in serum at 37°C for 30 minutes; and/or
(ii) the therapeutic agent is fully encapsulated in the particle; and/or
(iii) the particle has a lipid:therapeutic agent mass ratio of from about 5:1 to about 15:1.

5. A pharmaceutical composition comprising a lipid particle of any one of the preceding claims and a pharmaceutically acceptable carrier.

6. An *in vitro* method for introducing a therapeutic agent into a cell, the method comprising contacting the cell with a lipid particle of any one of claims 1 to 4 comprising a therapeutic agent.

7. A lipid particle of any one of claims 1 to 4 comprising a therapeutic agent, for use in a method for the *in vivo* delivery of said therapeutic agent, the method comprising administering said lipid particle to a mammal.

8. The lipid particle for use according to claim 7, wherein:
(i) the administration is selected from the group consisting of oral, intranasal, intravenous, intraperitoneal, intramuscular, intra-articular, intralesional, intratracheal, subcutaneous, and intradermal; and/or
(ii) the mammal is a human.

9. A lipid particle of any one of claims 1 to 4 comprising a therapeutic agent, for use in a method for treating a disease or disorder in a mammal in need thereof, the method comprising administering to the mammal a therapeutically effective amount of said lipid particle.

10. The lipid particle for use according to claim 9, wherein:
(i) the disease or disorder is selected from the group consisting of a viral infection, a liver disease or disorder, and cancer; and/or
(ii) the mammal is a human.

## Patentansprüche

1. Lipidpartikel, umfassend ein kationisches Lipid der Formel I mit der folgenden Struktur: 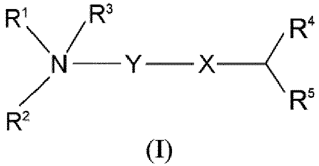 oder Salze davon, wobei R¹ und R² entweder gleich oder verschieden sind und unabhängig Wasserstoff (H) oder ein optional substituiertes C₁-C₆-Alkyl, C₂-C₆-Alkenyl oder C₂-C₆-Alkynyl sind oder sich R¹ und R² verbinden können, um einen optional substituierten heterocyclischen Ring auszubilden;
R³ entweder fehlt oder Wasserstoff (H) oder ein C₁-C₆-Alkyl ist, um ein quartäres Amin bereitzustellen;
R⁴ und R⁵ unabhängig aus der Gruppe ausgewählt sind, die aus einem Dodecatrienylrest, einem Tetradectrienylrest, einem Hexadecatrienylrest, einem Octadecatrienylrest und einem Icosatrienylrest besteht;
X O, S, N(R⁶), C(O), C(O)N(R⁶), N(R⁶)C(O), OC(O)N(R⁶), N(R⁶)C(O)O, C(O)S, C(S)O, S(O), S(O)(O), C(S) ist, wobei R⁶ Wasserstoff (H) oder ein optional substituiertes C₁-C₁₀-Alkyl, C₂-C₁₀-Alkenyl oder C₂-C₁₀-Alkynyl ist; und
Y entweder fehlt oder ein optional substituiertes C₁-C₆-Alkyl, C₂-C₆-Alkenyl oder C₂-C₆-Alkynyl ist.

2. Lipidpartikel nach Anspruch 1, wobei das kationische Lipid: 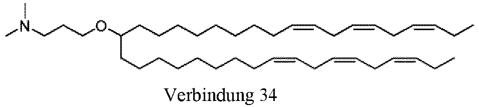 oder 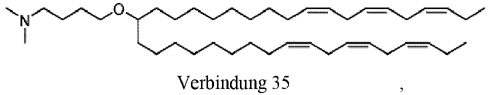 oder ein Salz davon ist.

3. Lipidpartikel nach Anspruch 1 oder 2, wobei:
(a) das Partikel ferner ein nichtkationisches Lipid umfasst, optional wobei das nichtkationische Lipid aus der Gruppe ausgewählt ist, die aus einem Phospholipid, Cholesterol oder einem Gemisch aus einem Phospholipid und einem Cholesterol besteht, wobei das Phospholipid optional Dipalmitoylphosphatidylcholin (DPPC), Distearoylphosphatidylcholin (DSPC) oder ein Gemisch davon umfasst und wobei das Cholesterol optional ein Cholesterolderivat ist; und/oder
(b) das Partikel ferner ein konjugiertes Lipid umfasst, das die Aggregation von Partikeln hemmt, optional wobei das konjugierte Lipid, das die Aggregation von Partikeln hemmt, ein Polyethylenglycol- (PEG-) Lipidkonjugat wie ein PEG-Diacylglycol- (PEG-DAG-) Konjugat, ein PEG-Dialkyloxypropyl- (PEG-DAA-) Konjugat oder ein Gemisch davon umfasst; und/oder
(c) das Partikel ferner ein Therapeutikum umfasst, optional wobei das Therapeutikum eine Nukleinsäure wie eine interferierende RNA ist, optional wobei die interferierende RNA aus der Gruppe ausgewählt ist, die aus einer kleinen interferierenden RNA (siRNA), einer asymmetrischen interferierenden RNA (aiRNA), einer microRNA (miRNA), einem Dicer-Substrat dsRNA, einer Small hairpin RNA (shRNA) oder Gemischen davon besteht, und/oder
(d) das Partikel einen Mediandurchmesser von etwa 30 nm bis etwa 150 nm aufweist.

4. Lipidpartikel nach Anspruch 3 (c) oder (d), wobei:
(i) das Therapeutikum nach der Inkubation des Partikels in Serum bei 37 °C für 30 Minuten im Wesentlichen nicht abgebaut ist; und/oder
(ii) das Therapeutikum in dem Partikel vollständig eingekapselt ist; und/oder
(iii) das Partikel ein Massenverhältnis von Lipid:Therapeutikum von etwa 5:1 zu etwa 15:1 aufweist.

5. Pharmazeutische Zusammensetzung, umfassend einen Lipidpartikel nach einem der vorhergehenden Ansprüche und einen pharmazeutisch unbedenklichen Träger.

6. *In-vitro*-Verfahren zum Einführen eines Therapeutikums in eine Zelle, wobei das Verfahren das Inberührungbringen der Zelle mit einem Lipidpartikel nach einem der Ansprüche 1 bis 4, umfassend ein Therapeutikum, umfasst.

7. Lipidpartikel nach einem der Ansprüche 1 bis 4, umfassend ein Therapeutikum, für die Verwendung in einem Verfahren für die *In-vivo*-Abgabe des Therapeutikums, wobei das Verfahren das Verabreichen des Lipidpartikels an ein Säugetier umfasst.

8. Lipidpartikel für die Verwendung nach Anspruch 7, wobei:
(i) die Verabreichung aus der Gruppe ausgewählt ist, die aus oral, intranasal, intravenös, intraperitoneal, intramuskulär, intra-artikular, intraläsional, intratracheal, subkutan und intradermal besteht; und/oder
(ii) das Säugetier ein Mensch ist.

9. Lipidpartikel nach einem der Ansprüche 1 bis 4, umfassend ein Therapeutikum für die Verwendung in einem Verfahren zum Behandeln einer Krankheit oder Erkrankung in einem Säugetier, das dies benötigt, wobei das Verfahren das Verabreichen einer therapeutisch wirksamen Menge des Lipidpartikels an das Säugetier umfasst.

10. Lipidpartikel für die Verwendung nach Anspruch 9, wobei:
(i) die Krankheit oder Erkrankung aus der Gruppe ausgewählt ist, die aus einer viralen Infektion, einer Leberkrankheit oder -erkrankung und einem Krebs besteht; und/oder
(ii) das Säugetier ein Mensch ist.

## Revendications

1. Particule lipidique, comprenant un lipide cationique de la formule I ayant la structure suivante : 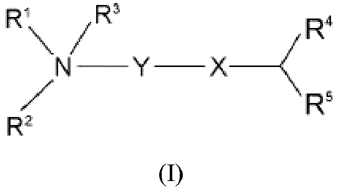 ou des sels de celui-ci, dans laquelle R¹ et R² sont les mêmes ou différents et sont indépendamment hydrogène (H) ou un C₁-C₆ alkyle, C₂-C₆ alcényle, ou C₂-C₆ alkynyle optionnellement substitué, ou R¹ et R² peuvent se joindre pour former un anneau hétérocyclique optionnellement substitué ;
R³ est absent ou est hydrogène (H) ou un C₁-C₆ alkyle pour fournir une amine quaternaire ;
R⁴ et R⁵ sont indépendamment sélectionnés parmi le groupe constitué de : un groupe caractéristique dodécatriényle, un groupe caractéristique tétradectriényle, un groupe caractéristique hexadécatriényle, un groupe caractéristique octadécatriényle, et un groupe caractéristique icosatriényle ;
X est O, S, N(R⁶), C(O), C(O)N(R⁶), N(R⁶)C(O), OC(O)N(R⁶), N(R⁶)C(O)O, C(O)S, C(S)O, S(O), S(O)(O), C(S), dans laquelle R⁶ est hydrogène (H) ou un C₁-C₁₀ alkyle, C₂-C₁₀ alcényle, ou C₂-C₁₀ alkynyle optionnellement substitué ; et
Y est absent ou est un C₁-C₆ alkyle, C₂-C₆ alcényle, ou C₂-C₆ alkynyle optionnellement substitué.

2. Particule lipidique selon la revendication 1, dans laquelle le lipide cationique est : 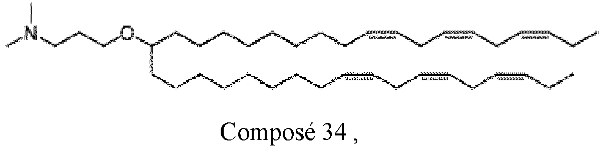 ou 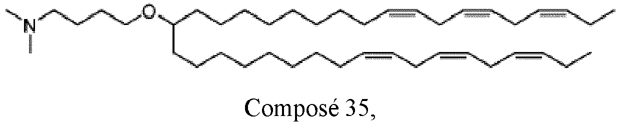 ou un sel de celui-ci.

3. Particule lipidique selon la revendication 1 ou la revendication 2, dans laquelle :
(a) la particule comprend en outre un lipide non cationique, optionnellement dans laquelle le lipide non cationique est sélectionné parmi le groupe constitué de : un phospholipide, cholestérol, ou un mélange d'un phospholipide et de cholestérol, dans laquelle le phospholipide comprend optionnellement dipalmitoylphosphatidylcholine (DPPC), distéaroylphosphatidylcholine (DSPC), ou un mélange de celles-ci, et dans laquelle le cholestérol est optionnellement un dérivé de cholestérol ; et/ou
(b) la particule comprend en outre un lipide conjugué qui empêche l'agrégation de particules, optionnellement dans laquelle le lipide conjugué qui empêche l'agrégation de particules comprend un conjugué polyéthylèneglycol (PEG)-lipide, tel qu'un conjugué PEG-diacylglycérol (PEG-DAG), un conjugué PEG-dialkyloxypropyle (PEG-DAA), ou un mélange de ceux-ci ; et/ou
(c) la particule comprend en outre un agent thérapeutique, optionnellement dans laquelle l'agent thérapeutique est un acide nucléique, tel qu'un ARN interférent, optionnellement dans laquelle l'ARN interférent est sélectionné parmi le groupe constitué d'un petit ARN interférent (siARN), un ARN interférent asymétrique (aiARN), un microARN (miARN), un ARN substrat de Dicer dsARN, un petit ARN en épingle à cheveux (shARN), et des mélanges de ceux-ci, et/ou
(d) la particule a un diamètre médian d'environ 30 nm à environ 150 nm.

4. Particule lipidique selon la revendication 3 (c) ou (d), dans laquelle :
(i) l'agent thérapeutique n'est pas sensiblement dégradé après l'incubation de la particule dans un sérum à 37°C pendant 30 minutes ; et/ou
(ii) l'agent thérapeutique est entièrement encapsulé dans la particule ; et/ou
(iii) la particule a un rapport de masse lipide : agent thérapeutique d'environ 5:1 à environ 15:1.

5. Composition pharmaceutique, comprenant une particule lipidique d'une quelconque des revendications précédentes et un vecteur pharmaceutiquement acceptable.

6. Procédé *in vitro* pour introduire un agent thérapeutique dans une cellule, le procédé comprenant la mise en contact de la cellule avec une particule lipidique d'une quelconque des revendications 1 à 4 comprenant un agent thérapeutique.

7. Particule lipidique selon l'une quelconque des revendications 1 à 4, comprenant un agent thérapeutique, pour l'utilisation dans un procédé pour la fourniture *in vivo* dudit agent thérapeutique, le procédé comprenant l'administration de ladite particule lipidique à un mammifère.

8. Particule lipidique pour l'utilisation selon la revendication 7, dans laquelle :
(i) l'administration est sélectionnée parmi le groupe constitué de : orale, intranasale, intraveineuse, intrapéritonéale, intramusculaire, intra-articulaire, intralésionnelle, intratrachéale, sous-cutanée, et intradermique ; et/ou
(ii) le mammifère est un humain.

9. Particule lipidique selon l'une quelconque des revendications 1 à 4 comprenant un agent thérapeutique, pour l'utilisation dans un procédé pour le traitement d'une maladie ou d'un trouble chez un mammifère en ayant besoin, le procédé comprenant l'administration, au mammifère, d'une quantité thérapeutiquement efficace de ladite particule lipidique.

10. Particule lipidique pour l'utilisation selon la revendication 9, dans laquelle :
(i) la maladie ou le trouble est sélectionné parmi le groupe constitué de : une infection virale, une maladie ou un trouble du foie, et un cancer ; et/ou
(ii) le mammifère est un humain.
